# EUROPEAN PATENT SPECIFICATION

(11) **EP 1 100 921 B1**
(45) Date of publication and mention of the grant of the patent: **02.05.2007**
(21) Application number: 99934989.7
(22) Date of filing: 27.07.1999
(51) Int. Cl.: C12N 15/31, C07K 14/315, C07K 16/12, G01N 33/50, A61K 39/09, C12Q 1/68

(54) **STREPTOCOCCUS PNEUMONIAE PROTEINS AND NUCLEIC ACID MOLECULES**
STREPTOCOCCUS PNEUMONIAE PROTEINE UND NUKLEINSÄUREN
PROTEINES DE STREPTOCOCCUS PNEUMONIAE ET MOLECULES D'ACIDE NUCLEIQUE

(30) Priority: 27.07.1998 GB 9816337; 19.03.1999 US 125164 P
(43) Date of publication of application: 23.05.2001
(60) Divisional application: 07000625.9
(73) Proprietor: Sanofi Pasteur Limited, Toronto, Ontario M2R 3T4 (CA)
(72) Inventor: GILBERT, Christophe François Guy, F-69622 Villeurbanne Cedex (FR); HANSBRO, Philip Michael, Newcastle NSW 2300 (AT)
(74) Representative: Chapman, Paul William
(86) International application number: PCT/GB1999/002451
(87) International publication number: WO 2000/006737

(56) References cited:
- EP-A- 0 622 081
- EP-A- 0 885 966
- EP-A- 0 887 413
- EP-A- 0 891 984
- WO-A-95/06732
- WO-A-98/18930
- WO-A-98/18931
- LANGE ROLAND ET AL: "Domain organization and molecular characterization of 13 two-component systems identified by genome sequencing of Streptococcus pneumoniae." GENE (AMSTERDAM) SEPT. 3, 1999, vol. 237, no. 1, pages 223-234, XP004183515 ISSN: 0378-1119
- GUENZI ERIC ET AL: "A two-component signal-transducing system is involved in competence and penicillin susceptibility in laboratory mutants of Streptococcus pneumoniae." MOLECULAR MICROBIOLOGY 1994, vol. 12, no. 3, 1994, pages 505-515, XP000905352 ISSN: 0950-382X
- FONTAN PA ET AL: "Streptococcus pmeumoniae choline transporter" EMBL DATABASE ENTRY AF162656, ACCESSION NUMBER AF162656,26 July 1999 (1999-07-26), XP002136498
- FONTAN P A ET AL: "A choline transporter as a virulence determinant of Streptococcus pneumoniae." 97TH GENERAL MEETING OF THE AMERICAN SOCIETY FOR MICROBIOLOGY;MIAMI BEACH, FLORIDA, USA; MAY 4-8, 1997, vol. 97, 1997, page 103 XP000892162 Abstracts of the General Meeting of the American Society for Microbiology 1997 ISSN: 1060-2011
- TAKEMOTO K ET AL: "Putative ferric transport ATP-binding protein AFUC" SWISSPROT DATABASE ENTRY AFUC_ECOLI, ACCESSION NUMBER P37009, 1 June 1994 (1994-06-01), XP002136499
- FLEISCHMANN RD ET AL: "Putative ferric transport ATP-binding protein AFUC" SWISSPROT DATABASE ENTRY AFUC_HAEIN, ACCESSION NUMBER P44531, 1 November 1995 (1995-11-01), XP002136500
- BLATTNER FR ET AL: "Spermidine/putrescine transport ATP-binding protein POTA" SWISSPROT DATABASE ENTRY POTA_ECOLI, ACCESSION NUMBER P23858, 1 November 1991 (1991-11-01), XP002136501
- POLISSI ALESSANDRA ET AL: "Large-scale identification of virulence genes from Streptococcus pneumoniae." INFECTION AND IMMUNITY DEC., 1998, vol. 66, no. 12, December 1998 (1998-12), pages 5620-5629, XP002136502 ISSN: 0019-9567
- DINTILHAC A ET AL: "The adc locus, which affects competence for genetic transformation in Streptococcus pneumoniae, encodes an ABC transporter with a putative lipoprotein homologous to a family of streptococcal adhesins." RESEARCH IN MICROBIOLOGY 1997, vol. 148, no. 2, 1997, pages 119-131, XP002115703 ISSN: 0923-2508

## Description

The present invention relates to proteins derived from *Streptococcus pneumoniae*, nucleic acid molecules encoding such proteins, the use of the nucleic acid and/or proteins as antigens/immunogens and in detection/diagnosis, as well as methods for screening the proteins/nucleic acid sequences as potential anti-microbial targets.

*Streptococcus pneumoniae,* commonly referred to as the pneumococcus, is an important pathogenic organism. The continuing significance of *Streptoccocus pneumoniae* infections in relation to human disease in developing and developed countries has been authoritatively reviewed (Fiber, G.R., *Science,* **265**: 1385-1387 (1994)). That indicates that on a global scale this organism is believed to be the most common bacterial cause of acute respiratory infections, and is estimated to result in 1 million childhood deaths each year, mostly in developing countries (Stansfield, S.K., *Pediatr. Infect. Dis.,* **6:** 622 (1987)). In the USA it has been suggested (Breiman *et al, Arch. Intern. Med.,* **150:** 1401 (1990)) that the pneumococcus is still the most common cause of bacterial pneumonia, and that disease rates are particularly high in young children, in the elderly, and in patients with predisposing conditions such as asplenia, heart, lung and kidney disease, diabetes, alcoholism, or with immunosupressive disorders, especially AIDS. These groups are at higher risk of pneumococcal septicaemia and hence meningitis and therefore have a greater risk of dying from pneumococcal infection. The pneumococcus is also the leading cause of otitis media and sinusitis, which remain prevalent infections in children in developed countries, and which incur substantial costs.

The need for effective preventative strategies against pneumococcal infection is highlighted by the recent emergence of penicillin-resistant pneumococci. It has been reported that 6.6% of pneumoccal isolates in 13 US hospitals in 12 states were found to be resistant to penicillin and some isolates were also resistant to other antibiotics including third generation cyclosporins (Schappert, S.M., *Vital and Health Statistics of the Centres for Disease Control*/*National Centre for Health Statistics,* **214**:1 (1992)). The rates of penicillin resistance can be higher (up to 20%) in some hospitals (Breiman *et al,* J. Am. Med. Assoc., **271:** 1831 (1994)). Since the development of penicillin resistance among pneumococci is both recent and sudden, coming after decades during which penicillin remained an effective treatment, these findings are regarded as alarming.

For the reasons given above, there are therefore compelling grounds for considering improvements in the means of preventing, controlling, diagnosing or treating pneumococcal diseases.

Various approaches have been taken in order to provide vaccines for the prevention of pneumococcal infections. Difficulties arise for instance in view of the variety of serotypes (at least 90) based on the structure of the polysaccharide capsule surrounding the organism. Vaccines against individual serotypes are not effective against other serotypes and this means that vaccines must include polysaccharide antigens from a whole range of serotypes in order to be effective in a majority of cases. An additional problem arises because it ahs been found that the capsular polysaccharides (each of which determines the serotype and is the major protective antigen) when purified and used as a vaccine do not reliably induce protective antibody responses in children under two years of age, the age group which suffers the highest incidence of invasive pneumococcal infection and meningitis.

A modification of the approach using capsule antigens relies on conjugating the polysaccharide to a protein in order to derive an enhanced immune response, particularly by giving the response T-cell dependent character. This approach has been used in the development of a vaccine against Haemophilus influenzae. There are issues of cost concerning both the multi-polysaccharide vaccines and those based on conjugates.

A third approach is to look for other antigenic components which offer the potential to be vaccine candidates. In the present application we provide a group of proteins antigens which are secreted/exported proteins.

Thus, in a first aspect the present invention provides a vaccine comprising a *Streptococcus pneumoniae* protein or polypeptide having the sequence

MSEIKIINAKKIYHDVPVIENLNITIPKGSLFTLLGASGCGKTTLLRMIAGFNSIE
GGEFYFDDTKINNMEPSKRNIGMVFQNYAIFPHLTVRDNVAFGLMQKKVPKE
ELIQQTNKYLELMQIAQYADRKPDKLSGGQQQRVTLACALAVNPSVLLMDE
PLSNLEAKLRLDMRQAIREIQHEVGITTVYVTHDQEEAMAISDQIAVMKDGVI
QQIGRPKELYHKPANEFVATFIGRTNIIPANLEKRSDGAYIVFSDGYALRMPAL
DQVEEQAIHVSIRPEEFIKDESGDIEGTIRDSVYLGLNTDYFIETGFASKIQVSEE
STFEEDLQKGNRIRLRINTQKLNIFSADGSQNLIKGVNHGT.

As disussed herein, the proteins and polypeptides of the invention are useful as antigenic material. Such material can be "antigenic" and/or "immunogenic". Generally, "antigenic" is taken to mean that the protein or polypeptide is capable of being used to raise antibodies or indeed is capable of inducing an antibody response in a subject. "Immunogenic" is taken to mean that the protein or polypeptide is capable of eliciting a protective immune response in a subject. Thus, in the latter case, the protein or polypeptide may be capable of not only generating an antibody response and in addition non-antibody based immune responses.

The skilled person will appreciate that homologues or derivatives of the proteins or polypeptides of the invention will also find use in the context of the present invention, ie as antigenic/immunogenic material. Thus, for instance proteins or polypeptides which include one or more additions, deletions, substitutions or the like are encompassed by the present invention. In addition, it may be possible to replace one amino acid with another of similar "type". For instance replacing one hydrophobic amino acid with another. One can use a program such as the CLUSTAL program to compare amino acid sequences. This program compares amino acid sequences and finds the optimal alignment by inserting spaces in either sequence as appropriate. It is possible to calculate amino acid identity or similarity (identity plus conservation of amino acid type) for an optimal alignment. A program like BLASTx will align the longest stretch of similar sequences and assign a value to the fit. It is thus possible to obtain a comparison where several regions of similarity are found, each having a different score. Both types of analysis are contemplated in the present invention.

In the case of homologues and derivatives, the degree of identity with a protein or polypeptide as described herein is less important than that the homologue or derivative should retain its antigenicity or immunogenicity to streptoccocus pneumoniae. However, suitably, homologues or derivatives having at least 60% similarity (as discussed above) with the proteins or polypeptides described herein are provided.

Preferably, homologues or derivatives having at least 70% similarity, more preferably at least 80% similarity are provided. Most preferably, homologues or derivatives having at least 90% or even 95% similarity are provided.

In an alternative approach, the homologues or derivatives could be fusion proteins, incorporating moieties which render purification easier, for example by effectively tagging the desired protein or polypeptide. It may be necessary to remove the "tag" or it may be the case that the fusion protein itself retains sufficient antigenicity to be useful.

In addition the vaccine of the present invention may also comprise antigenic fragments of the proteins or polypeptides of the invention.

For fragments of the proteins or polypeptides described herein, the situation is slightly different. It is well known that is possible to screen an antigenic protein or polypeptide to identify epitopic regions, ie those regions which are responsible for the protein or polypeptide's antigenicity or immunogenicity. Methods for carrying out such screening are well known in the art. Thus, the fragments of the present invention should include one or more such epitopic regions or be sufficiently similar to such regions to retain their antigenic/immunogenic properties. Thus, for fragments according to the present invention the degree of identity is perhaps irrelevant, since they may be 100% identical to a particular part of a protein or polypeptide, homologue or derivative as described herein. The key issue, once again, is that the fragment retains the antigenic/immunogenic properties.

Thus, what is important for homologues, derivatives and fragments is that they possess at least a degree of the antigenicity/immunogenicity of the protein or polypeptide from which they are derived.

Gene cloning techniques may be used to provide a protein of the invention in substantially pure form. These techniques are disclosed, for example, in J. Sambrook *et al Molecular Cloning* 2^{nd} Edition, Cold Spring Harbor Laboratory Press (1989). In another aspect, the present invention provides a vaccine composition comprising one or more nucleic acid sequences selected from
i. 
ATGAGTGAGATCAAAATTATTAACGCCAAAAAAATCTACCACGATGTCCCT
GTTATTGAGAATTTGAACATTACAATTCCAAAAGGAAGTCTCTTTACCCTTC
TTGGAGCTTCAGGATGTGGAAAAACGACCCTTCTTCGTATGATTGCAGGTTT
CAACAGTATCGAAGGTGGAGAATTTTACTTCGATGATACAAAAATCAATAA
TATGGAACCCAGCAAACGCAATATCGGGATGGTTTTCCAAAACTACGCTATT
TTCCCACATTTGACTGTCCGAGACAACGTTGCTTTTGGTCTTATGCAAAAGA
AGGTTCCAAAAGAAGAATTGATTCAACAGACCAACAAGTATCTTGAACTCA
TGCAAATTGCTCAATATGCGGATCGAAAGCCCGATAAACTCAGTGGTGGAC
AACAACAACGTGTCACCTTGGCATGCGCCTTAGCGGTTAATCCAAGTGTTCT
CCTCATGGACGAGCCACTTAGTAATCTGGAGGCCAAACTTCGCTTGGATATG
CGTCAAGCCATCCGAGAAATCCAACACGAAGTGGGAATTACAACTGTTTAT
GTAACCCACGACCAAGAAGAAGCCATGGCTATTTCAGACCAAATTGCTGTT
ATGAAAGATGGGGTGATCCAACAAATCGGCCGACCAAAAGAACTCTATCAT
AAACCAGCTAATGAGTTTGTGGCAACCTTTATCGGACGCACAAATATTATCC
CTGCCAATCTTGAAAAACGGAGCGACGGCGCTTATATCGTCTTTTCAGATGG
CTATGCCCTTCGAATGCCAGCTCTTGATCAGGTTGAGGAGCAAGCTATTCAT
GTAAGCATTCGTCCCGAAGAGTTTATCAAAGATGAATCTGGAGATATTGAA
GGAACTATTAGAGATAGCGTCTATCTTGGACTAAATACGGATTATTTCATTG
AGACAGGTTTTGCCTCAAAAATTCAAGTTAGTGAAGAATCAACTTTTGAAG
AAGATCTACAAAAAGGCAATCGTATTCGTCTACGAATCAATACGCAAAAAT
TAAACATCTTTTCTGCAGATGGTTCCCAAAACCTGATAAAGGAGTCAACCA
TGGAACGTAA or its RNA equivalent;
ii. a sequence which is complementary to the sequences of (i);
iii. a sequence which codes for the same protein or polypeptide, as those sequences of (i) or (ii);
iv. a sequence which has at least 80% sequence identity with any of those of (i), (ii) and (iii);
v. a sequence which codes for an antigenic and/or immunogenic fragment of a protein or polypeptide encoded by the sequence of (a) wherein said fragment retains the antigenic and/or immunogenic properties of said protein or polypeptide.

The nucleic acid molecules of the invention may include a plurality of such sequences, and/or fragments. The skilled person will appreciate that the present invention can include novel variants of those particular novel nucleic acid molecules which are exemplified herein. Such variants are encompassed by the present invention. These may occur in nature, for example because of strain variation. For example, additions, substitutions and/or deletions are included. In addition, and particularly when utilising microbial expression systems, one may wish to engineer the nucleic acid sequence by making use of known preferred codon usage in the particular organism being used for expression. Thus, synthetic or non-naturally occurring variants are also included within the scope of the invention.

The term "RNA equivalent" when used above indicates that a given RNA molecule has a sequence which is complementary to that of a given DNA molecule (allowing for the fact that in RNA "U" replaces "T" in the genetic code).

When comparing nucleic acid sequences for the purposes of determining the degree of homology or identity one can use programs such as BESTFIT and GAP (both from the Wisconsin Genetics Computer Group (GCG) software package) BESTFIT, for example, compares two sequences and produces an optimal alignment of the most similar segments. GAP enables sequences to be aligned along their whole length and finds the optimal alignment by inserting spaces in either sequence as appropriate. Suitably, in the context of the present invention compare when discussing identity of nucleic acid sequences, the comparison is made by alignment of the sequences along their whole length.

Preferably, sequences which have substantial identity have at least 50% sequence identity, desirably at least 75% sequence identity and more desirably at least 90 or at least 95% sequence identity with said sequences. In some cases the sequence identity may be 99 % or above.

Desirably, the term "substantial identity" indicates that said sequence has a greater degree of identity with any of the sequences described herein than with prior art nucleic acid sequences.

It should however be noted that where a nucleic acid sequence of the present invention codes for at least part of a novel gene product the present invention includes within its scope all possible sequence coding for the gene product or for a novel part thereof.

The nucleic acid molecule may be in isolated or recombinant form. It may be incorporated into a vector and the vector may be incorporated into a host.

Therefore, for example, by using probes based upon the nucleic acid sequences provided herein, genes in *Streptococcus pneumoniae* can be identified. They can then be excised using restriction enzymes and cloned into a vector. The vector can be introduced into a suitable host for expression.

Nucleic acid molecules of the present invention may be obtained from *S.pneumoniae* by the use of appropriate probes complementary to part of the sequences of the nucleic acid molecules. Restriction enzymes or sonication techniques can be used to obtain appropriately sized fragments for probing.

Alternatively PCR techniques may be used to amplify a desired nucleic acid sequence. Thus the sequence data provided herein can be used to design two primers for use in PCR so that a desired sequence, including whole genes or fragments thereof, can be targeted and then amplified to a high degree. One primer will normally show a high degree of specificity for a first sequence located on one strand of a DNA molecule, and the other primer will normally show a high degree of specificity for a second sequence located pn the complementary strand of the DNA sequence and being spaced from the complementary sequence to the first sequence.

Typically primers will be at least 15-25 nucleotides long.

As a further alternative chemical synthesis may be used. This may be automated. Relatively short sequences may be chemically synthesised and ligated together to provide a longer sequence.

In the case of vaccines suitable additional excipients, diluents, adjuvants or the like may be included. Numerous examples of these are well known in the art.

The second aspect relates to the use of so-called DNA vaccines. The use of such DNA vaccines is described in the art. See for instance, Donnelly *et al, Ann. Rev. Immunol.,* 15:617-648 (1997).

The invention will now be described with reference to the following examples, which should not be construed as in any way limiting the invention. The examples refer to the figures in which:
- Figure 1:: shows the results of various DNA vaccine trials; and
- Figure 2:: shows the results of further DNA vaccine trials.

### EXAMPLE 1

The Genome sequencing of *Streptococcus pneumoniae* type 4 is in progress at the

Institute for Genomic Research (TIGR, Rockville, MD, USA). Up to now, the whole sequence has not been completed or published. On 21^{st} November 1997, the TIGR centre released some DNA sequences as contigs which are not accurate reflections of the finished sequence. These contigs can be downloaded from their Webster (www@tigr.org). We downloaded these contigs and created a local database using the application GCGToBLAST (Wisconsin Package Version 9.1, Genetics Computer Group (GCG), Madison, USA). This database can be searched with the FastA and TfastA procedures (using the method of Pearson and Lipman (*PNAS USA,* **85**:2444-2448 (1988)).

Using FastA and TfastA procedures, the local pneumococcus database was searched for putative leader sequence or anchor sequence features. Relevant sequences were used to interrogate for comparative novel sequences. These were:
(i) already described leader sequences of *Streptococcus pneumoniae* (from proteins NanA, NanB, LytA, PapA, pcpA, PsaA and PspA);
(ii) the leader sequence of Usp45, a secreted protein from *Lactococcus lactis*;
(iii) new hypothetical leader sequences derived from the searches in (i) and (ii);
(iv) the anchor motif LPxTG, a feature common to many Gram-positive bacteria surface proteins which are anchored by a mechanism involving the Sortase complex proteins.

Provided below is an example of this approach, with reference to the sequences derived from the database (see table 1).

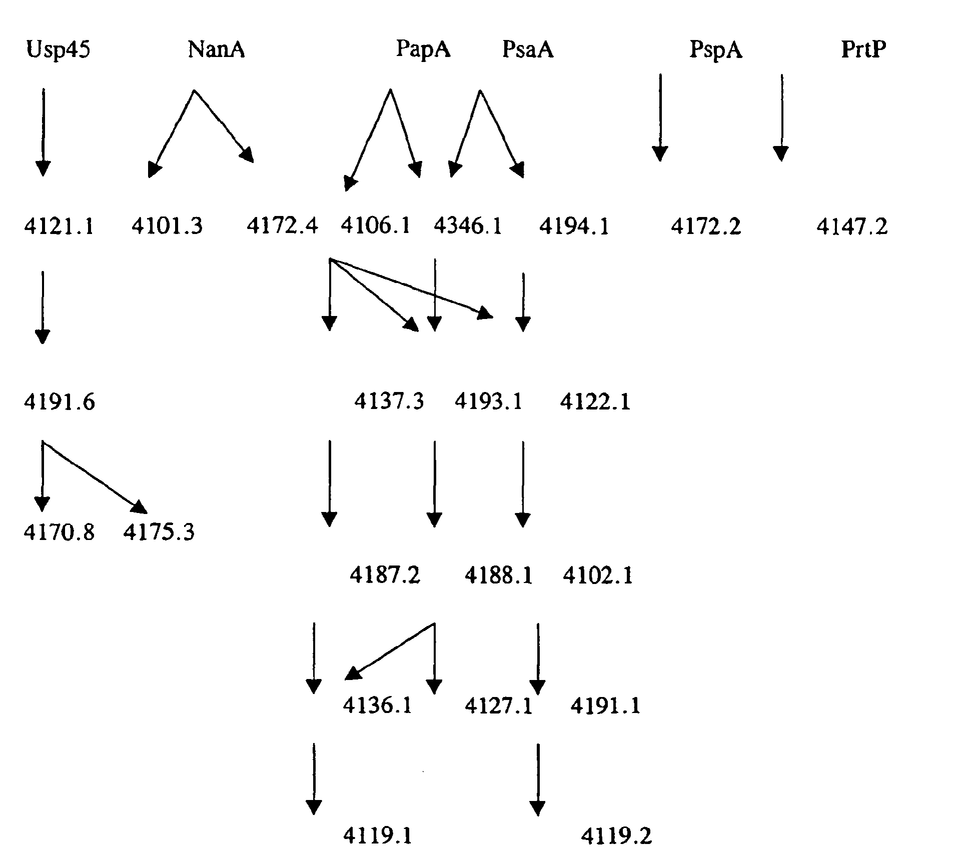

The protein leader sequences of different known exported proteins were used as a starting point for a search of the local pneumococcus database described above. The hypothetical proteins found with this search were then submitted to a Blast search in general databases such as EMBL, Swissprot etc. Proteins remaining unknown in the pneumococcus are kept and annotated. Then the search is performed again using the new potential protein leader sequence as a probe, using the TfastA procedure.

### Example 2: DNA vaccine trials

### pcDNA3.1+ as a DNA vaccine vector

### pcDNA3.1+

The vector chosen for use as a DNA vaccine vector was pcDNA3.1 (Invitrogen) (actually pcDNA3.1 +, the forward orientation was used in all cases but may be referred to as pcDNA3.1 here on). This vector has been widely and successfully employed as a host vector to test vaccine candidate genes to give protection against pathogens in the literature (Zhang, *et al. ,* Kurar and Splitter, Anderson *et al.).* The vector was designed for high-level stable and non-replicative transient expression in mammalian cells. pcDNA3.1 contains the ColE1 origin of replication which allows convenient high-copy number replication and growth in *E. coli*. This in turn allows rapid and efficient cloning and testing of many genes. The pcDNA3.1 vector has a large number of cloning sites and also contains the gene encoding ampicillin resistance to aid in cloning selection and the human cytomegalovirus (CMV) immediate-early promoter/enhancer which permits efficient, high-level expression of the recombinant protein. The CMV promoter is a strong viral promoter in a wide range of cell types including both muscle and immune (antigen presenting) cells. This is important for optimal immune response as it remains unknown as to which cells types are most important in generating a protective response *in vivo*. A T7 promoter upstream of the multiple cloning site affords efficient expression of the modified insert of interest and which allows *in vitro* transcription of a cloned gene in the sense orientation.

Zhang, D., Yang, X., Berry, J. Shen, C., McClarty, G. and Brunham, R.C. (1997) "DNA vaccination with the major outer-membrane protein genes induces acquired immunity to *Chlamydia trachomatis* (mouse pneumonitis) infection". *Infection and Immunity*, **176,** 1035-40.

Kurar, E. and Splitter, G.A. (1997) "Nucleic acid vaccination of *Brucella abortus* ribosomal *L7*/*L12* gene elicits immune response". *Vaccine,* **15**, 1851-57.

Anderson, R., Gao, X.-M., Papakonstantinopoulou, A., Roberts, M. and Dougan, G. (1996) "Immune response in mice following immunisation with DNA encoding fragment C of tetanus toxin". *Infection and Immunity,* **64**, 3168-3173.

### Preparation of DNA vaccines

Oligonucleotide primers were designed for each individual gene of interest derived using the LEEP system. Each gene was examined thoroughly, and where possible, primers were designed such that they targeted that portion of the gene thought to encode only the mature portion of the gene protein. It was hoped that expressing those sequences that encode only the mature portion of a target gene protein, would facilitate its correct folding when expressed in mammalian cells. For example, in the majority of cases primers were designed such that putative N-terminal signal peptide sequences would not be included in the final amplification product to be cloned into the pcDNA3.1 expression vector. The signal peptide directs the polypeptide precursor to the cell membrane via the protein export pathway where it is normally cleaved off by signal peptidase I (or signal peptidase II if a lipoprotein). Hence the signal peptide does not make up any part of the mature protein whether it be displayed on the surface of the bacteria surface or secreted. Where a N-terminal leader peptide sequence was not immediately obvious, primers were designed to target the whole of the gene sequence for cloning and ultimately, expression in pcDNA3.1.

Having said that, however, other additional features of proteins may also affect the expression and presentation of a soluble protein. DNA sequences encoding such features in the genes encoding the proteins of interest were excluded during the design of oligonucleotides. These features included:
1. LPXTG cell wall anchoring motifs.
2. LXXC ipoprotein attachment sites.
3. Hydrophobic C-terminal domain.
4. Where no N-terminal signal peptide or LXXC was present the start codon was excluded.
5. Where no hydrophobic C-terminal domain or LPXTG motif was present the stop codon was removed.

Appropriate PCR primers were designed for each gene of interest and any and all of the regions encoding the above features was removed from the gene when designing these primers. The primers were designed with the appropriate enzyme restriction site followed by a conserved Kozak nucleotide sequence (in all cases) GCCACC was used. The Kozak sequence facilitates the recognition of initiator sequences by eukaryotic ribosomes) and an ATG start codon upstream of the insert of the gene of interest. For example the forward primer using a BamH1 site the primer would begin GCGGGATCCGCCACCATG followed by a small section of the 5' end of the gene of interest. The reverse primer was designed to be compatible with the forward primer and with a Not1 restriction site at the 5' end in all cases (this site is TTGCGGCCGC).

### PCR primers

The following PCR primers were designed and used to amplify the truncated genes of interest.

### ID210

Forward Primer 5' CGGATCCGCCACCATGTCTTCTAATGAATCTGCCGATG 3'
Reverse Primer 5' TTGCGGCCGCCTGTTTAGATTGGATATCTGTAAAGACTT 3'

### 4172.5

Forward Primer 5'
CGCGGATCCGCCACCATGGATTTTCCTTCAAATTTGGAGG 3'
Reverse Primer 5' TTGCGGCCGCACCGTACTGGCTGCTGACT 3'

### ID211

Forward Primer 5'
CGGATCCGCCACCATGAGTGAGATCAAAATTATTAACGC 3'
Reverse Primer 5' TTGCGGCCGCCGTTCCATGGTTGACTCCT 3'

### 4197.4

Forward Primer 5' CGCGGATCCGCCACCATGTGGGACATATTGGTGGAAAC 3'
Reverse Primer 5' TTGCGGCCGCTTCACTTGAGCAAACTGAATCC 3'

### 4122.1

Forward Primer 5'
CGCGGATCCGCCACCATGTCACAAGAAAAAACAAAAAATGAA3'
Reverse Primer 5' TTGCGGCCGCATCGACGTAGTCTCCGCC 3'

### 4126.7

Forward Primer 5'
CGCGGATCCGCCACCATGCTGGTTGGAACTTTCTACTATCAAT3'
Reverse Primer 5' TTGCGGCCGCAACTTTCGTCCCTTTTTGG 3'

### 4188.11

Forward Primer 5' CGCGGATCCGCCACCATGGGCAATTCTGGCGGAA 3'
Reverse Primer 5' TTGCGGCCGCTTGTTTCATAGCTTTTTTGATTGTT 3'

### ID209

Forward Primer 5'
CGCGGATCCGCCACCATGCTATTGATACGAAATGCAGGG3'
Reverse Primer 5' TTGCGGCCGCAACATAATCTAGTAAATAAGCGTAGCC 3'

### ID215

Forward Primer 5' CGCGGATCCGCCACCATGACGGCGACGAATTTTC 3'
Reverse Primer 5' TTGCGGCCGCTTAATTCGTTTTTGAACTAGTTGCT 3'

### 4170.4

Forward Primer 5'
CGCGGATCCGCCACCATGGCTGTTTTTCTTCGCTATCATG 3'
Reverse Primer 5' TTGCGGCCGCTTTCTTCAACAAACCTTGTTCTTG 3'

### 4193.1

Forward Primer 5'
CGCGGATCCGCCACCATGGGTAACCGCTCTTCTCGTAAC3'
Reverse Primer 5' TTGCGGCCGCGCTTCCATCAAGGATTTTAGC 3'

### Cloning

The insert along with the flanking features described above was amplified using PCR against a template of genomic DNA isolated from type 4 S. *pneumoniae* strain 11886 obtained from the National Collection of Type Cultures. The PCR product was cut with the appropriate restriction enzymes and cloned in to the multiple cloning site of pcDNA3.1 using conventional molecular biological techniques. Suitably mapped clones of the genes of interested were cultured and the plasmids isolated on a large scale (> 1.5 mg) using Plasmid Mega Kits (Qiagen). Successful cloning and maintenance of genes was confirmed by restriction mapping and sequencing ~ 700 base pairs through the 5' cloning junction of each large scale preparation of each construct.

### Strain validation

A strain of type 4 was used in cloning and challenge methods which is the strain from which the S. *pneumoniae* genome was sequenced. A freeze dried ampoule of a homogeneous laboratory strain of type 4 *S. pneumoniae* strain NCTC 11886 was obtained from the National Collection of Type Strains. The ampoule was opened and the cultured re suspended with 0.5 ml of tryptic soy broth (0.5 % glucose, 5% blood). The suspension was subcultured into 10 ml tryptic soy broth (0.5% glucose, 5% blood) and incubated statically overnight at 37°C. This culture was streaked on to 5 % blood agar plates to check for contaminants and confirm viability and on to blood agar slopes and the rest of the culture was used to make 20% glycerol stocks. The slopes were sent to the Public Health Laboratory Service where the type 4 serotype was confirmed.

A glycerol stock of NCTC 11886 was streaked on a 5% blood agar plate and incubated overnight in a CO2 gas jar at 37°C. Fresh streaks were made and optochin sensitivity was confirmed.

### Pneumococcal challenge

A standard inoculum of type 4 *S. pneumoniae* was prepared and frozen down by passaging a culture of pneumococcus 1x through mice, harvesting from the blood of infected animals, and grown up to a predetermined viable count of around 10⁹ cfu/ml in broth before freezing down. The preparation is set out below as per the flow chart.

Streak pneumococcal culture and confirm identity
|
V
Grow over-night culture from 4-5 colonies on plate above
|
V
Animal passage pneumococcal culture
(i.p. injection of cardiac bleed to harvest)
|
V
Grow over-night culture from animal passaged pneumococcus

|
V

Grow day culture (to pre-determined optical density) from over-night of animal
passage and freeze down at -70°C - This is standard minimum

|
V

Thaw one aliquot of standard inoculum to viable count

|
V

Use standard inoculum to determine effective dose (called Virulence Testing)

|
V

All subsequent challenges - use standard inoculum to effective dose

An aliquot of standard inoculum was diluted 500x in PBS and used to inoculate the mice.

Mice were lightly anaesthetised using halothane and then a dose of 1.4 x 10⁵ cfu of pneumococcus was applied to the nose of each mouse. The uptake was facilitated by the normal breathing of the mouse, which was left to recover on its back.

### S. pneumoniae vaccine trials

Vaccine trials in mice were carried out by the administration of DNA to 6 week old CBA/ca mice (Harlan, UK). Mice to be vaccinated were divided into groups of six and each group was immunised with recombinant pcDNA3.1 + plasmid DNA containing a specific target-gene sequence of interest. A total of 100 µg of DNA in Dulbecco's PBS (Sigma) was injected intramuscularly into the tibialis anterior muscle of both legs (50 µl in each leg). A boost was carried using the same procedure 4 weeks later. For comparison, control groups were included in all vaccine trials. These control groups were either unvaccinated animals or those administered with non-recombinant pcDNA3.1 + DNA (sham vaccinated) only, using the same time course described above. 3 weeks after the second immunisation, all mice groups were challenged intra-nasally with a lethal dose of *S. pneumoniae* serotype 4 (strain NCTC 11886). The number of bacteria administered was monitored by plating serial dilutions of the inoculum on 5% blood agar plates. A problem with intranasal immunisations is that in some mice the inoculum bubbles out of the nostrils, this has been noted in results table and taken account of in calculations. A less obvious problem is that a certain amount of the inoculum for each mouse may be swallowed. It is assumed that this amount will be the same for each mouse and will average out over the course of innoculations. However, the sample sizes that have been used are small and this problem may have significant effects in some experiments. All mice remaining after the challenge were killed 3 or 4 days after infection. During the infection process, challenged mice were monitored for the development of symptoms associated with the onset of *S. pneumoniae* induced-disease. Typical symptoms in an appropriate order included piloerection, an increasingly hunched posture, discharge from eyes, increased lethargy and reluctance to move. The latter symptoms usually coincided with the development of a moribund state at which stage the mice were culled to prevent further suffering. These mice were deemed to be very close to death, and the time of culling was used to determine a survival time for statistical analysis. Where mice were found dead, the survival time was taken as the last time point when the mouse was monitored alive.

### Interpretation of Results

A positive result was taken as any DNA sequence that was cloned and used in challenge experiments as described above which gave protection against that challenge. Protection was taken as those DNA sequences that gave statistically significant protection (to a 95% confidence level (p<0.05)) and also those which were marginal or close to significant using Mann-Whitney or which show some protective features for example there were one or more outlying mice or because the time to the first death was prolonged. It is acceptable to allow marginal or non-significant results to be considered as potential positives when it is considered that the clarity of some of the results may be clouded by the problems associated with the administration of intranasal infections.

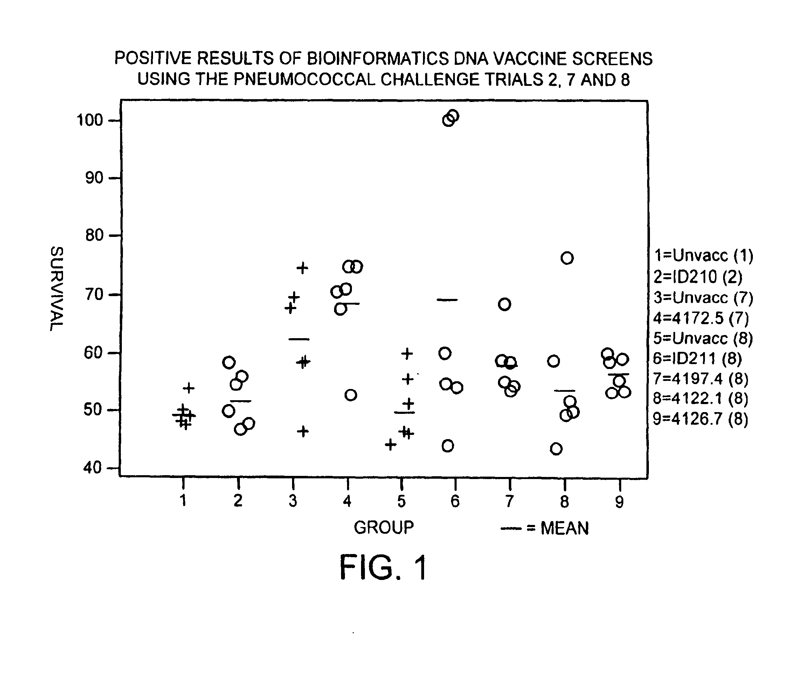

**Results for vaccine trials 2, 7 and 8 (see figure 1)**

| **Mouse number** | **Mean survival times (hours)** | | | | | | | | |
|---|---|---|---|---|---|---|---|---|---|
| | **Unvacc control (2)** | **ID210 (2)** | **Unvacc control (7)** | **4172.5 (7)** | **Unvacc control (8)** | **ID211 (8)** | **4197.4 (8)** | **4122.1 (8)** | **4126.7 (8)** |
| 1 | 49.0 | 55.0 | 59.6 | 72.6 | 45.1 | 102.3T | 60.1 | 50.6 | 60.0 |
| 2 | 51.0 | 46.5 | 47.2 | 67.9 | 50.8 | 55.5 | 54.9 | 77.2 | 60.0 |
| 3 | 49.0 | 49.0 | 59.6 | 54.4 | 60.4 | 60.6* | 68.4 | 60.3 | 54.8 |
| 4 | 55.0 | 59.0 | 70.9 | 75.3 | 55.2 | 45.3 | 60.1 | 50.6 | 52.6 |
| 5 | 49.0 | 55.0 | 68.6* | 70.9 | 45.1 | 55.5 | 54.9 | 50.6* | 54.8 |
| 6 | 49.0 | 49.0 | 76.0 | 75.3 | 45.1 | 102.3T | 52.7 | 44.9 | 60 |
| **Mean** | **50.3** | **52.3** | **63.6** | **69.4** | **50.2** | **70.2** | **58.5** | **55.7** | **57.0** |
| **sd** | **2.4** | **4.8** | **10.3** | **7.9** | **6.4** | **25.3** | **5.7** | **11.6** | **3.4** |
| **p value 1** | **-** | **0.3333** | **-** | **0.2104** | **-** | **0.0215** | **0.0621** | **0.4038** | **0.0833** |

| | | | | | | | | | |
|---|---|---|---|---|---|---|---|---|---|
| * - bubbled when dosed so may not have received full inoculum. T - terminated at end of experiment having no symptoms of infection. Numbers in brackets - survival times disregarded assuming incomplete dosing p value 1 refers to significance tests compared to unvaccinated controls | | | | | | | | | |

### Statistical Analyses.

Trial 2 - The group vaccinated with ID210 also had a longer mean survival time than the unvaccinated controls but the results are not statistically significant.
Trial 7 - The group vaccinated with 4172.5 showed much greater survival times than unvaccinated controls although the differences were not statistically significant.
Trial 8 - The group vaccinated with ID211 survived significantly longer than unvaccinated controls. 4197.4, 4122.1 and 4126.7 vaccinated groups showed longer mean survival times than the unvaccinated group but the results were not statistically significant. The 4197.4 and 4126.7 groups also showed a prolonged time to the first death and the 4122.1 group showed 1 outlying result.

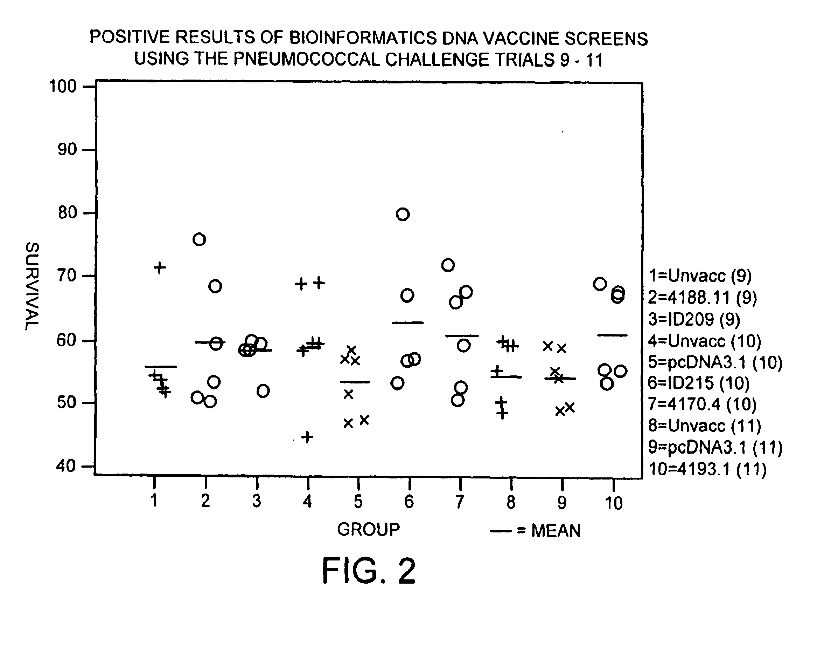

### Results of pneumococcal challenge DNA vaccination trials 9-11 (see figure 2)

| **Mouse number** | **Mean survival times (hours)** | | | | | | | | | |
|---|---|---|---|---|---|---|---|---|---|---|
| | **Unvacc control (9)** | **4188.1 1 (9)** | **ID209 (9)** | **Unvacc control (10)** | **pcDNA3.1 + (10)** | **ID215 (10)** | **4170. 4 (10)** | **Unvacc control (11)** | **pcDNA3.1 + (11)** | **4193.1 (11)** |
| 1 | (98.5)T | 69.4 | 60.2 | 68.4 | 58.6 | 79.2 | 68.1 | 60.0 | 53.2 | 54.8 |
| 2 | 53.4 | 53.7 | 60.2 | 59.0 | 58.6 | 54.2 | 58.6 | 50.0 | 50.4 | 54.8 |
| 3 | 53.4 | 51.2 | 60.2 | 59.0 | 50.8 | (103.2)*T | 50.9 | 60.0 | 55.4 | 68.7* |
| 4 | 53.4 | 75.0 | (98.0)*T | 45.1* | 58.6 | 58.8 | 72.1 | 55.0 | 60.6 | 54.8 |
| 5 | 70.8 | 51.2 | 60.2 | 68.4 | 46.5 | 68.3 | 68.1 | 60.0 | 50.4 | 68.7 |
| 6 | 53.4 | 61.2 | 52.9 | 59.0 | 48.9 | 58.8 | 54.0 | 50.0 | 60.6 | 68.7* |
| **Mean** | **56.9** | **60.3** | **58.8** | **59.8** | **53.6** | **63.9** | **62.0** | **55.8** | **55.1** | **61.7** |
| **Sd** | **7.8** | **10.0** | **3.3** | **8.5** | **5.6** | **10.0** | **8.7** | **5.0** | **4.6** | **7.6** |
| **p value** 1 | **-** | **0.3894** | **0.2519** | **-** | **0.0307** | **<30.0** | **<39. 0** | **-** | **-** | **0.1837** |
| **p value 2** | **-** | **-** | **-** | **-** | **-** | **0.0168** | **0.031 6** | **-** | **-** | **0.0829** |

| | | | | | | | | | | |
|---|---|---|---|---|---|---|---|---|---|---|
| * - bubbled when dosed so may not have received full inoculum. T - terminated at end of experiment having no symptoms of infection. Numbers in brackets - survival times disregarded assuming incomplete dosing p value I refers to significance tests compared to unvaccinated controls p value 2 refers to significance tests compared to pcDNA3.1 + vaccinated controls | | | | | | | | | | |

### Statistical Analyses.

Trial 9 - Although not statistically significant the groups vaccinated with 4188.11 and ID209 did have noticeably higher mean survival times than unvaccinated controls.
Trial 10 - The unvaccinated control group survived for a significantly longer period than the pcDNA3.1 + vaccinated group. The groups vaccinated with ID215 and 4170.4 showed statistically significant longer survival times compared to the sham vaccinated group (p=0.0168 and 0.0316) but not compared to the unvaccinated group.
Trial 11 - The group vaccinated with 4193.1 was the most promising and survived an average of 6.5 hours longer than the pcDNA3.1 + vaccinated group and 6 hours longer than the unvaccinated group although the results were not statistically significant.

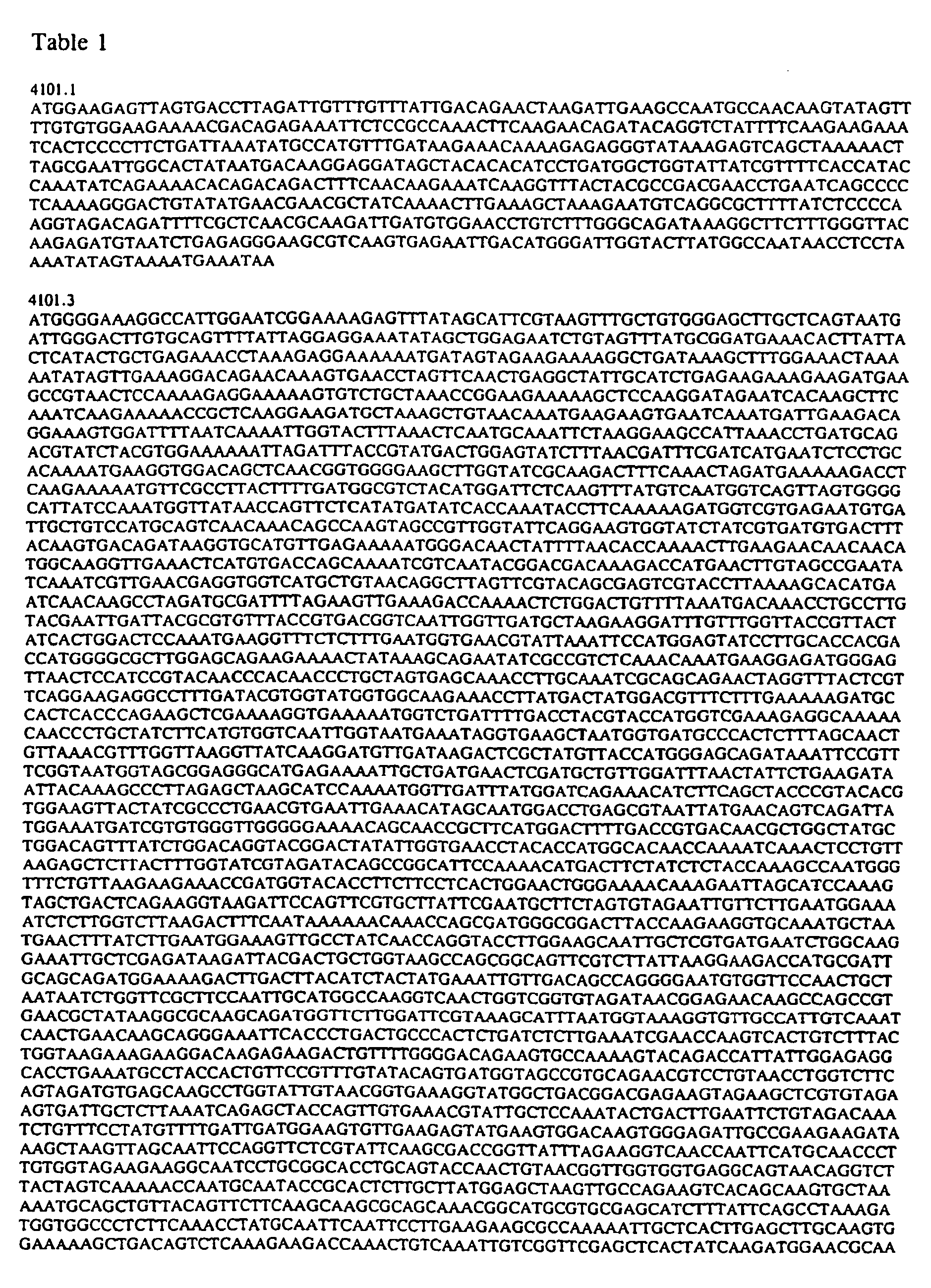

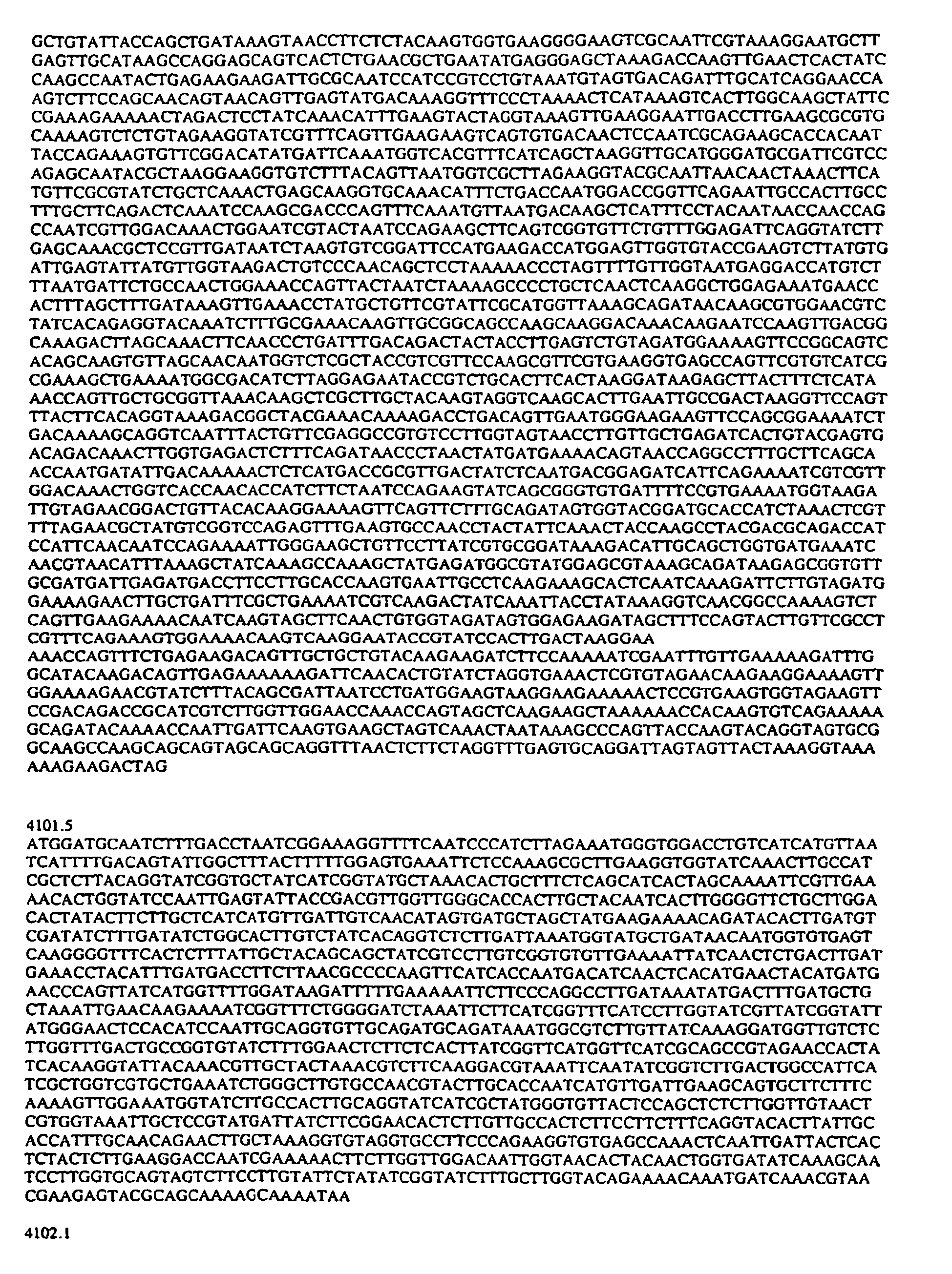

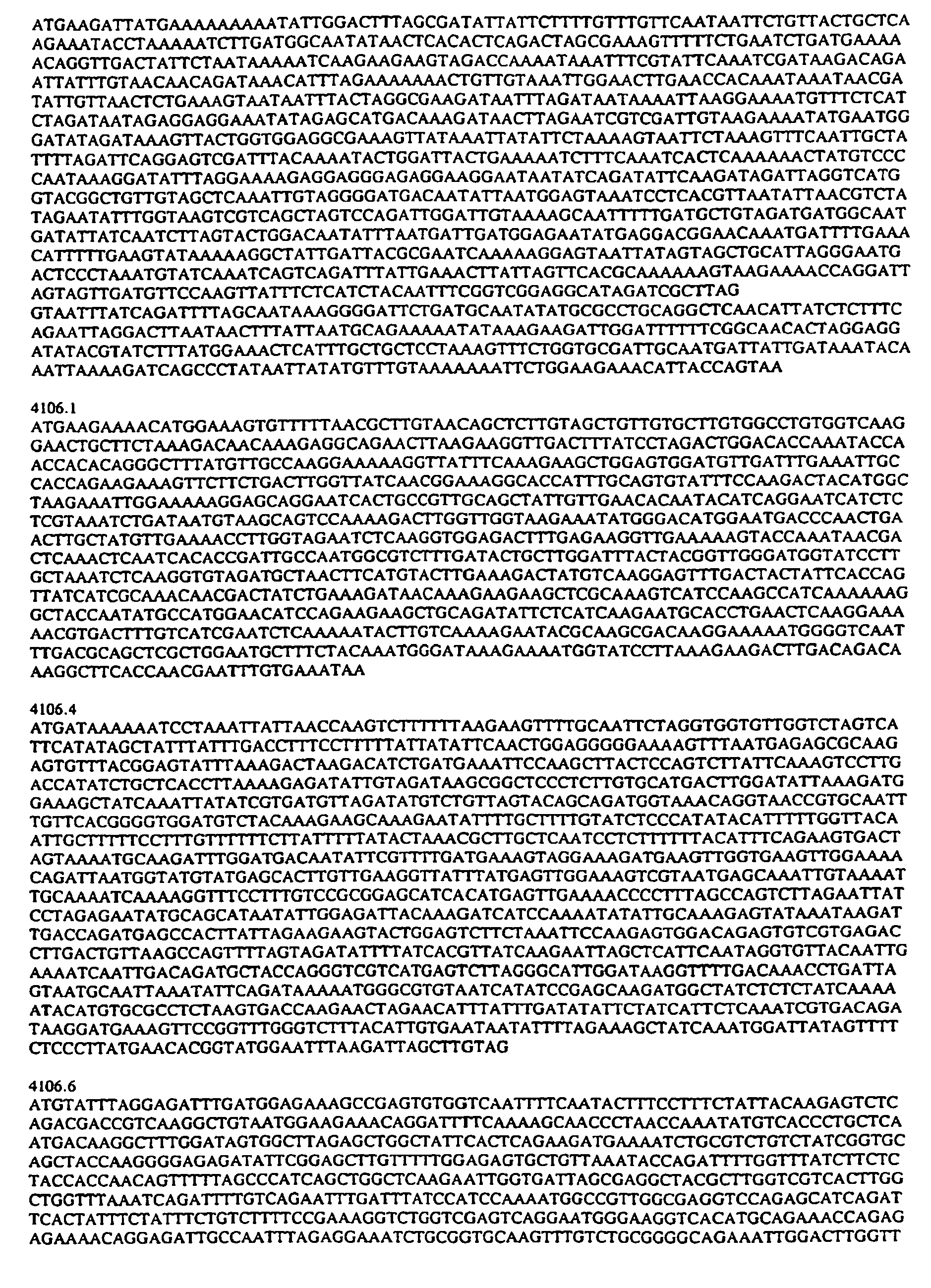

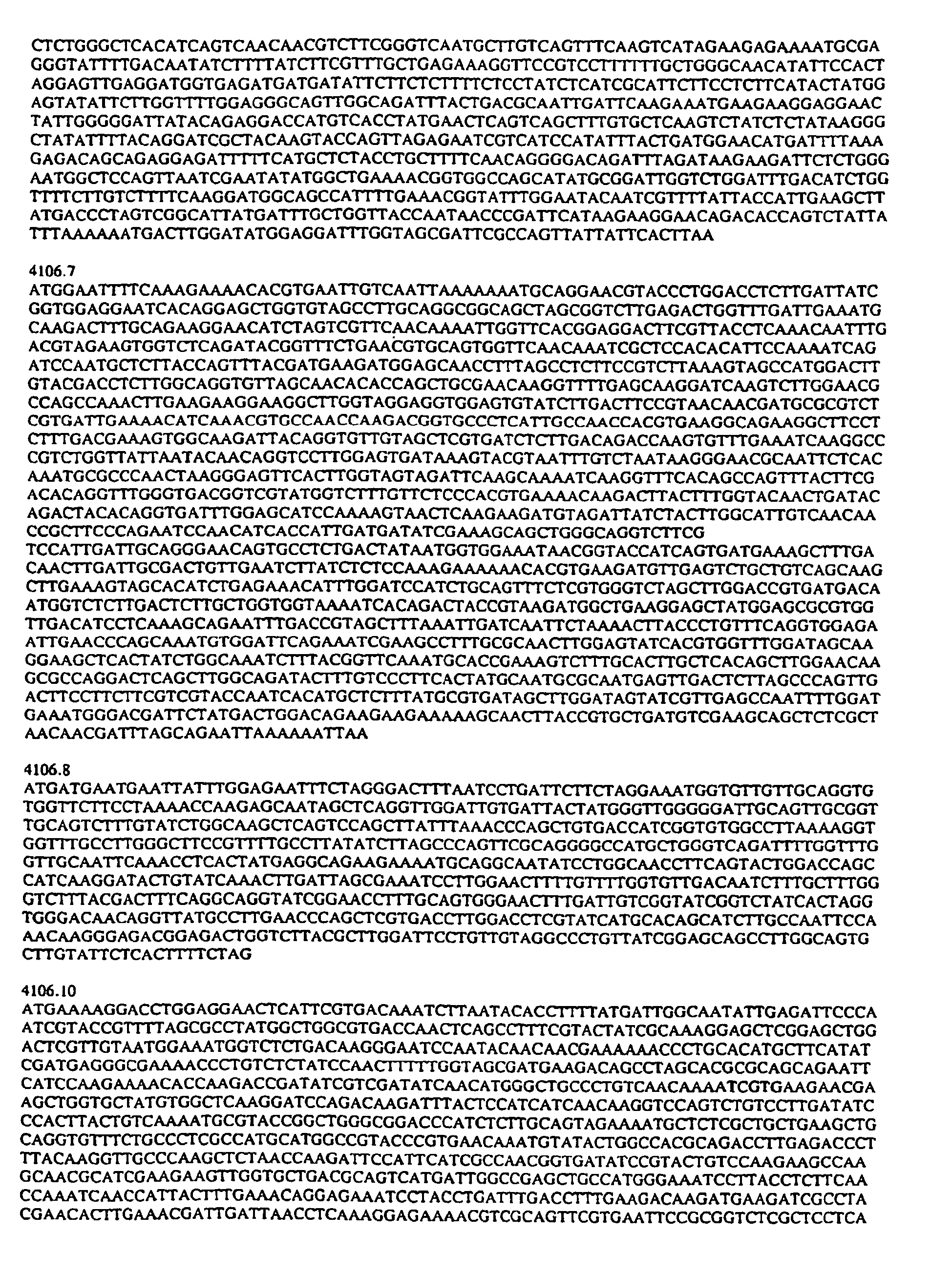

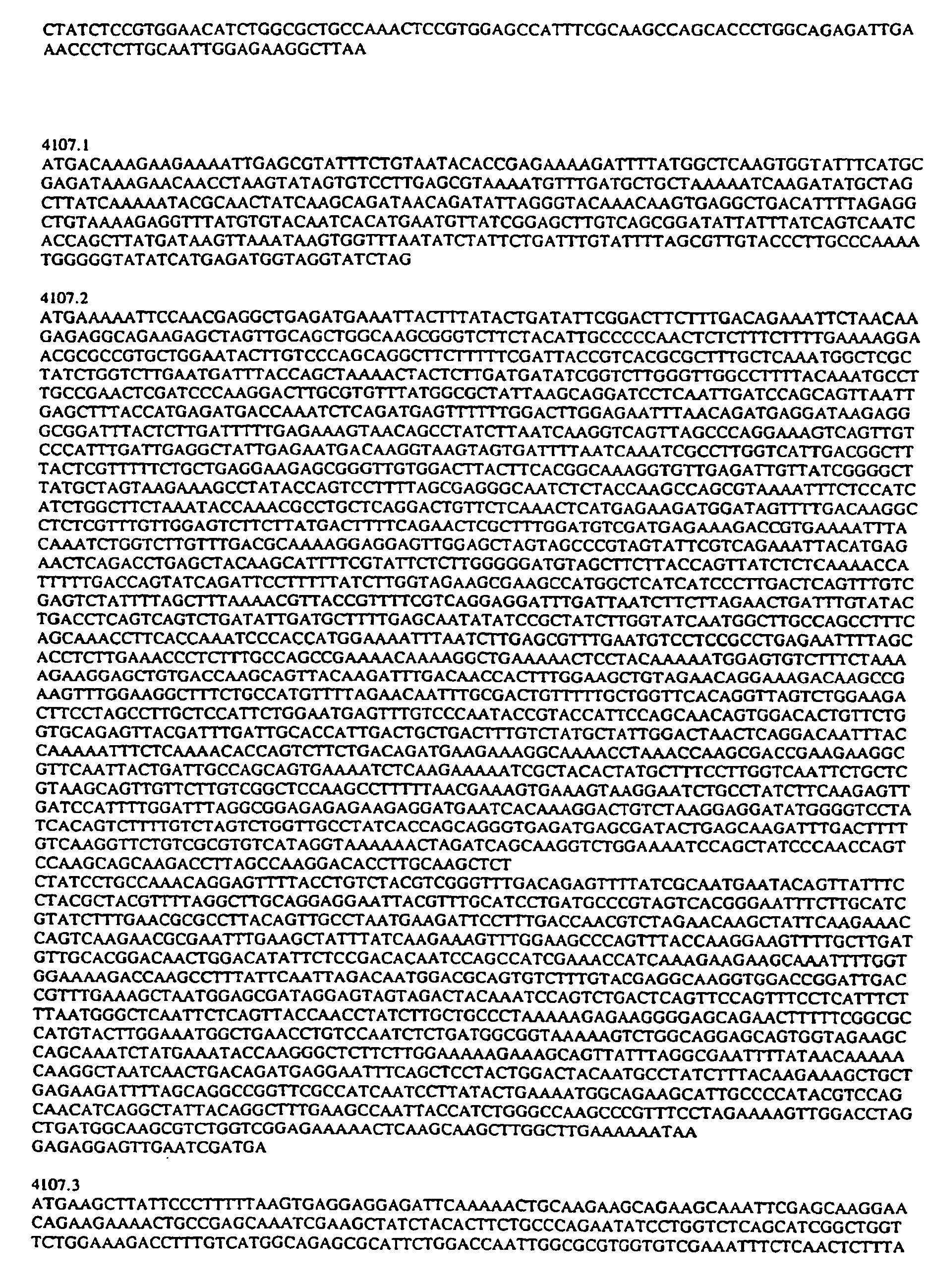

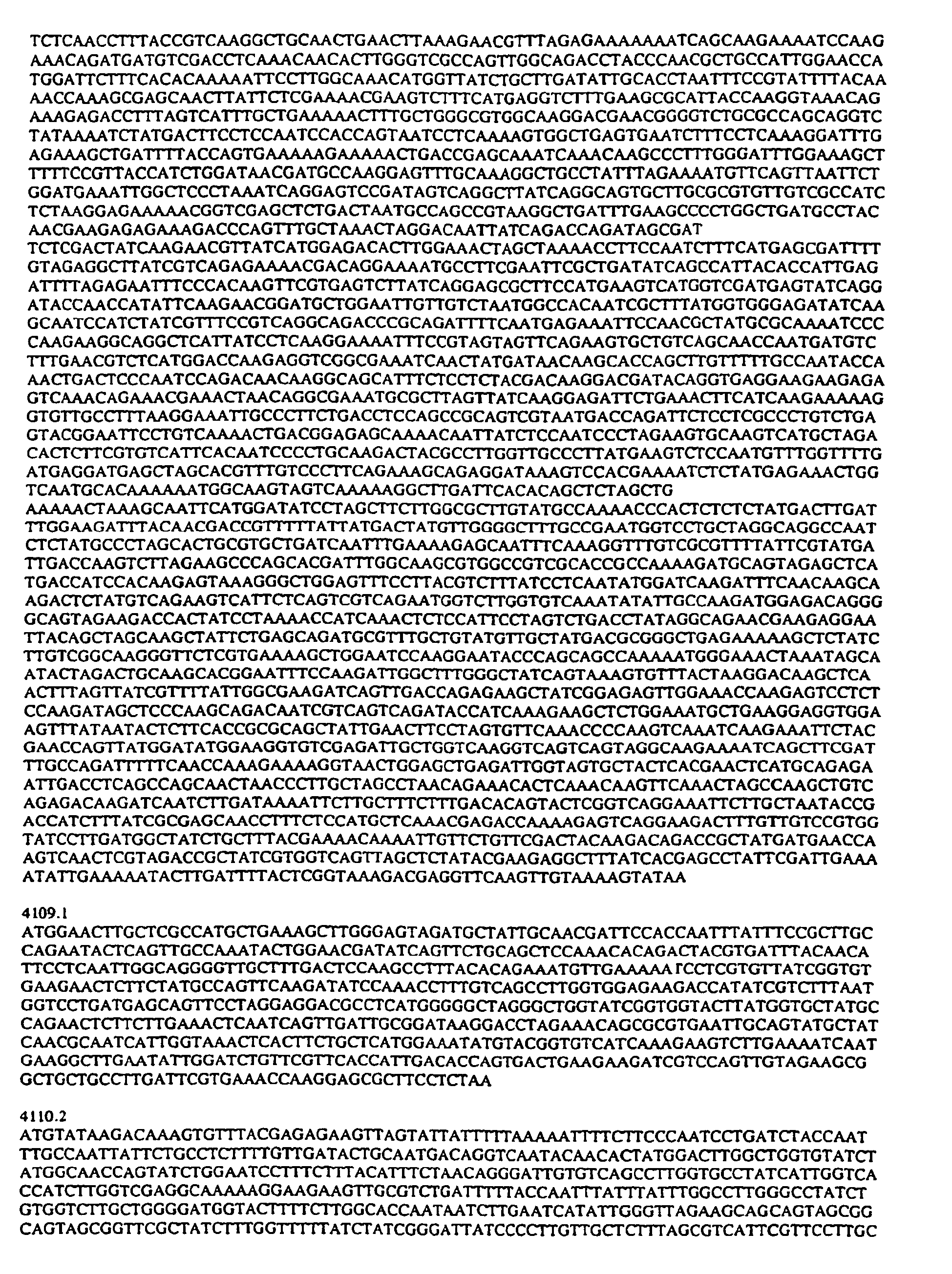

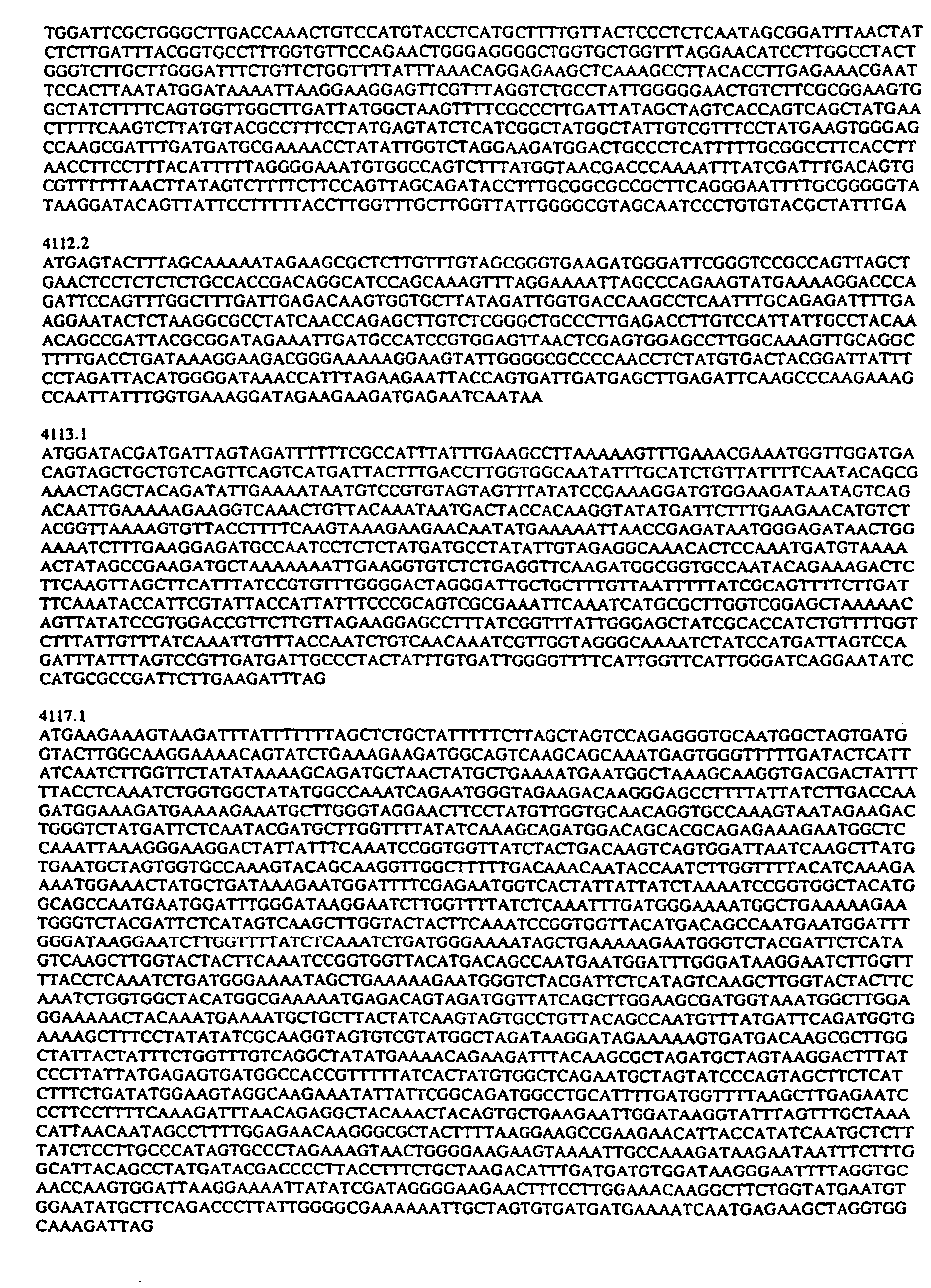

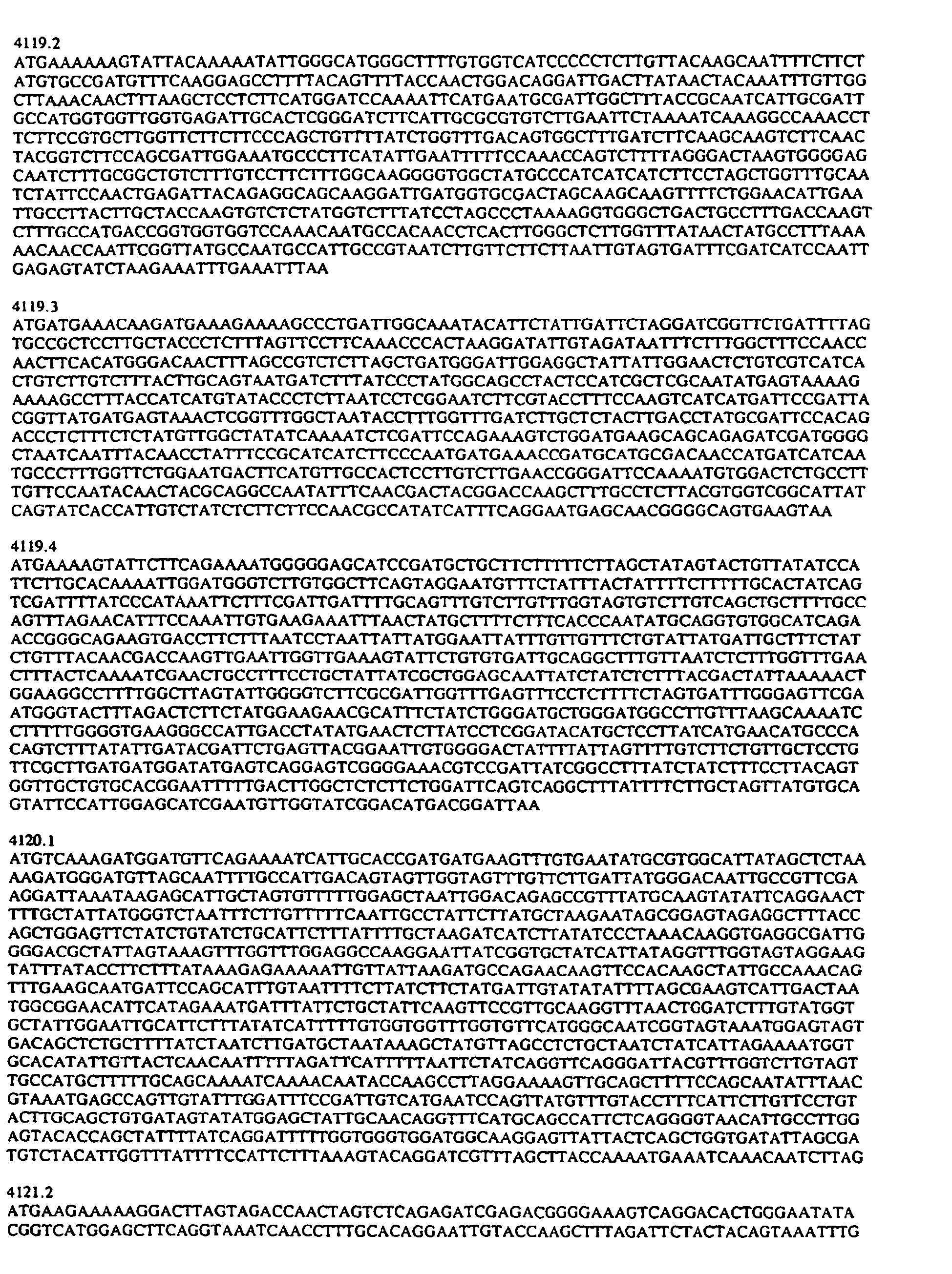

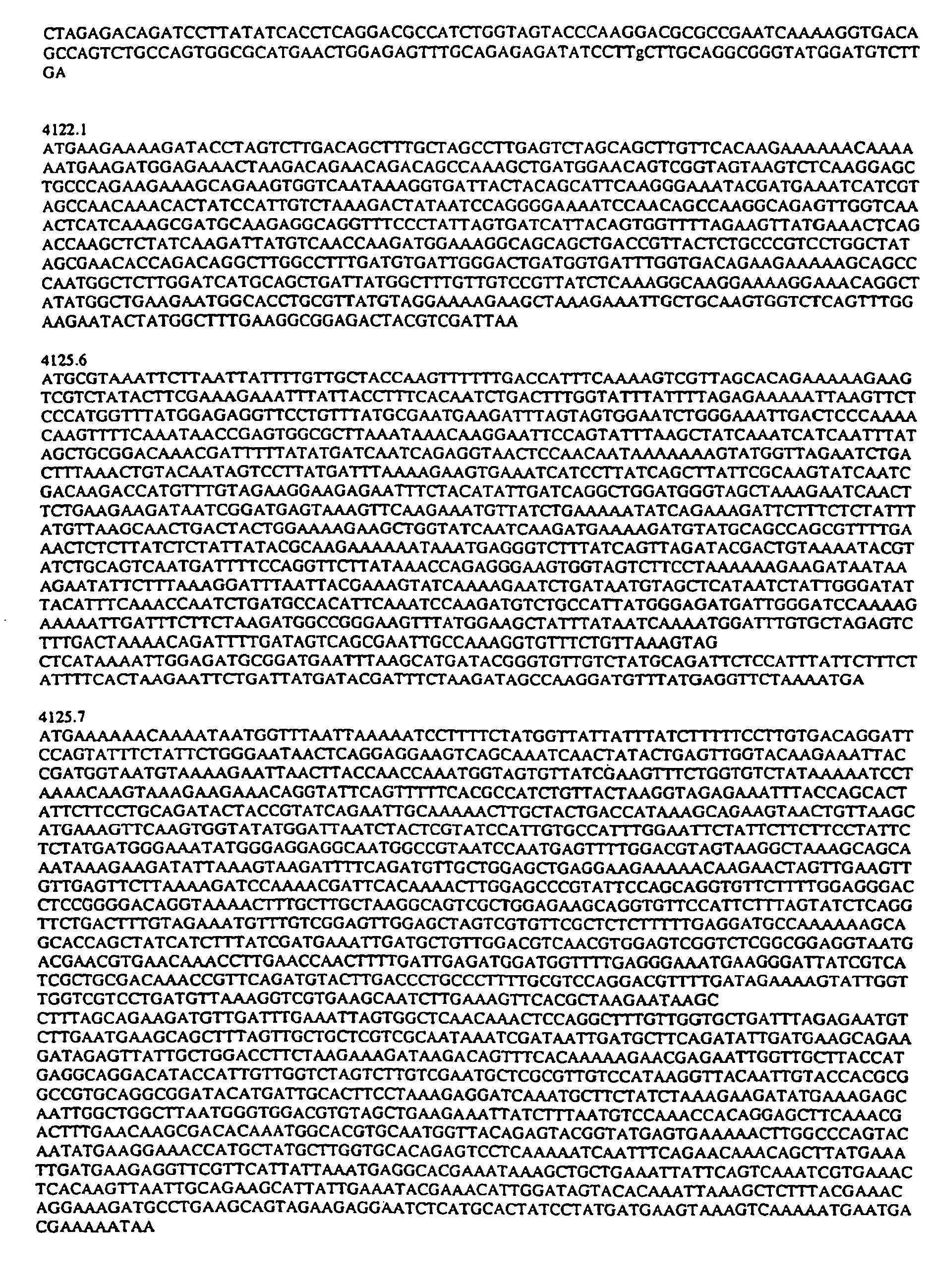

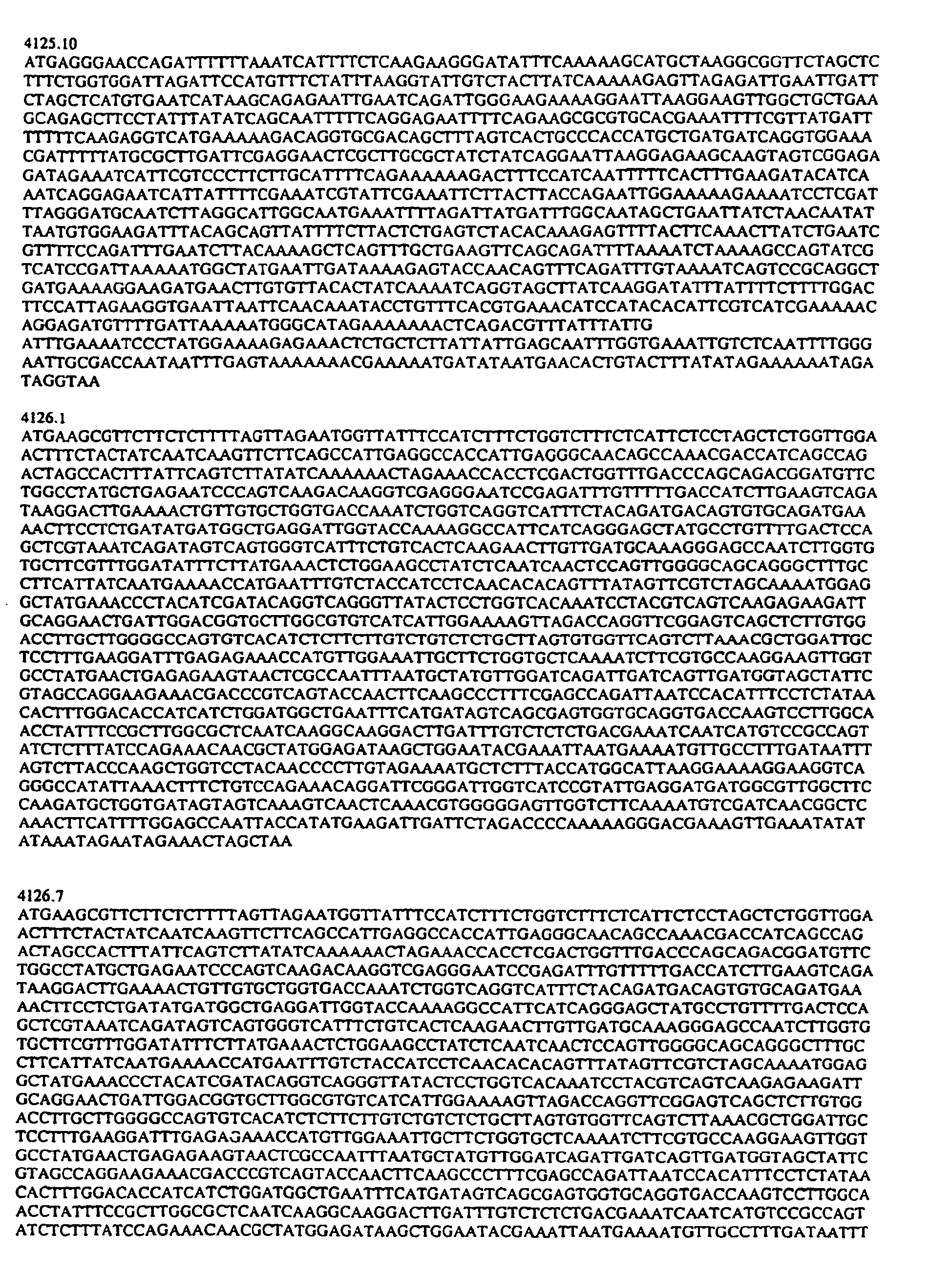

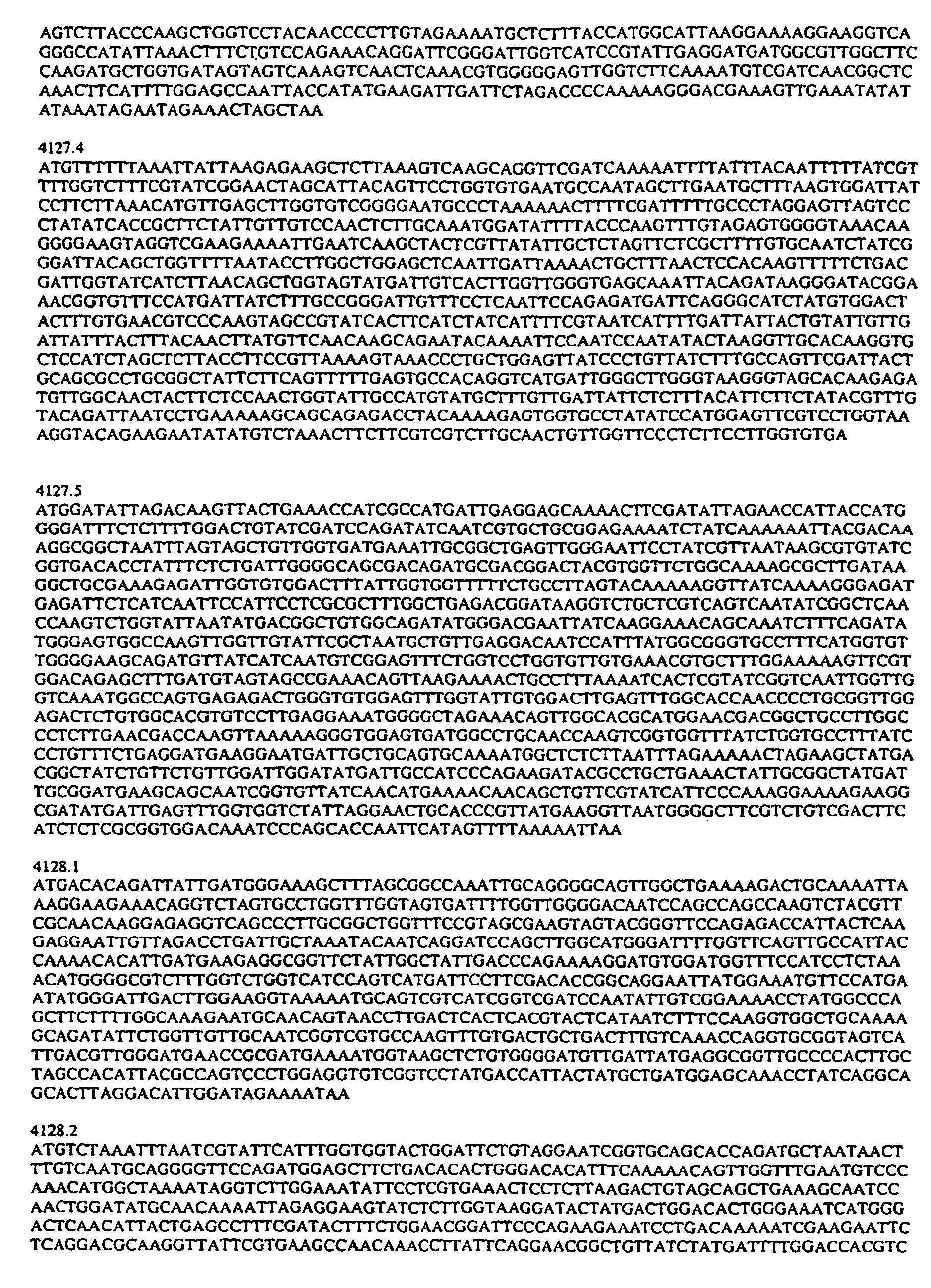

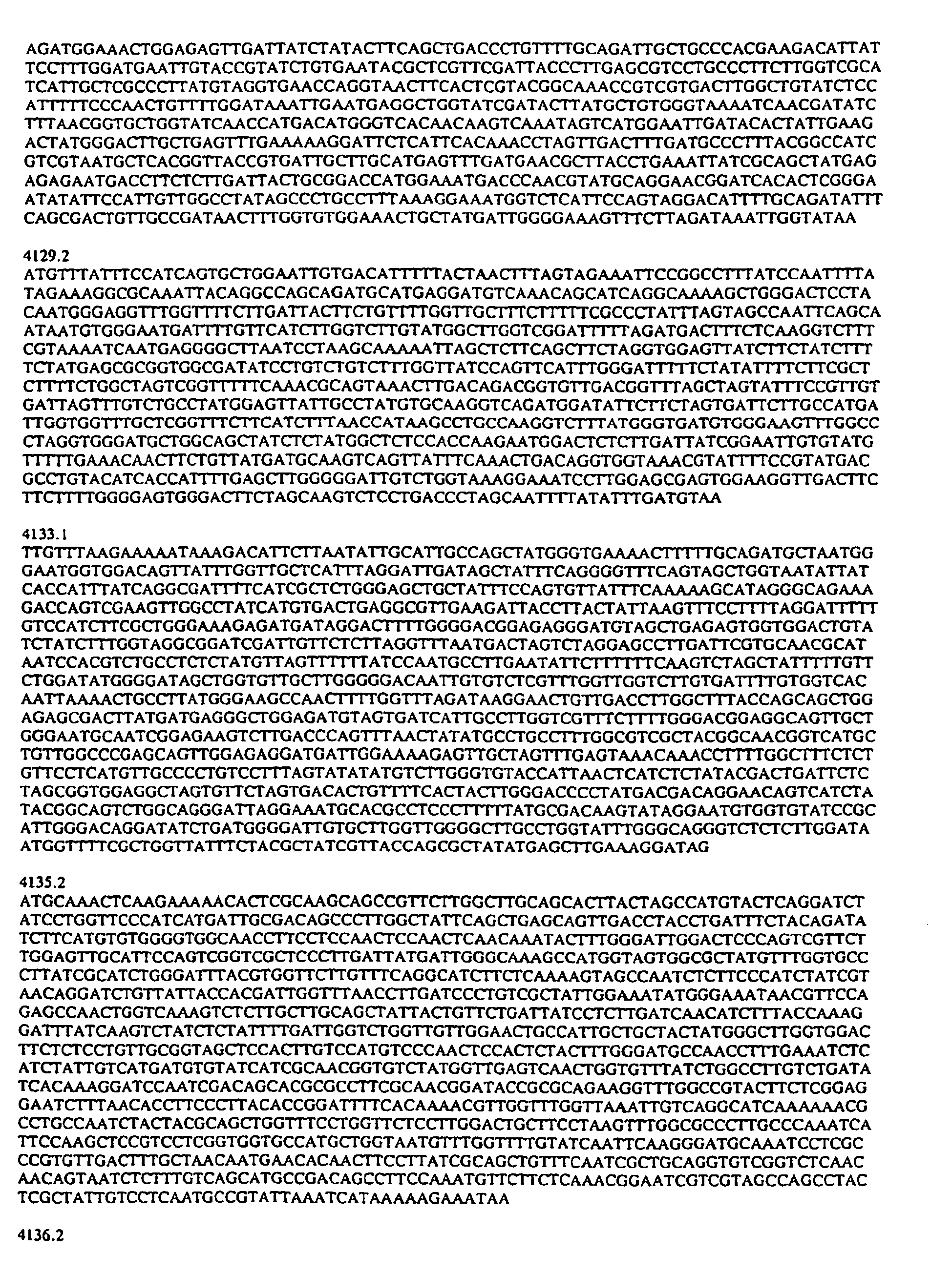

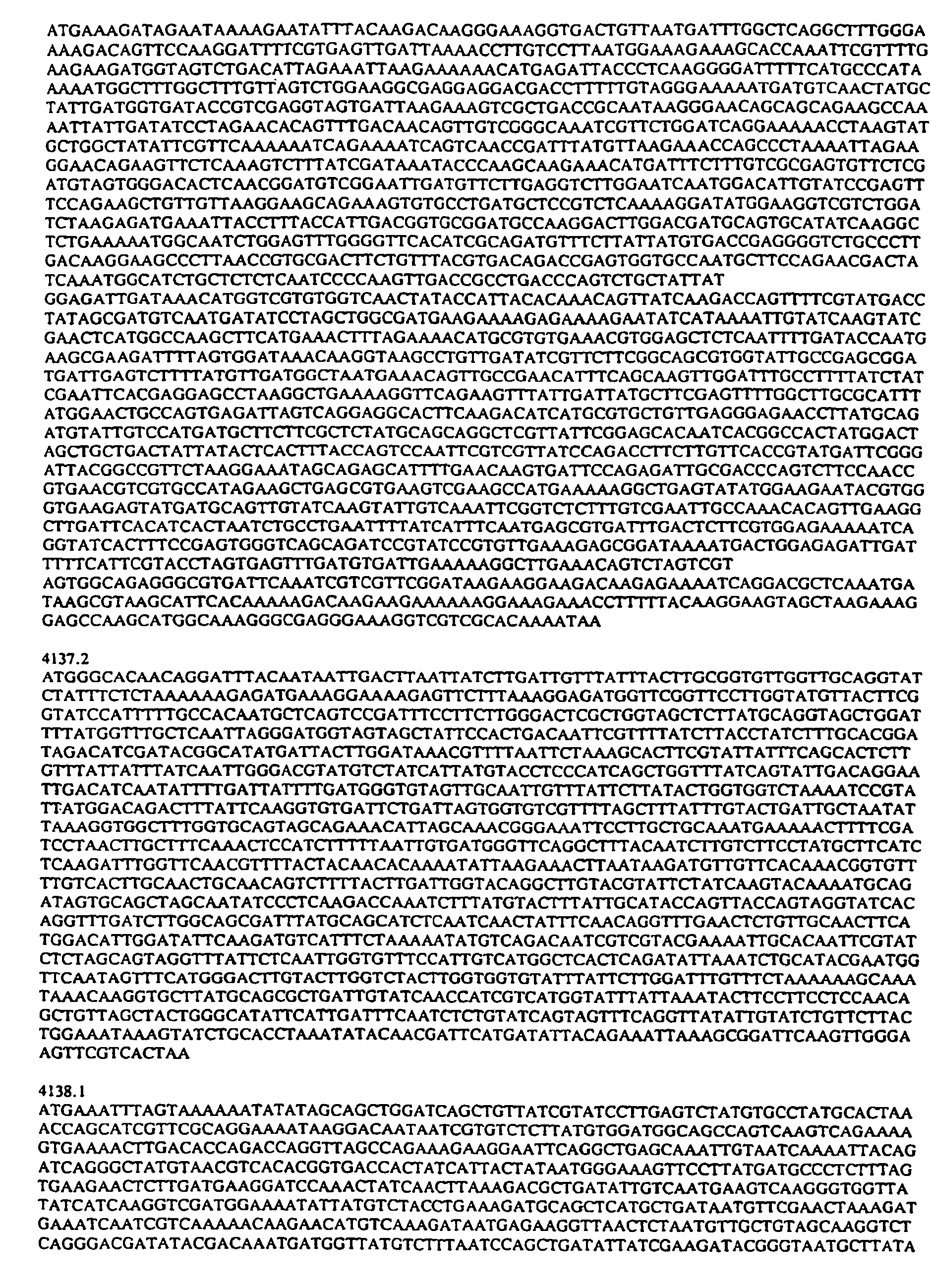

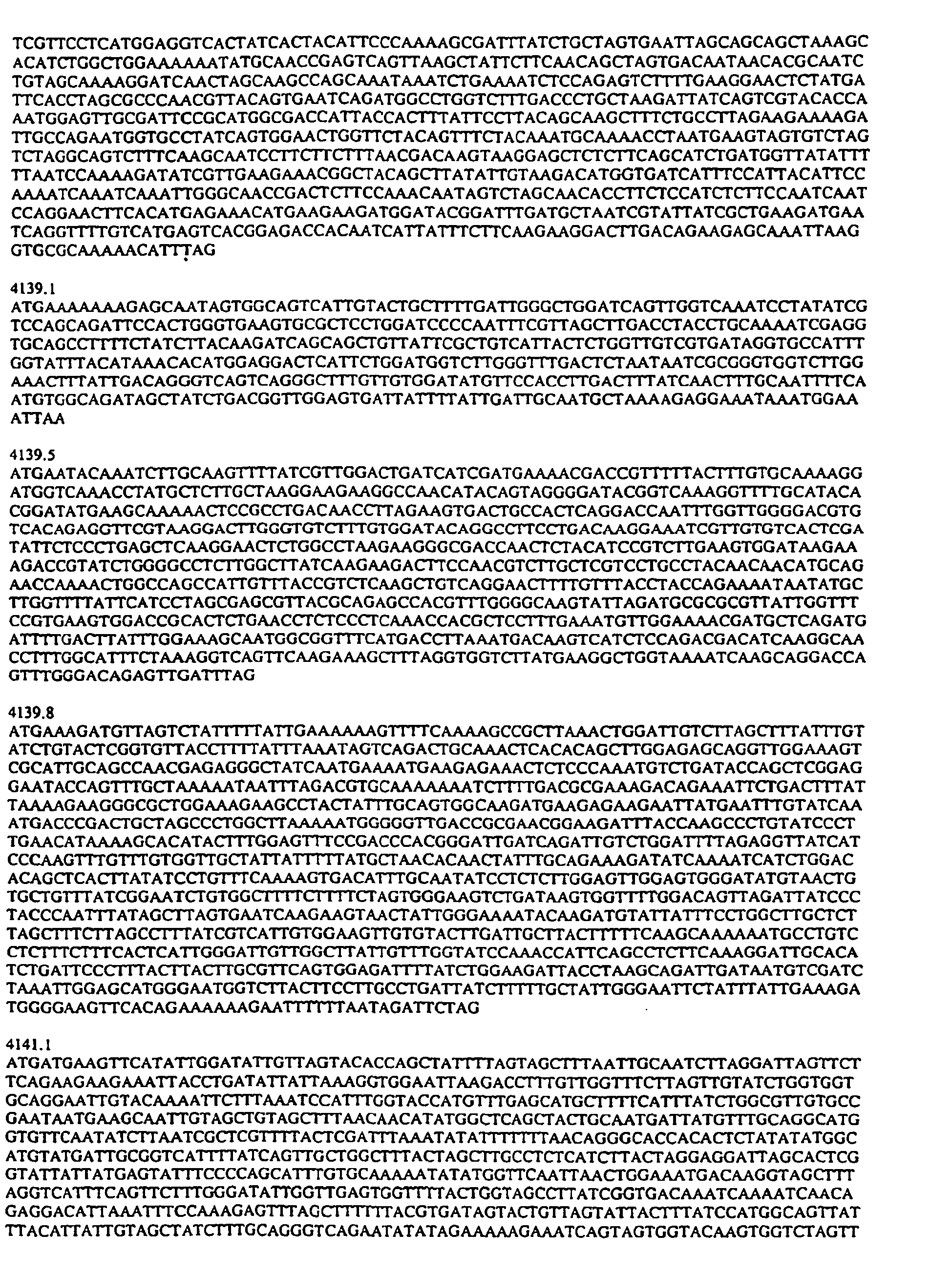

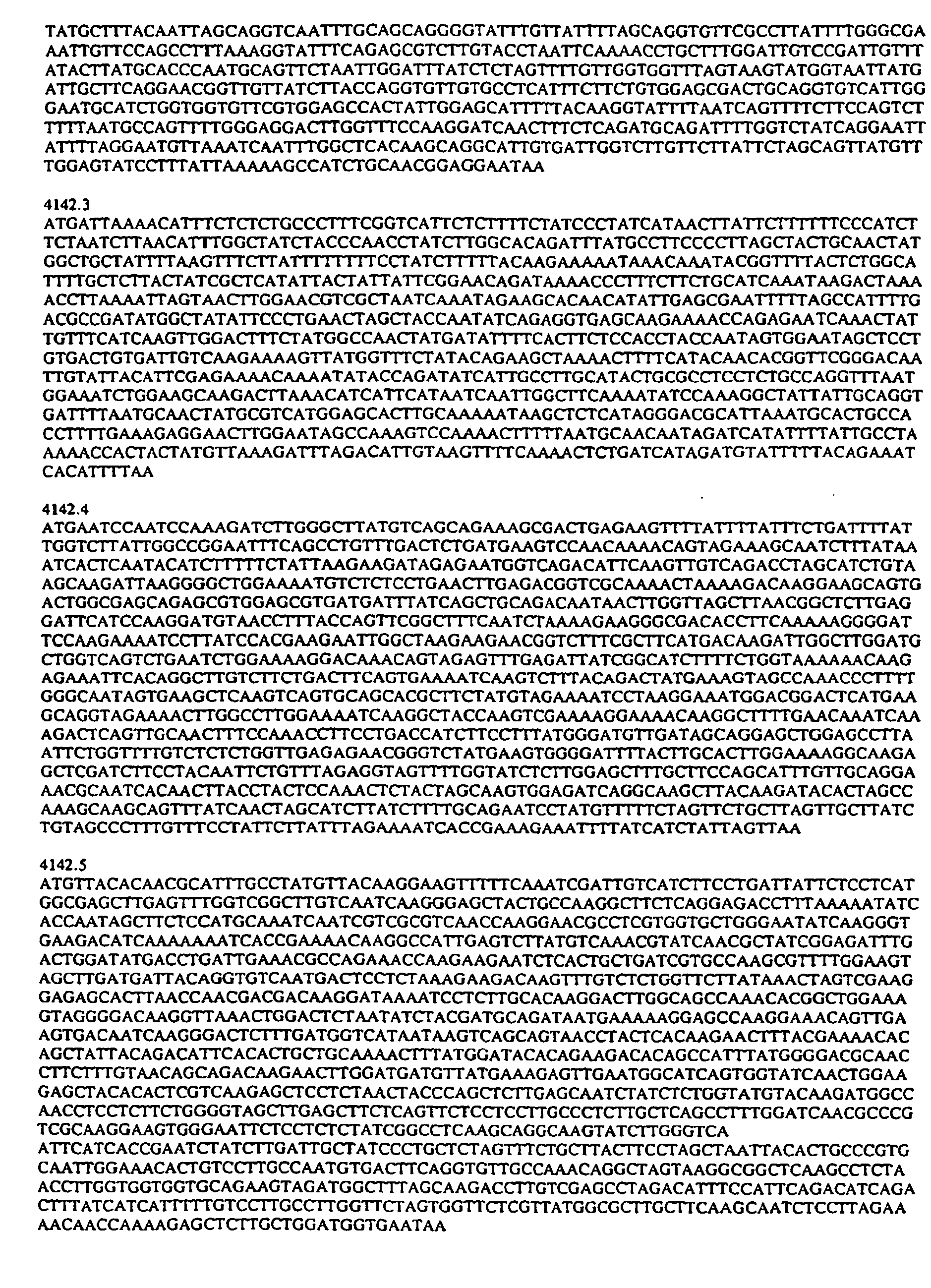

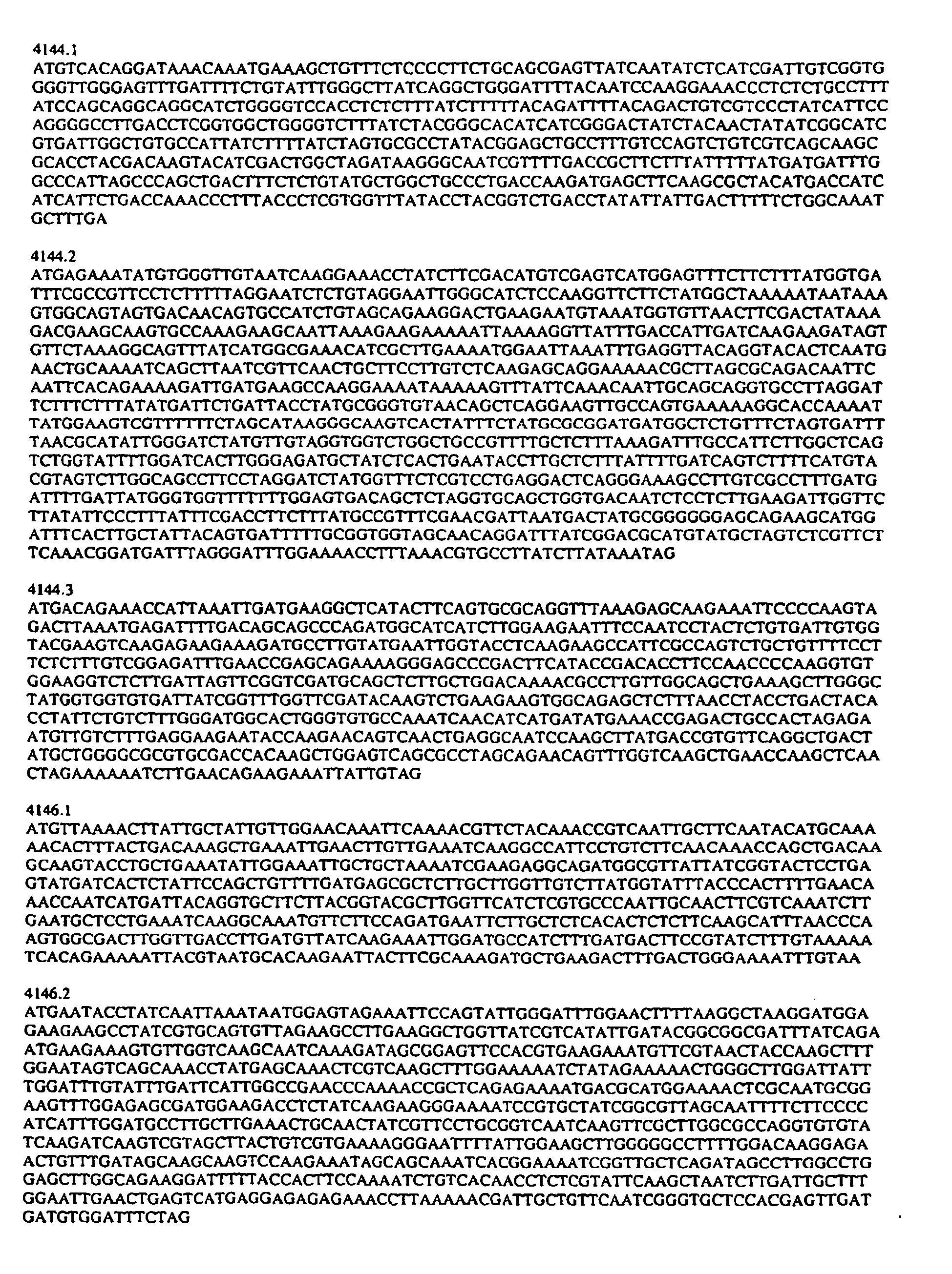

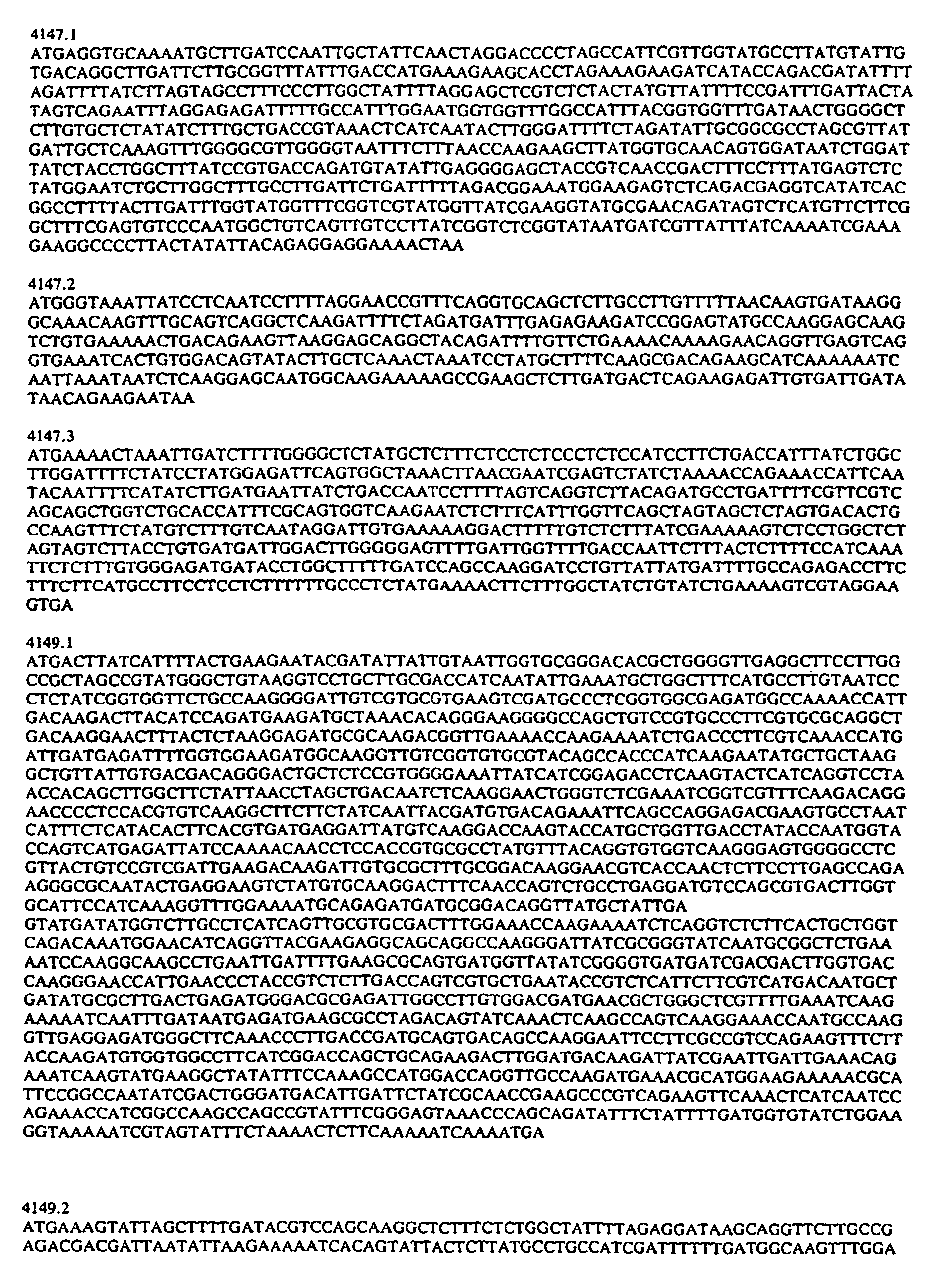

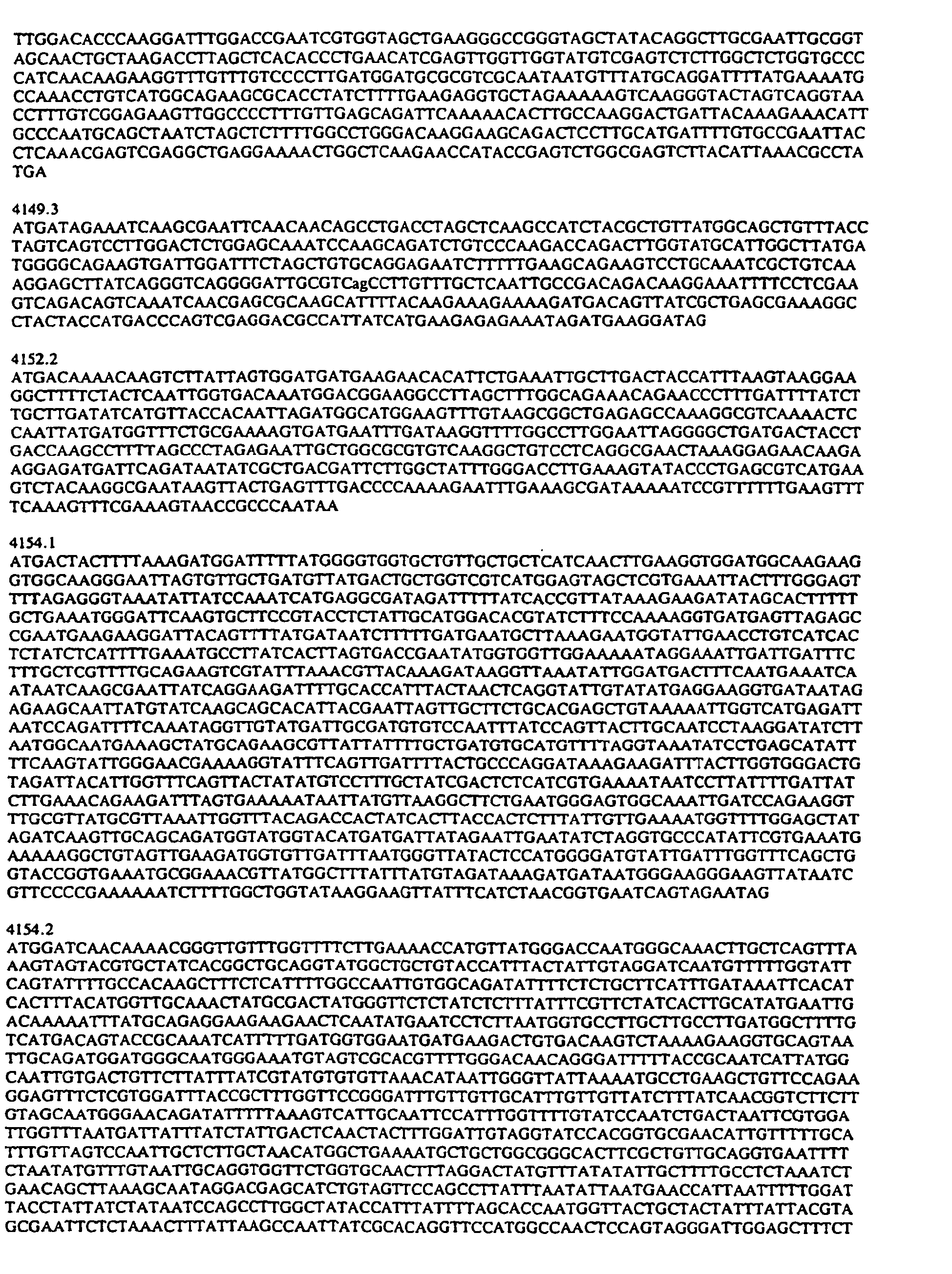

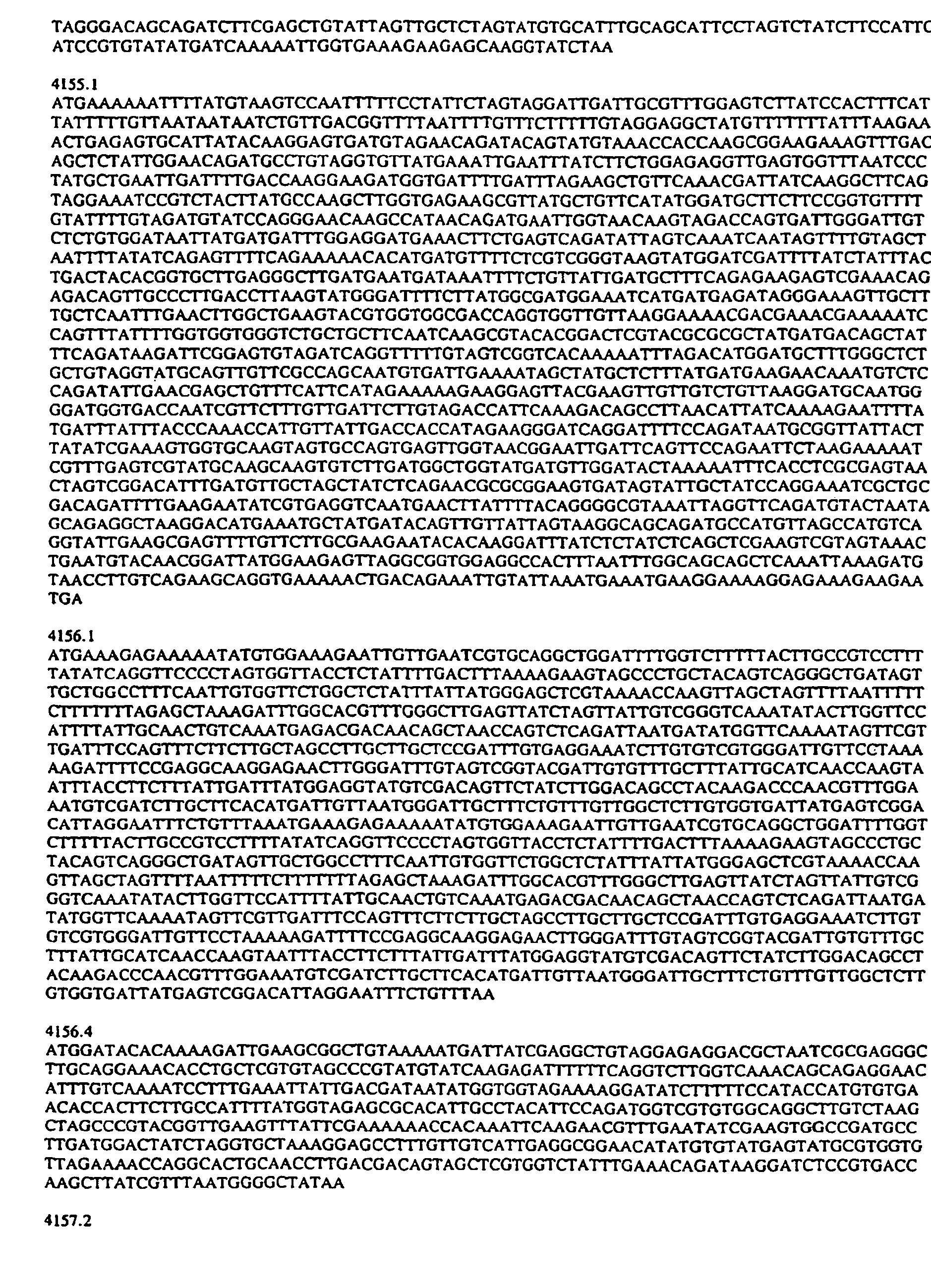

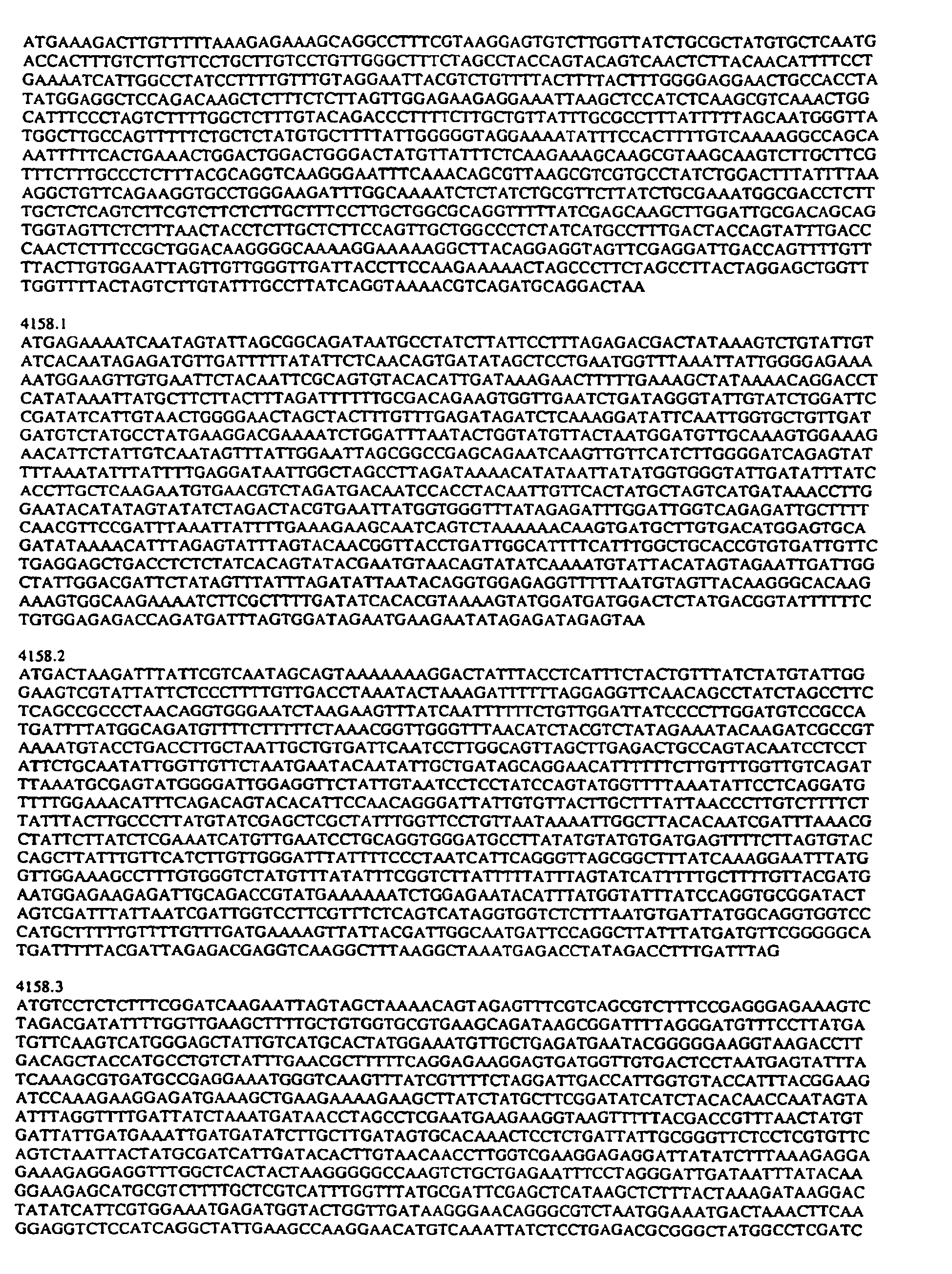

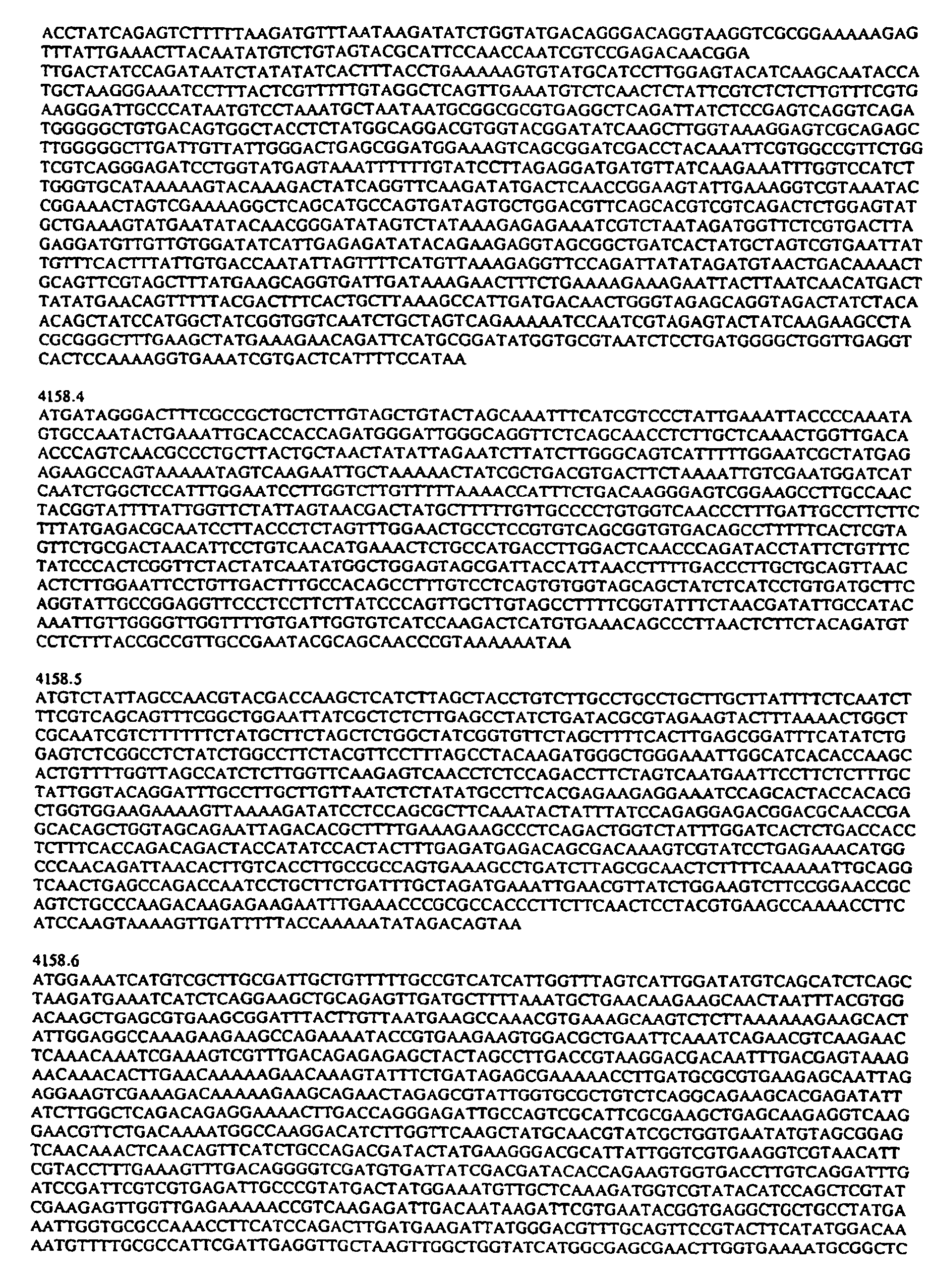

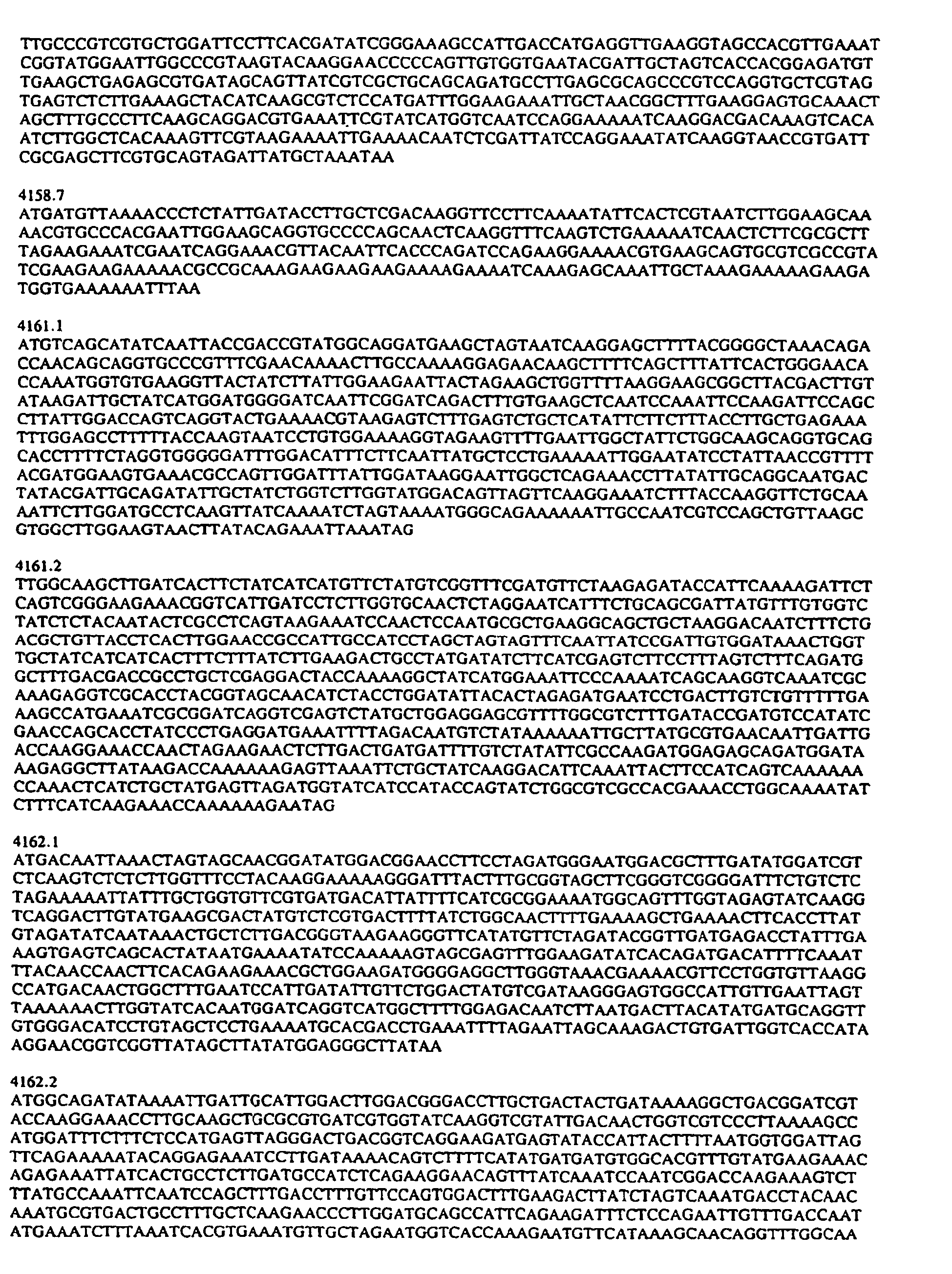

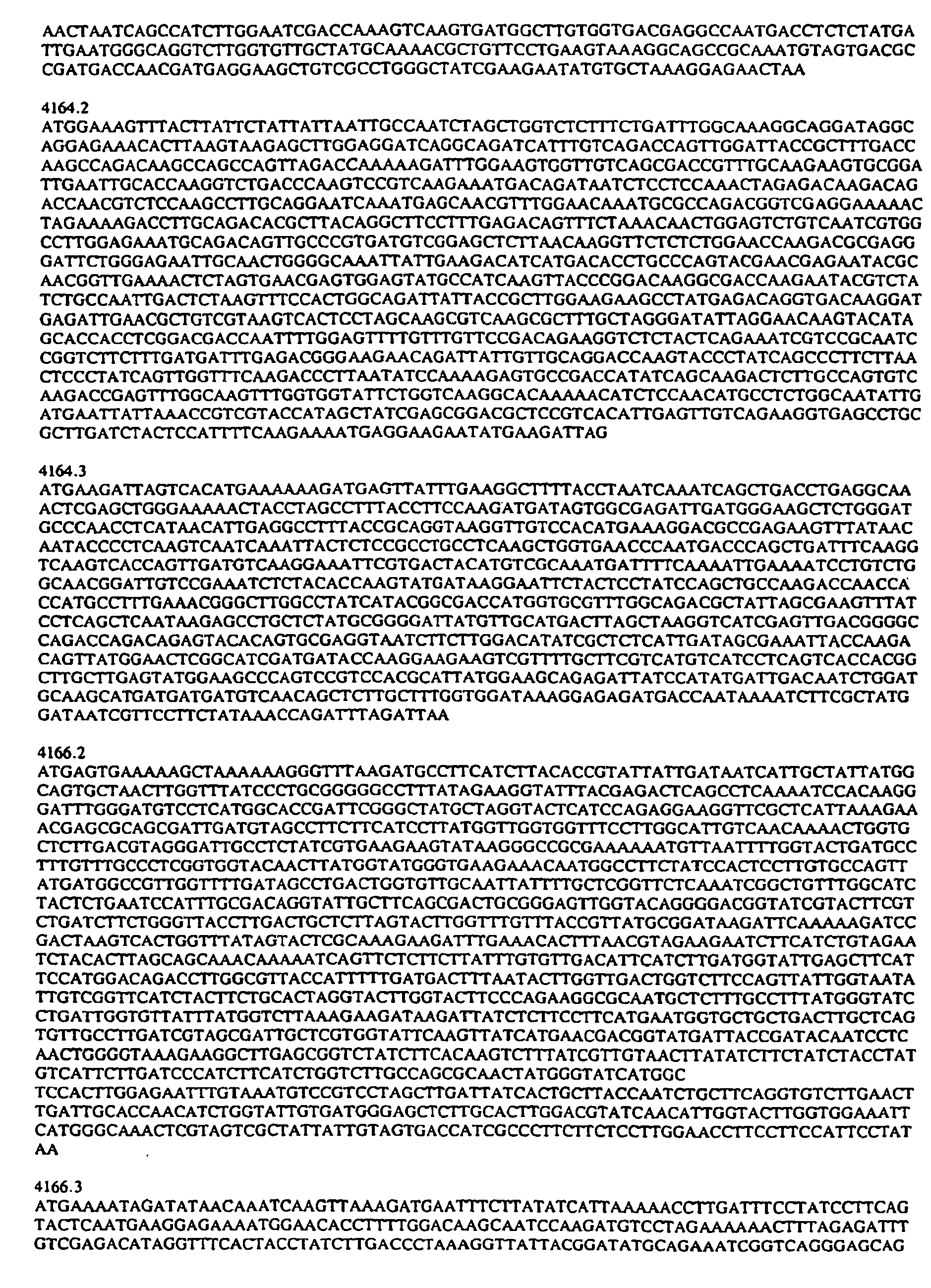

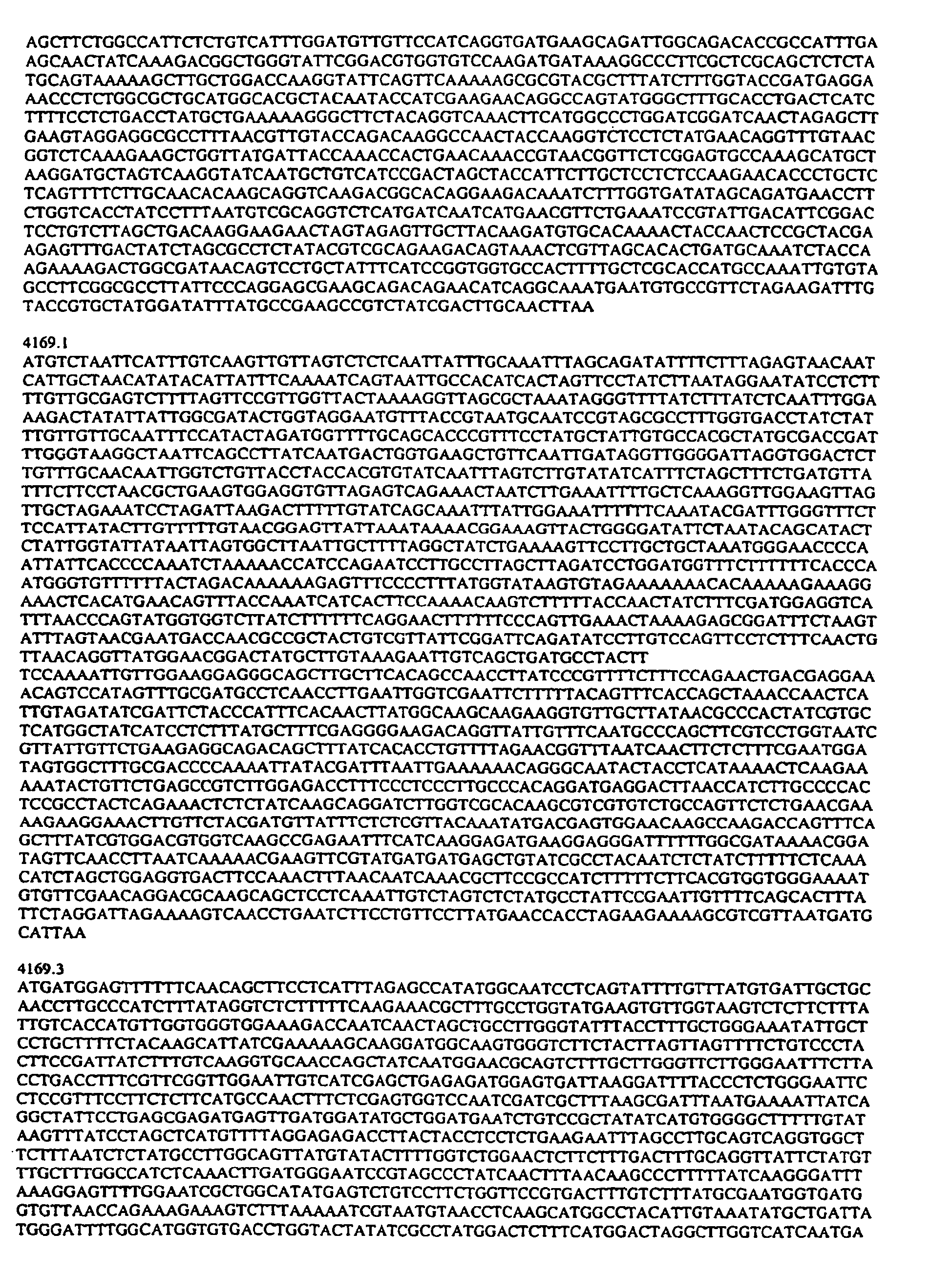

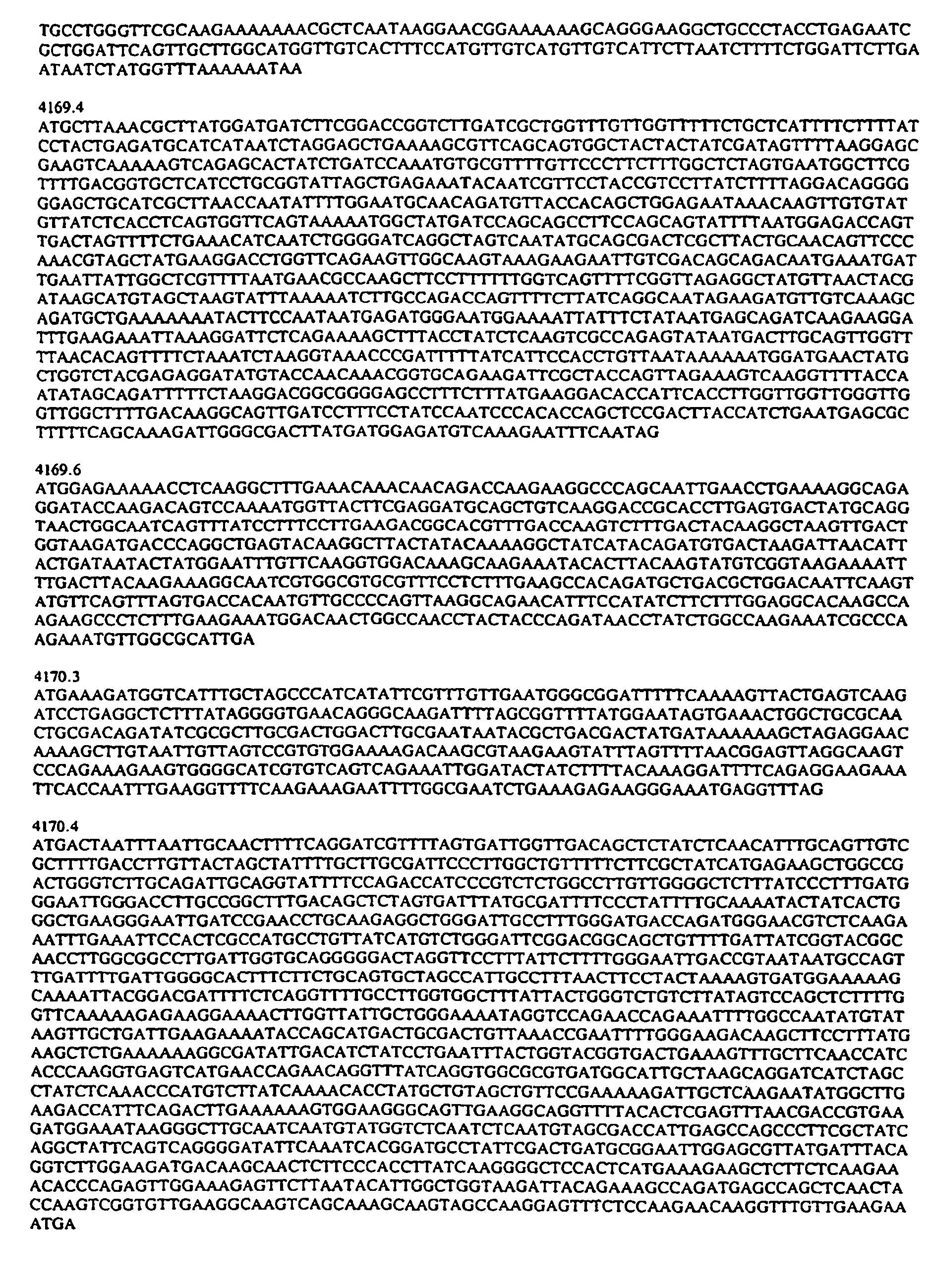

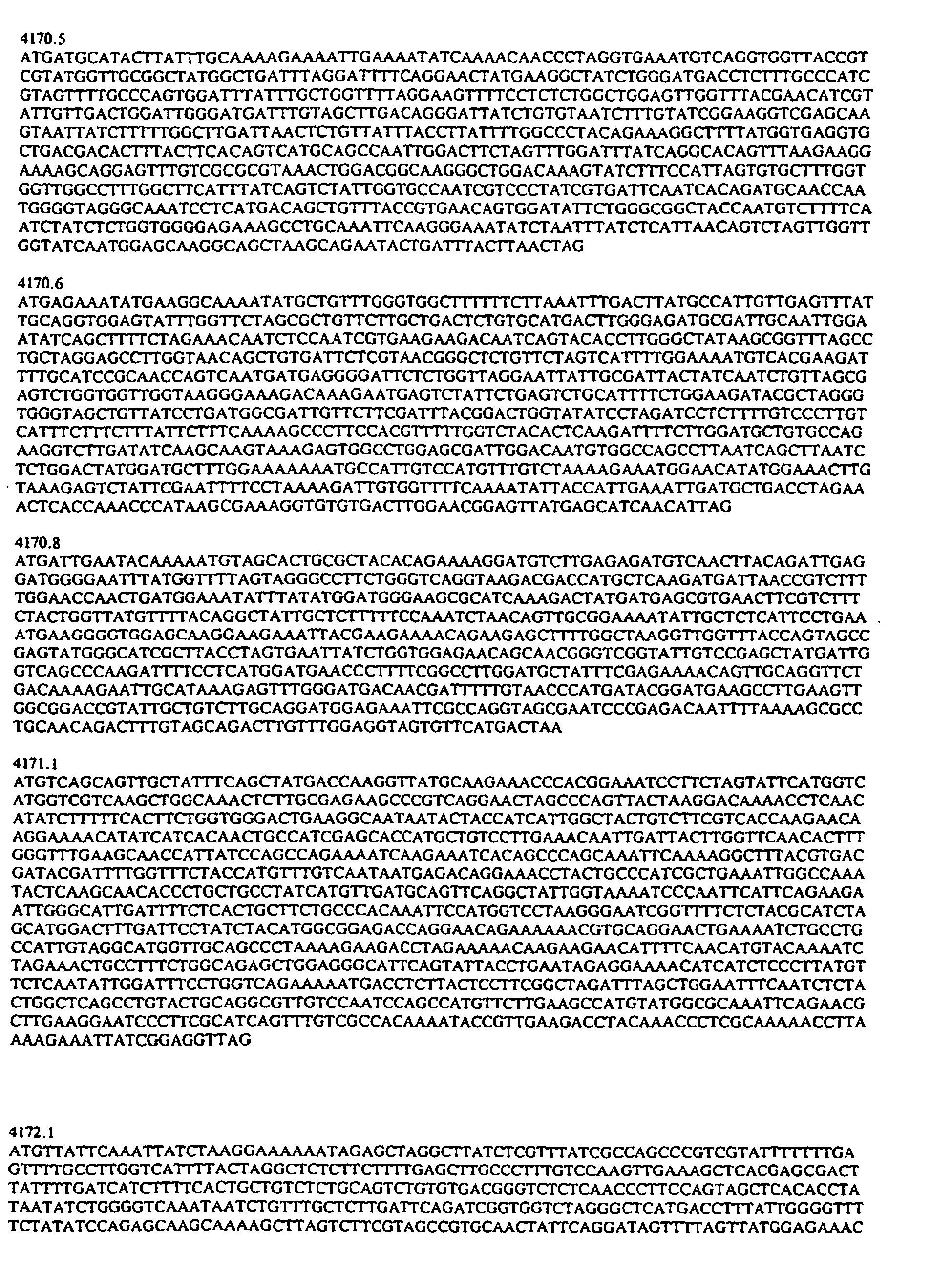

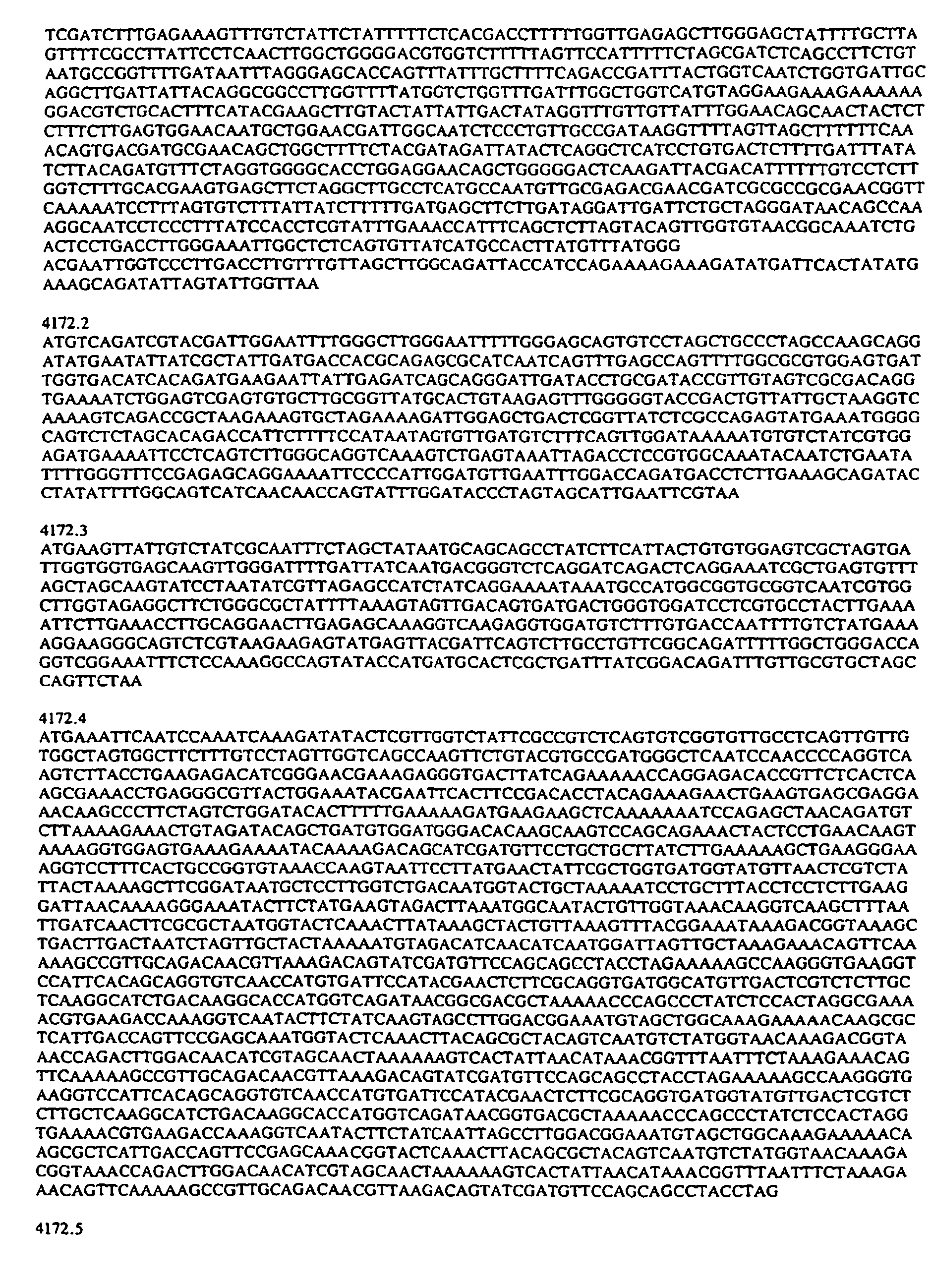

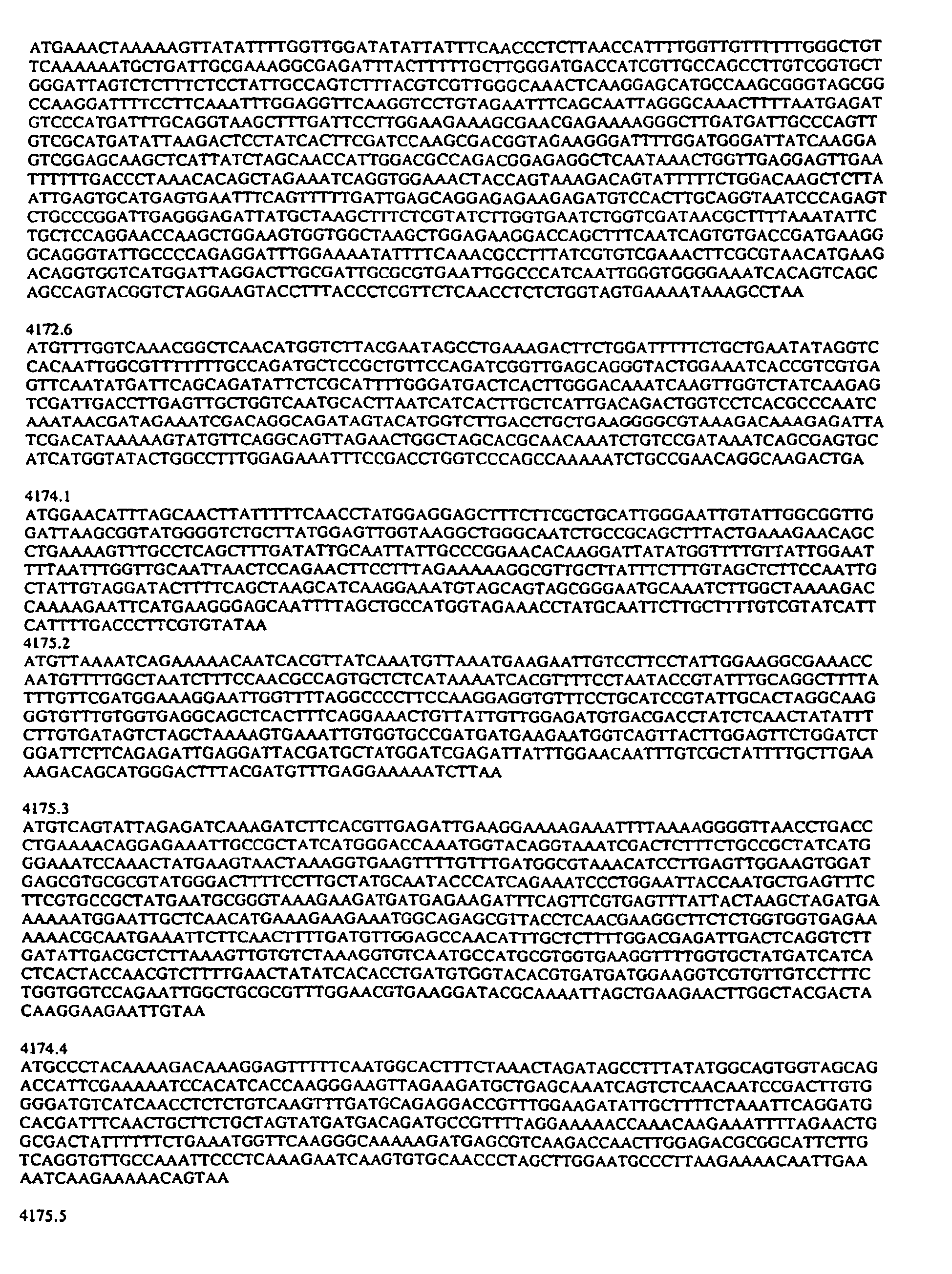

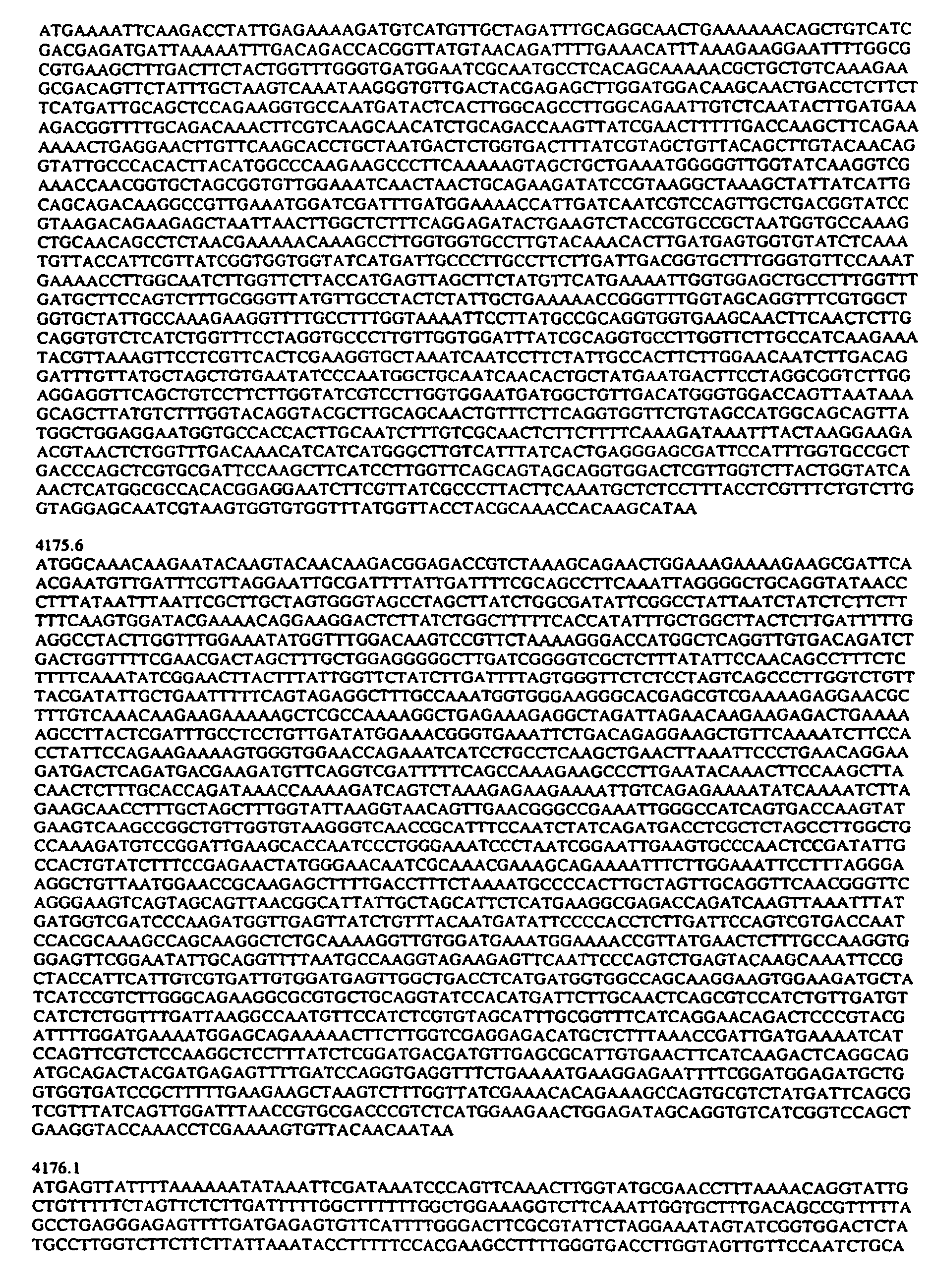

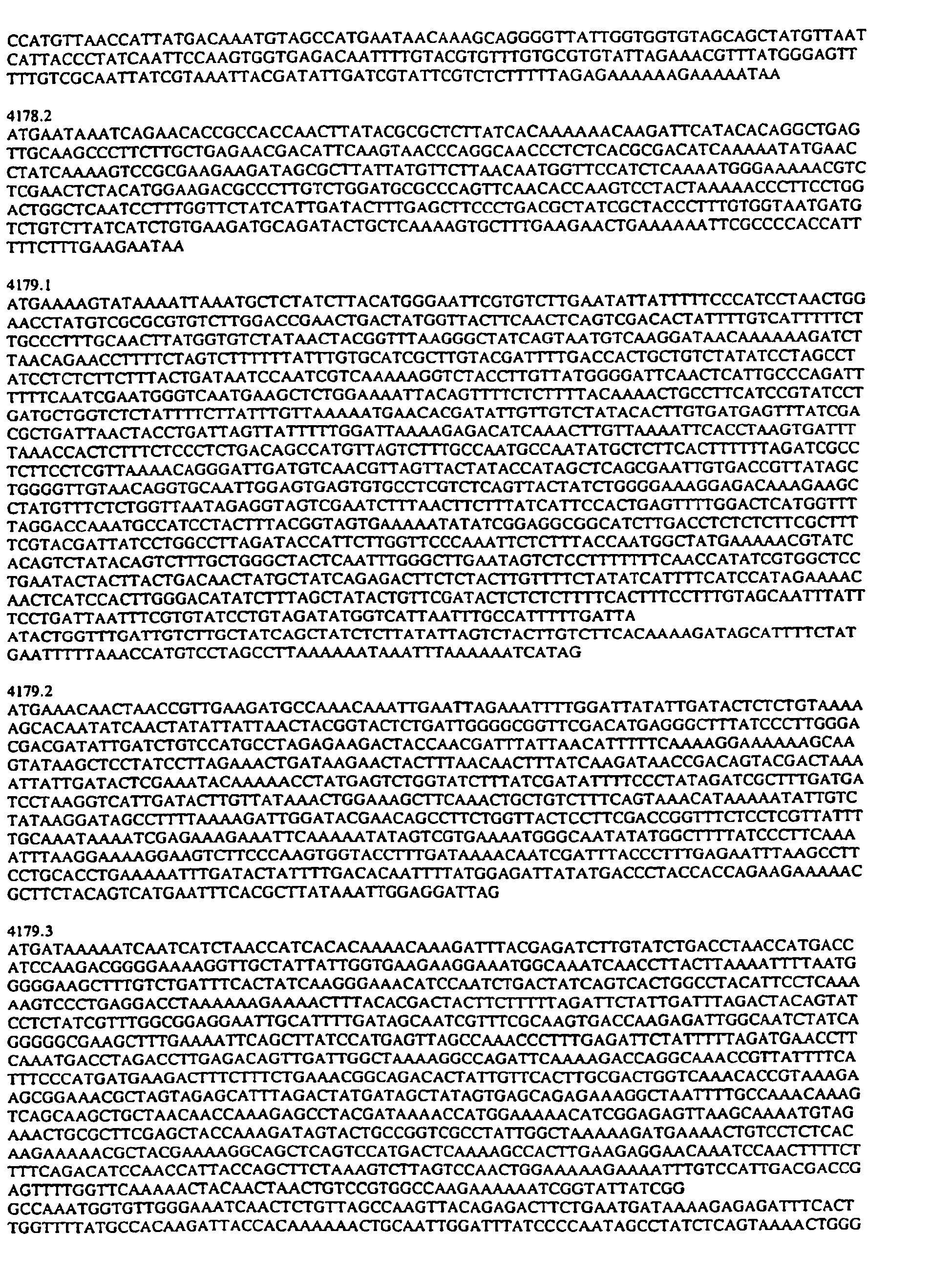

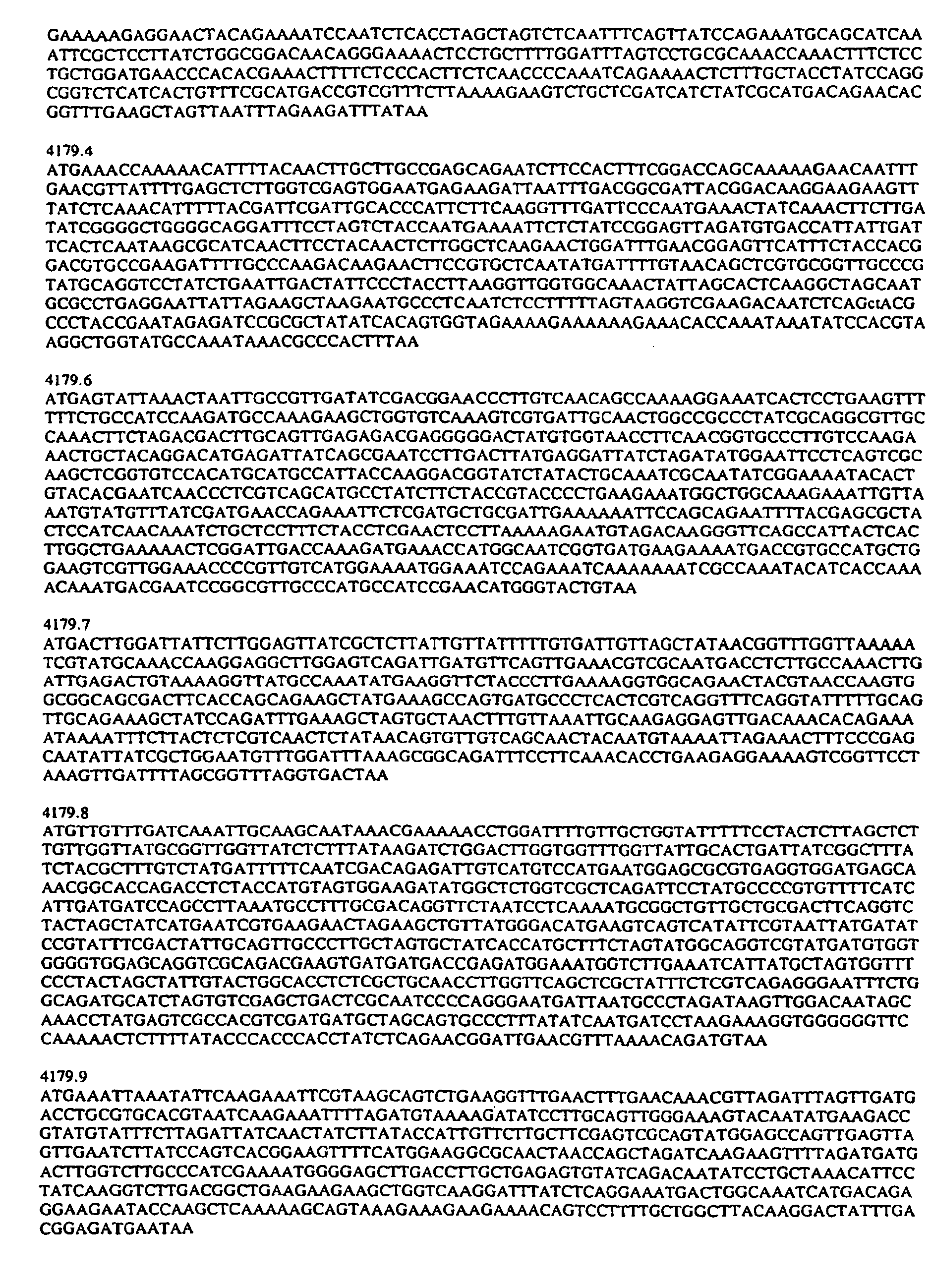

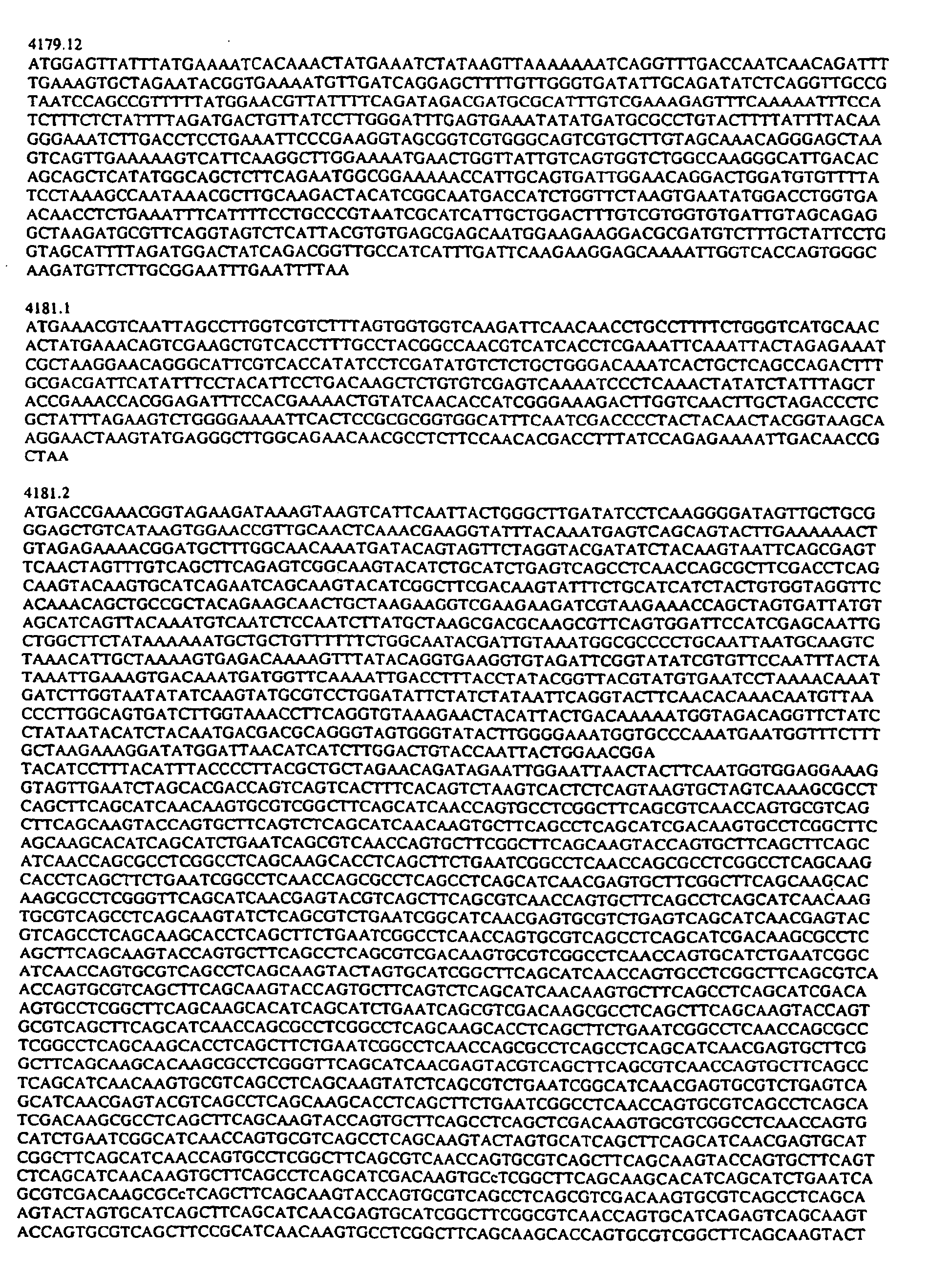

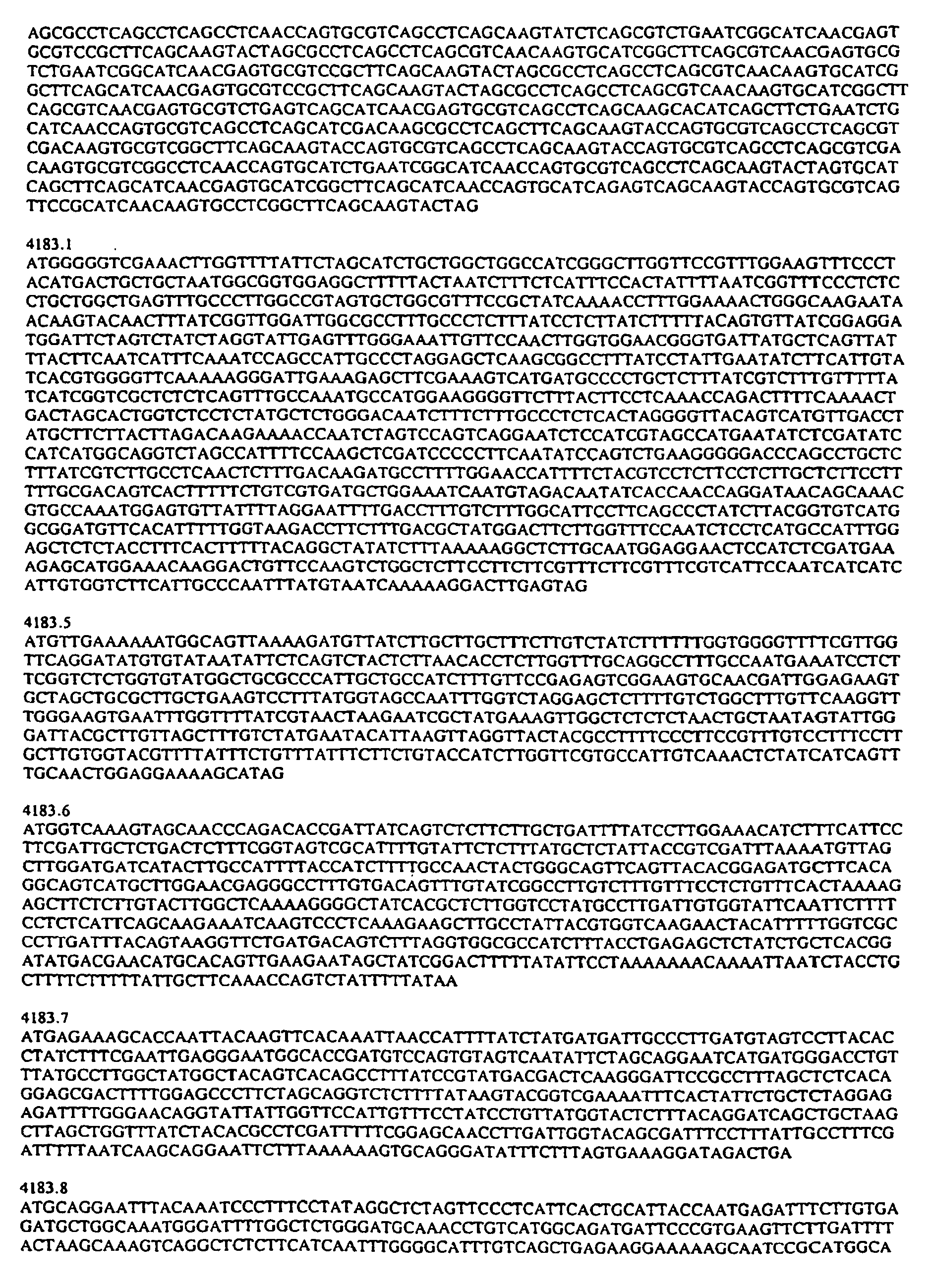

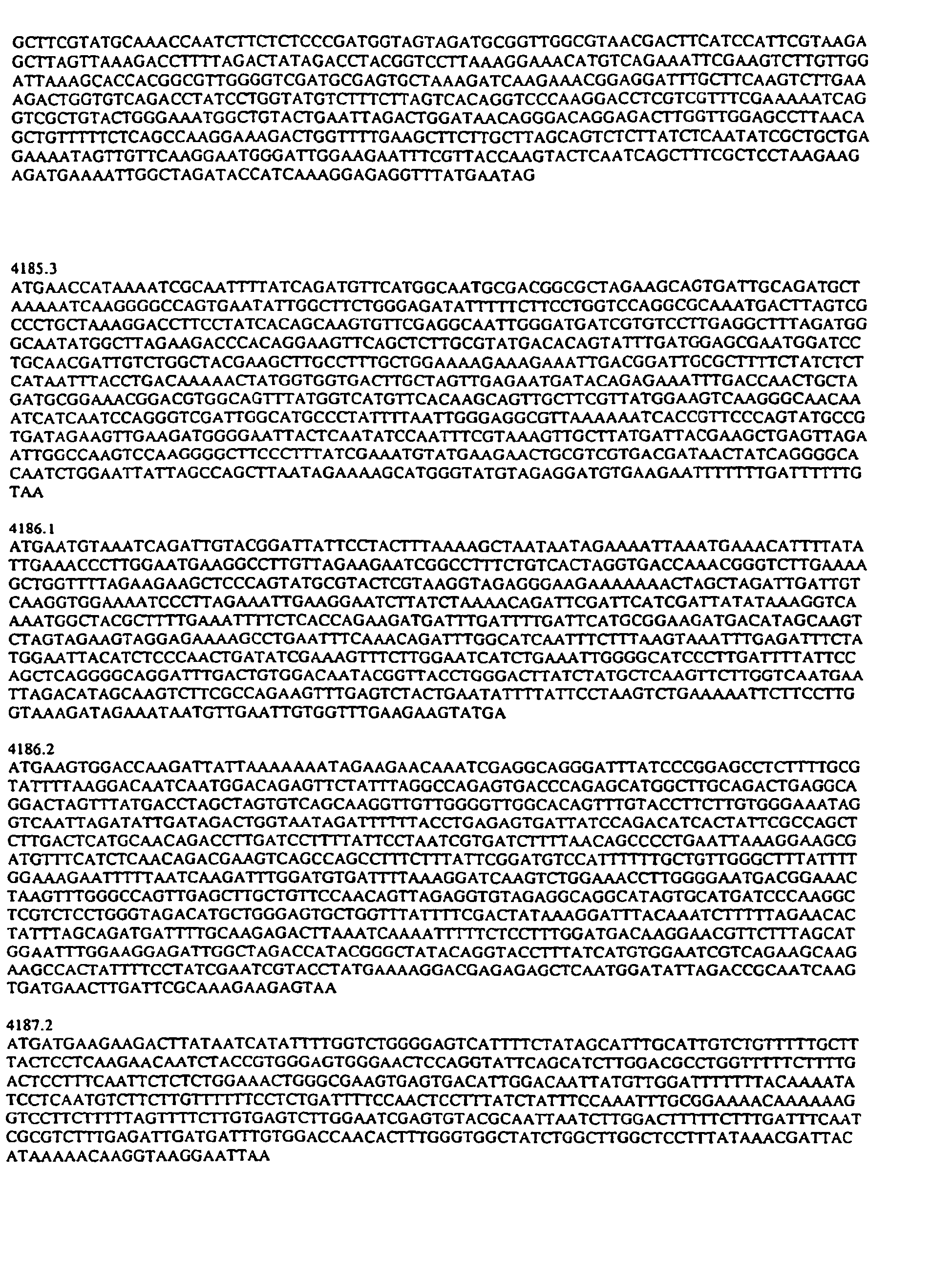

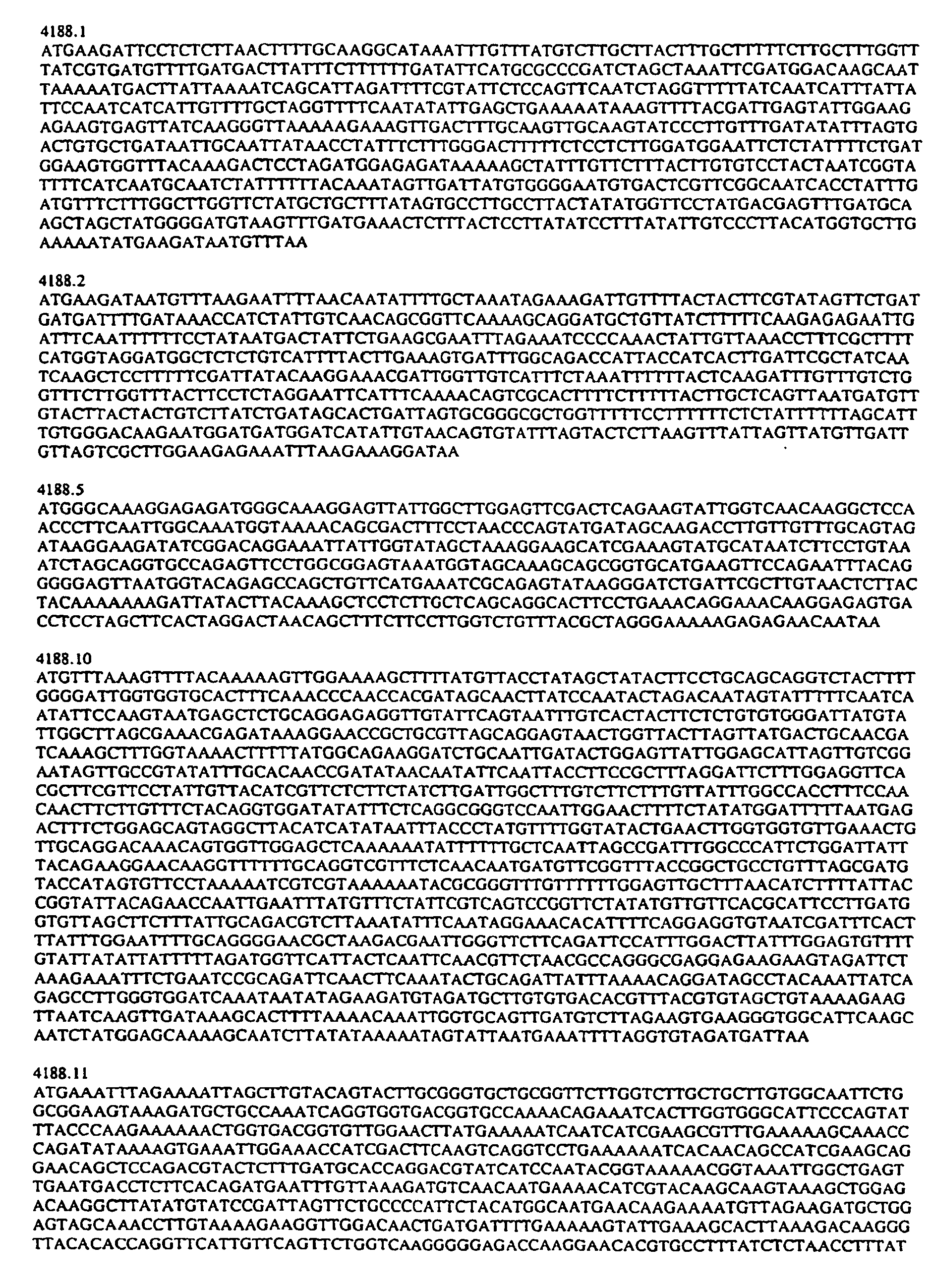

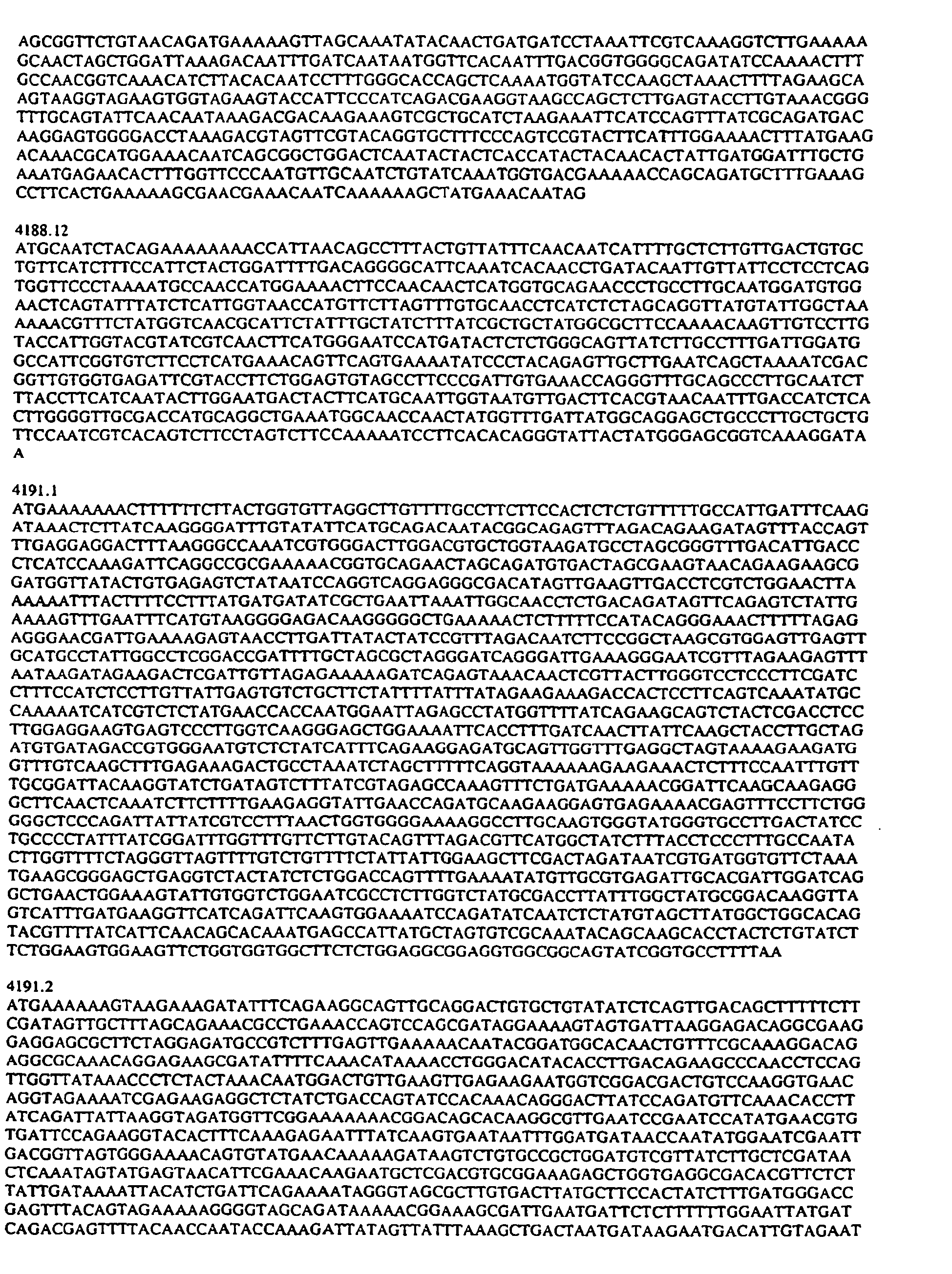

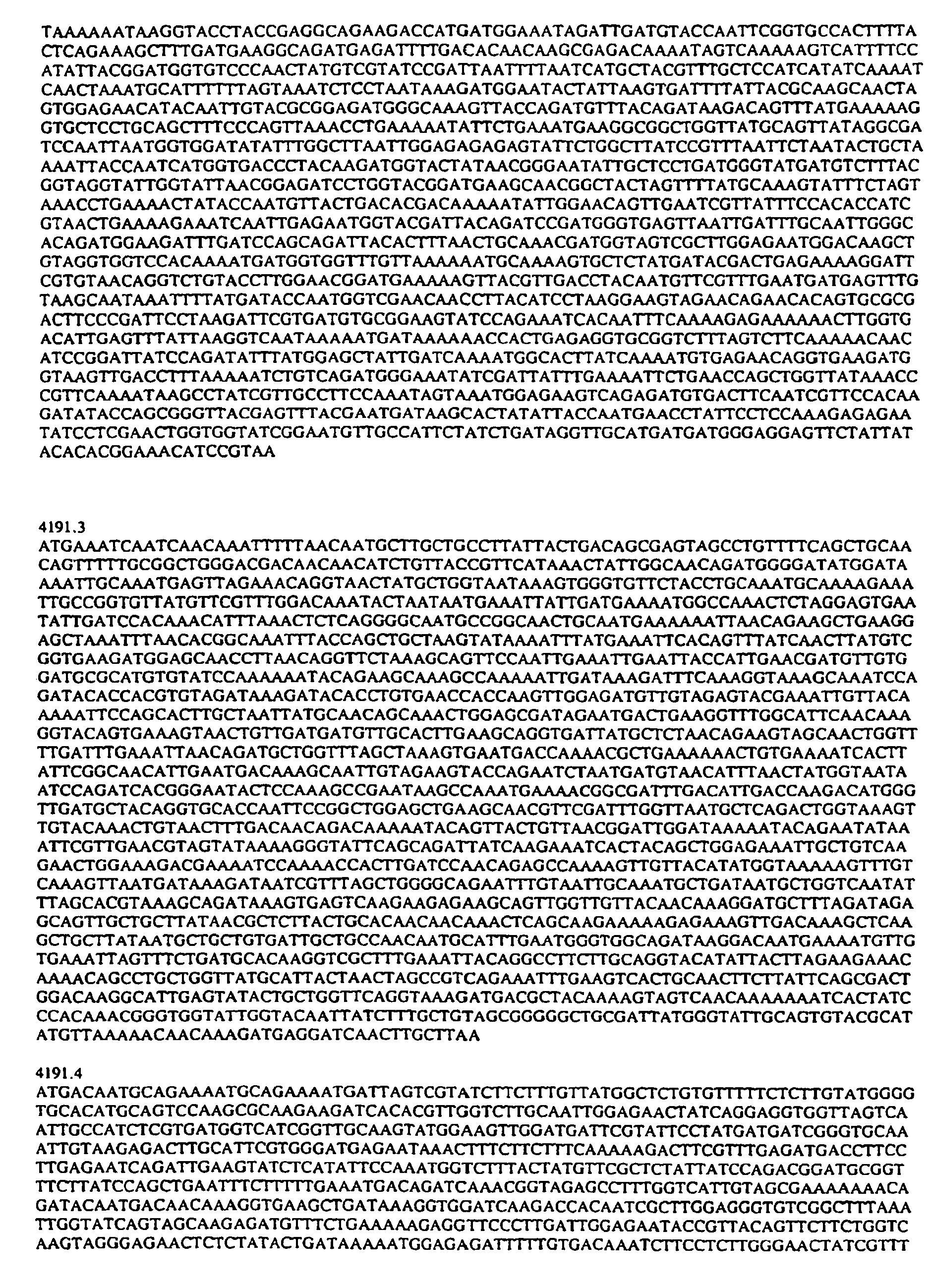

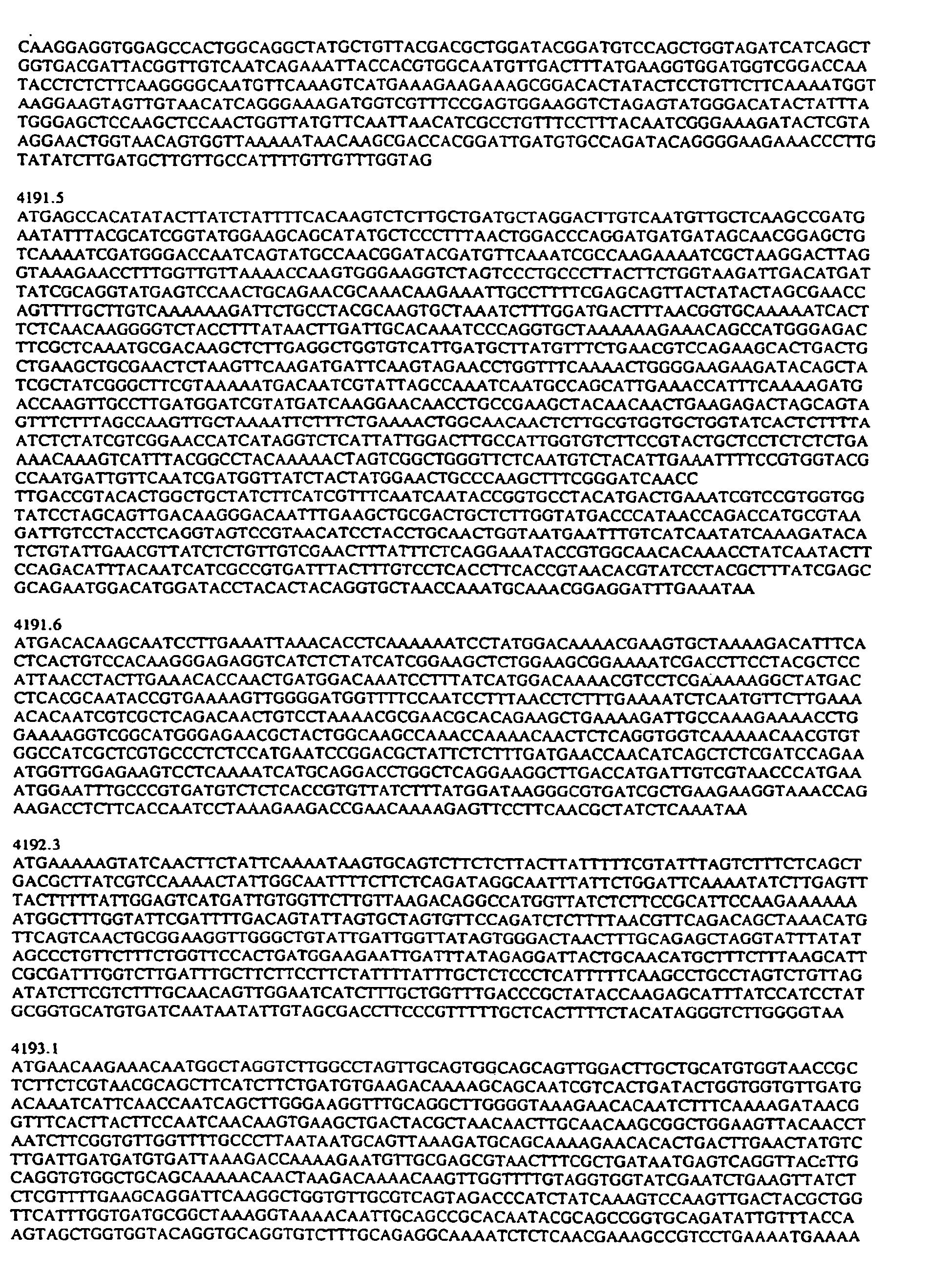

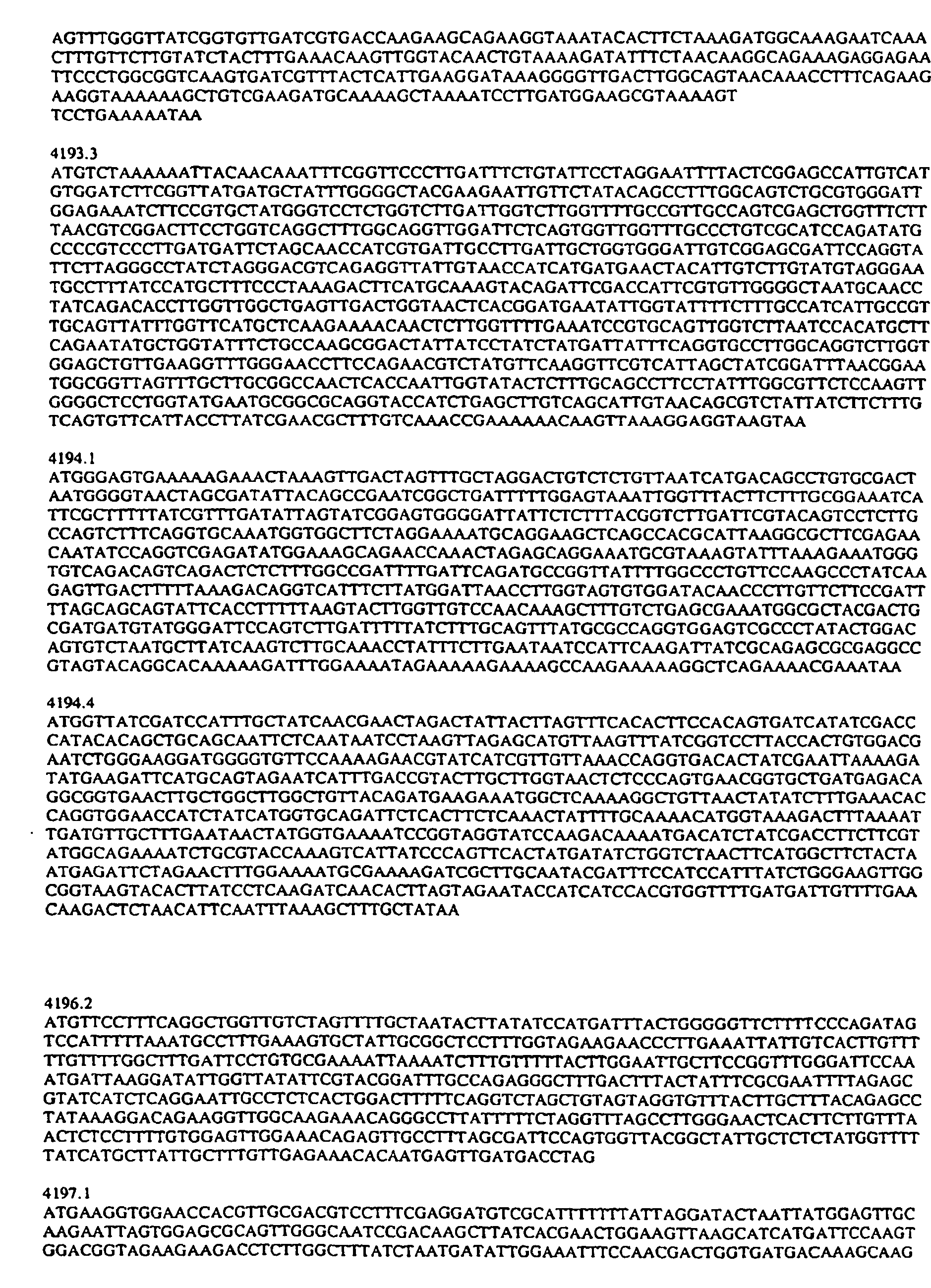

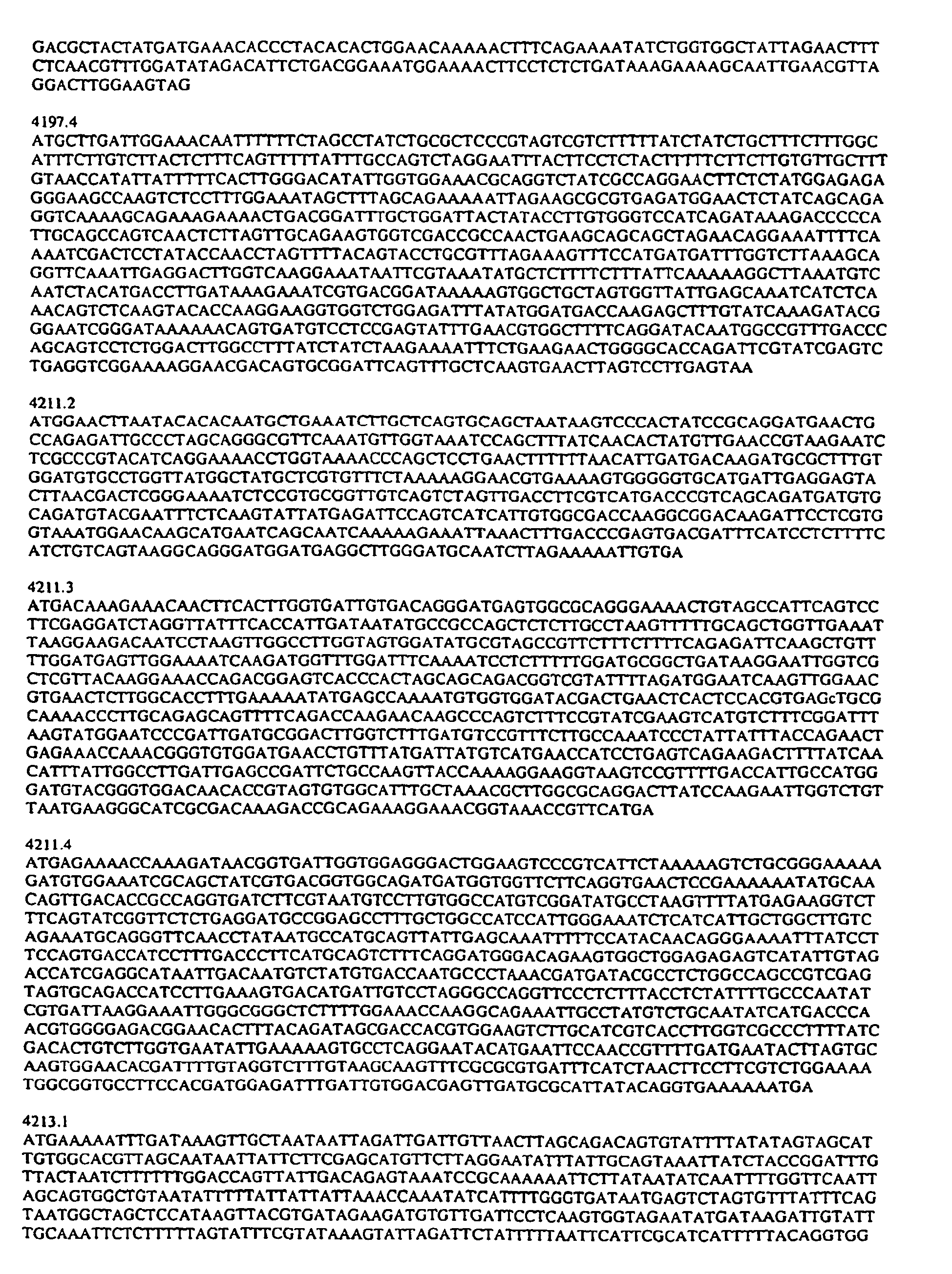

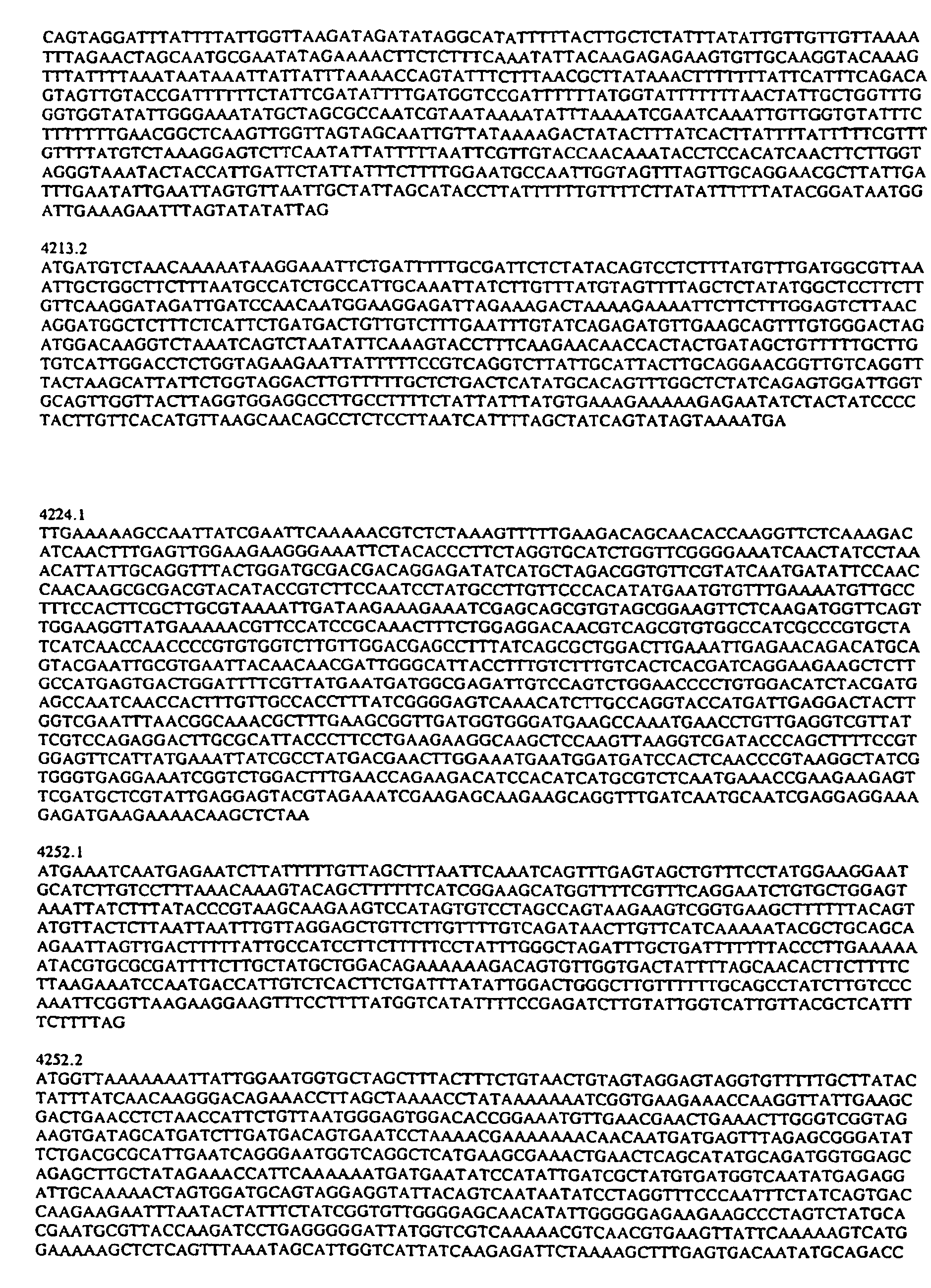

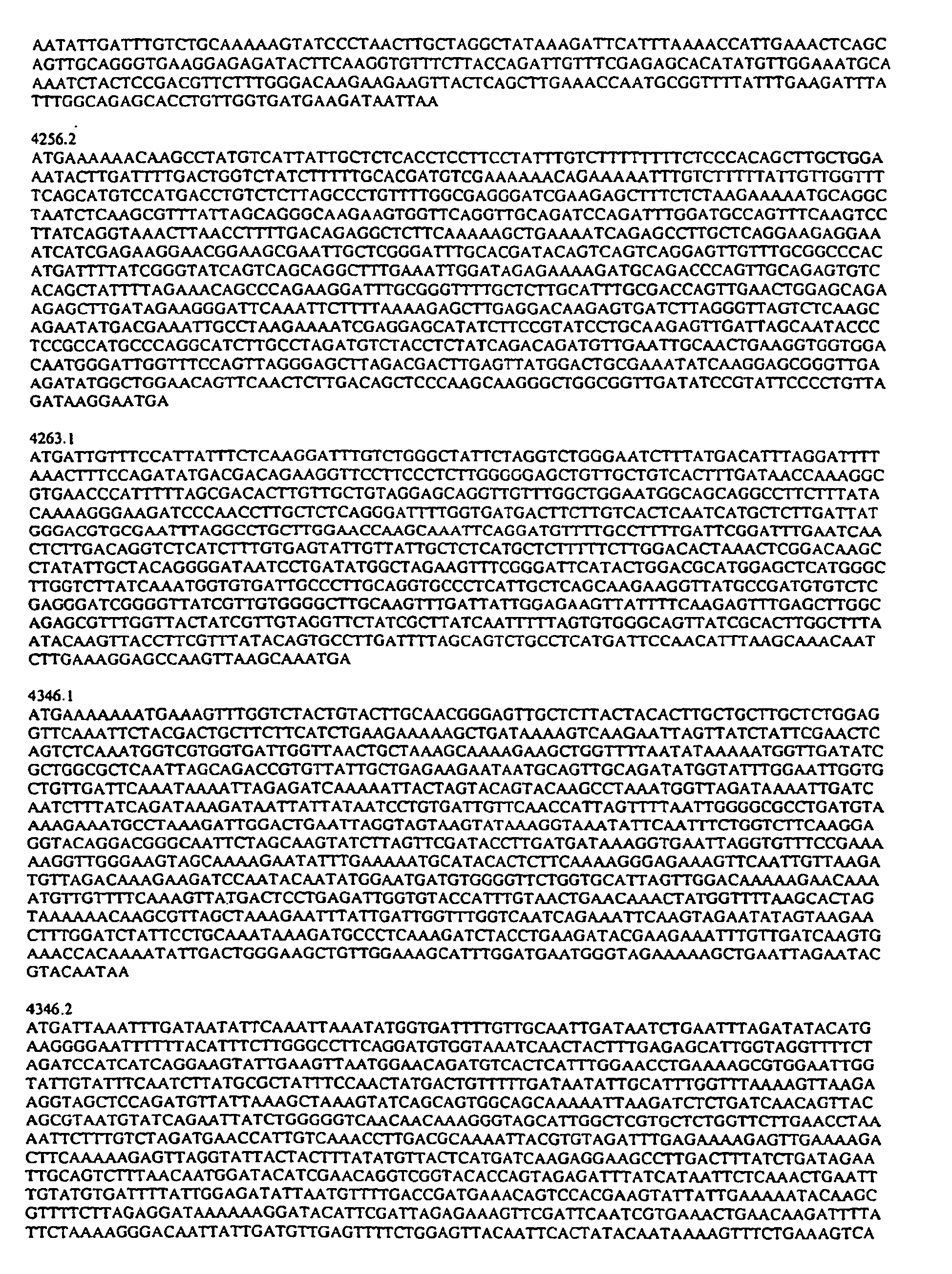

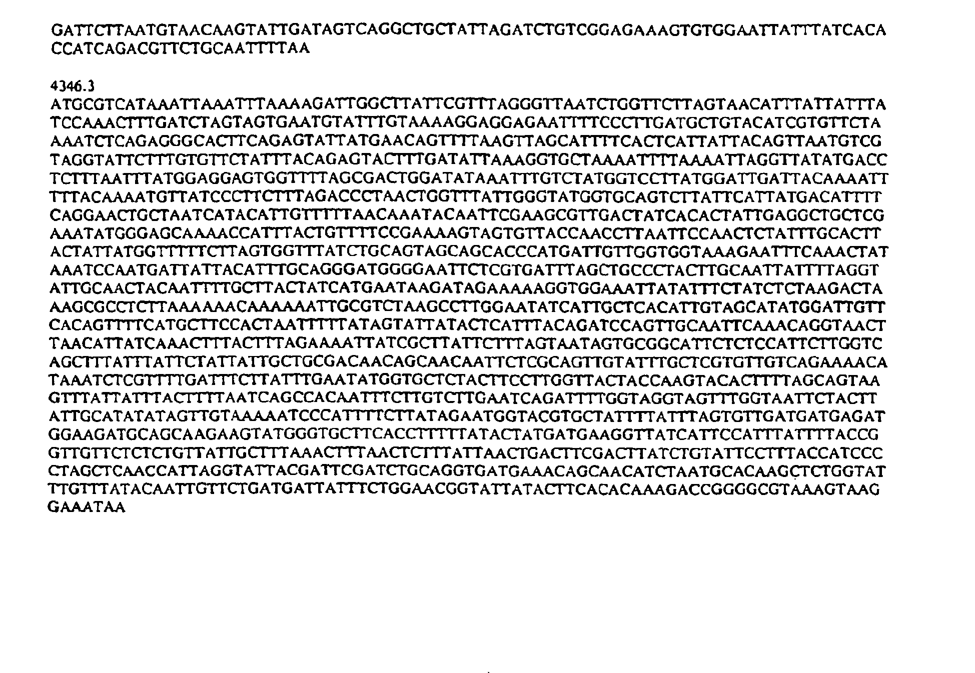

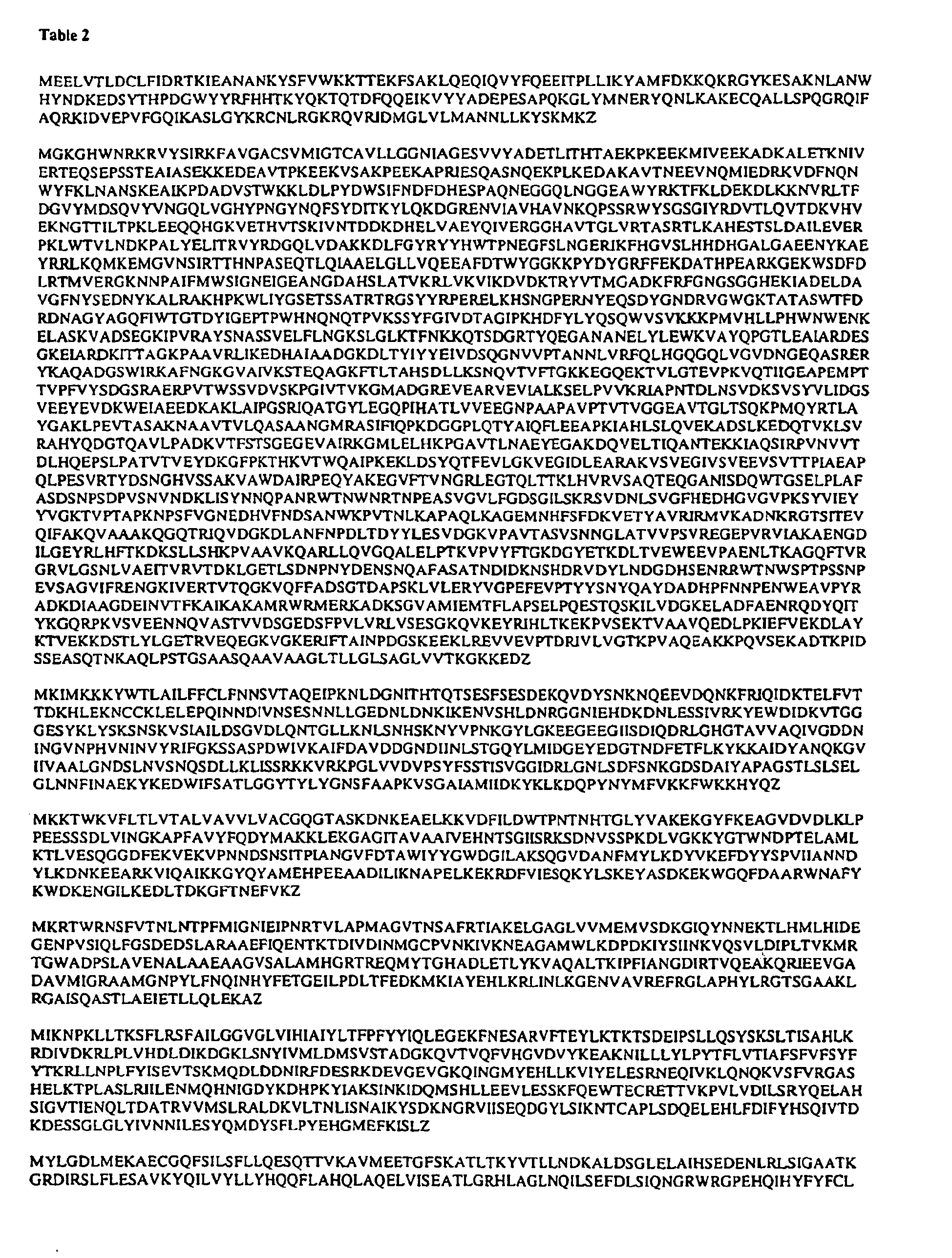

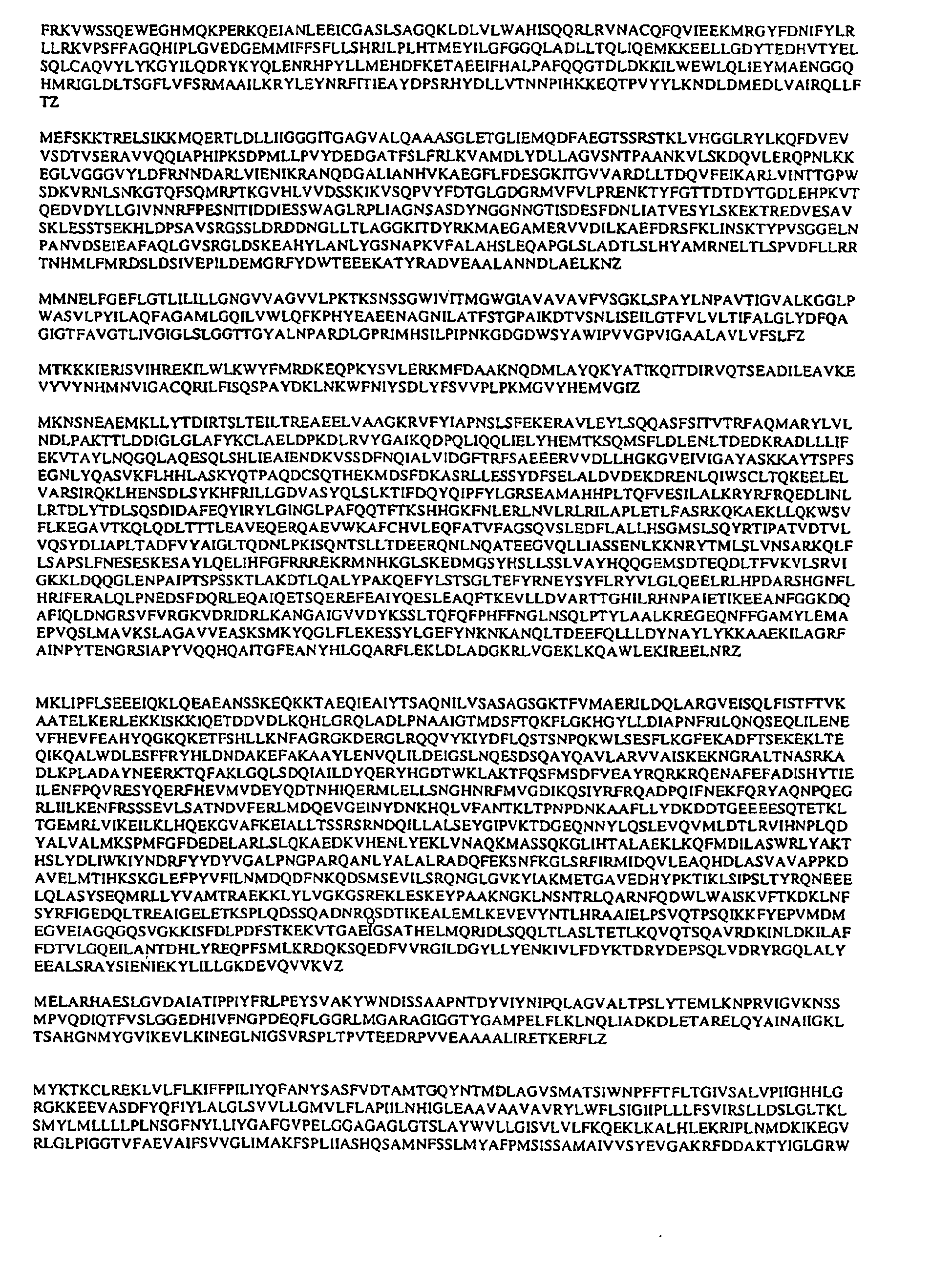

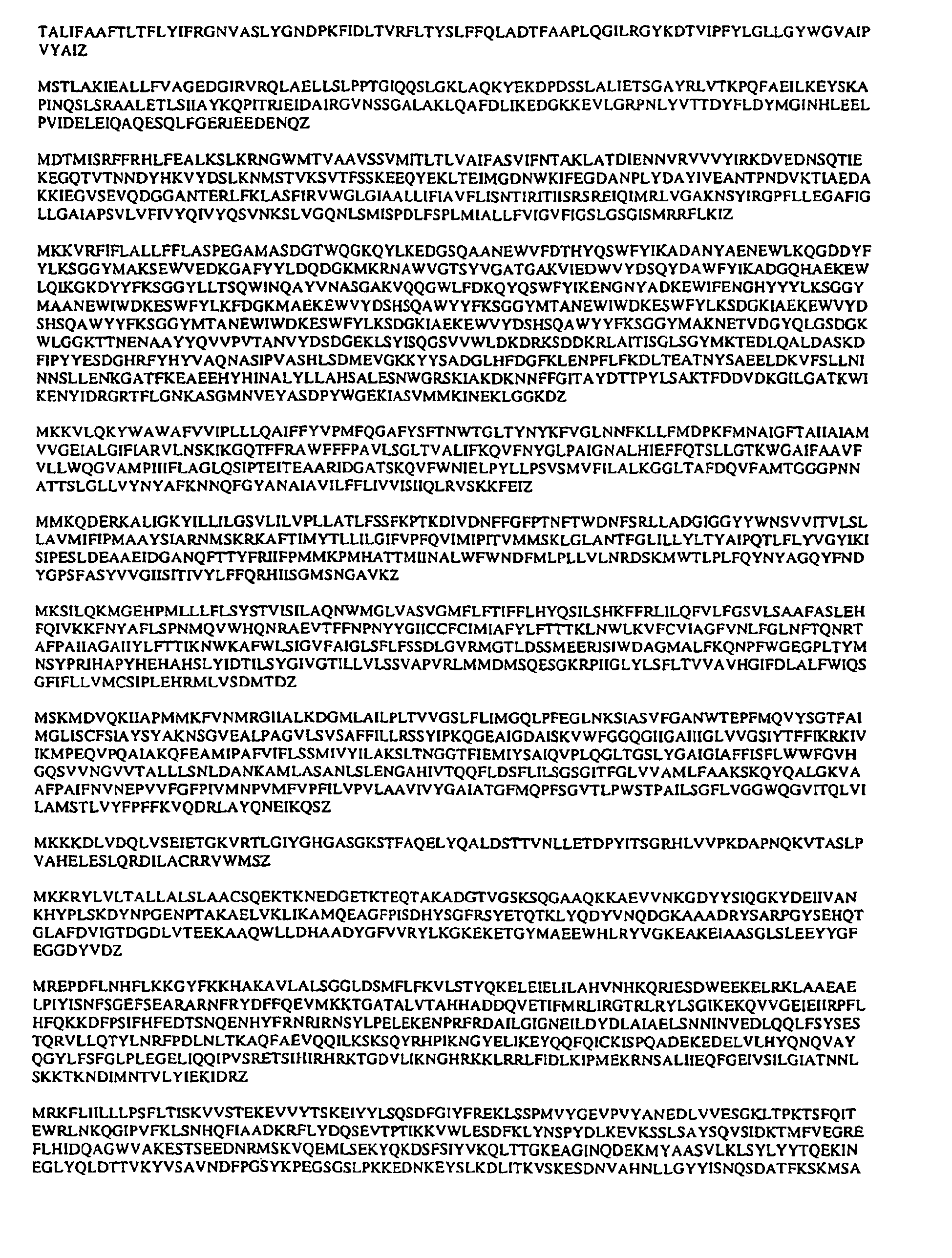

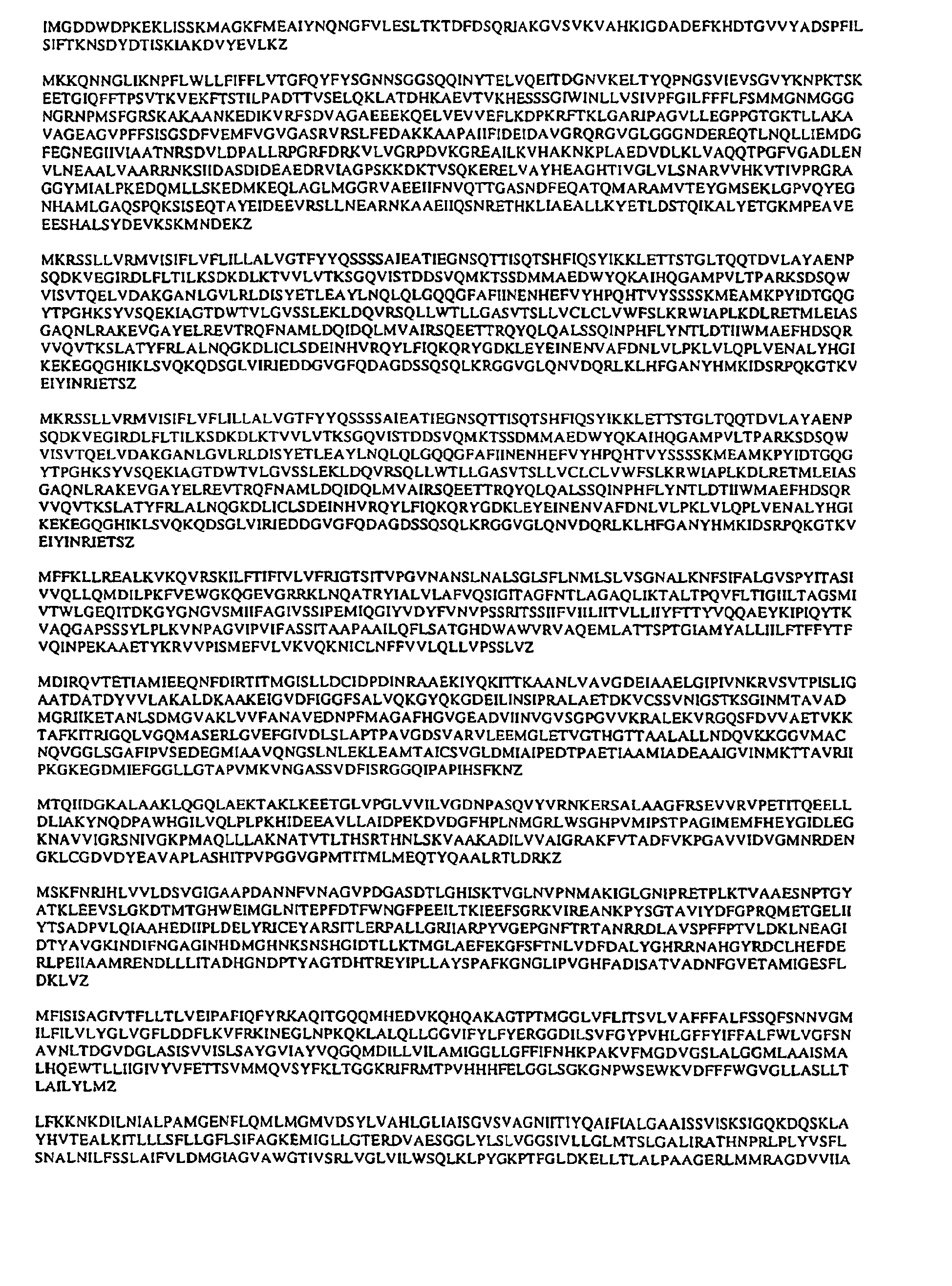

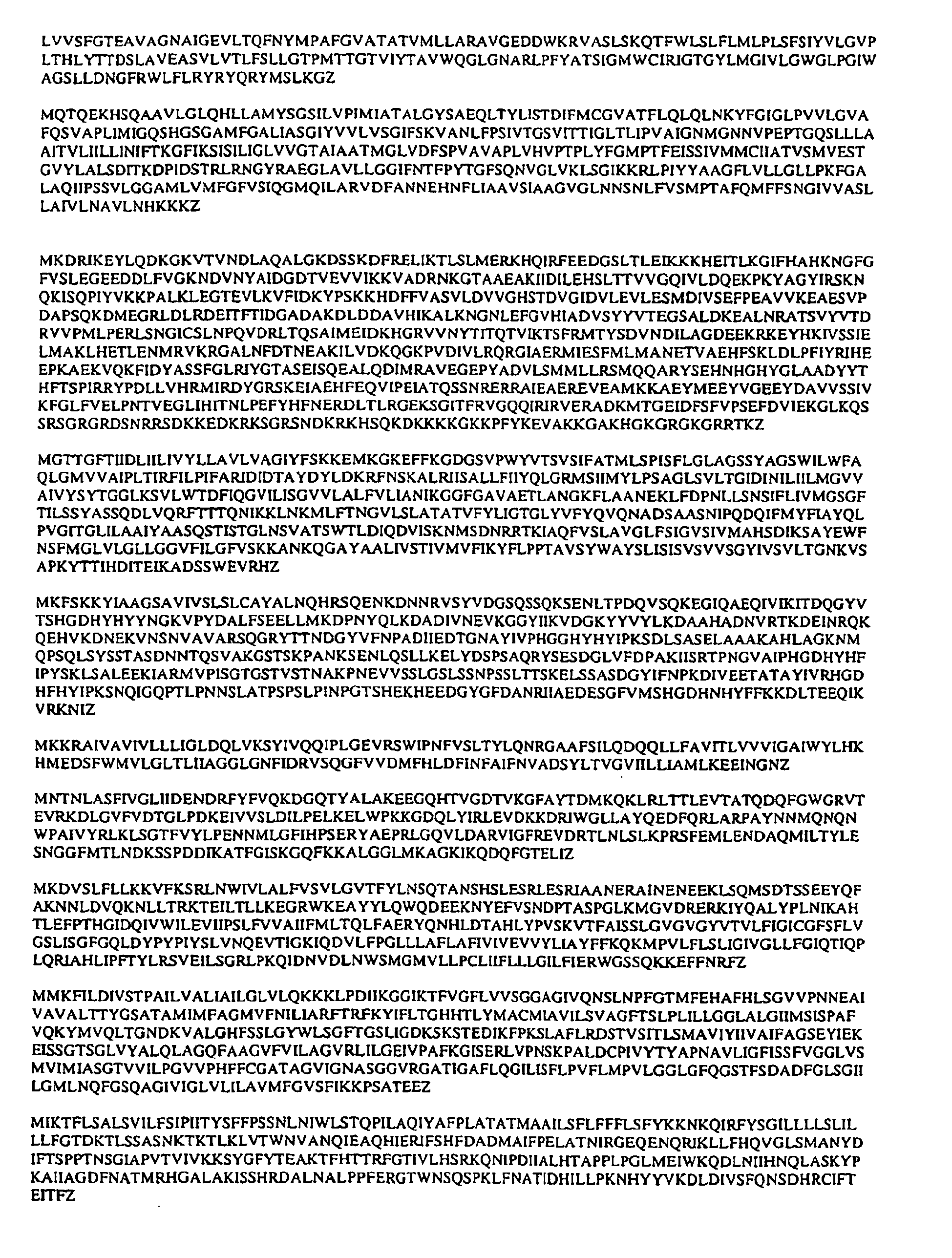

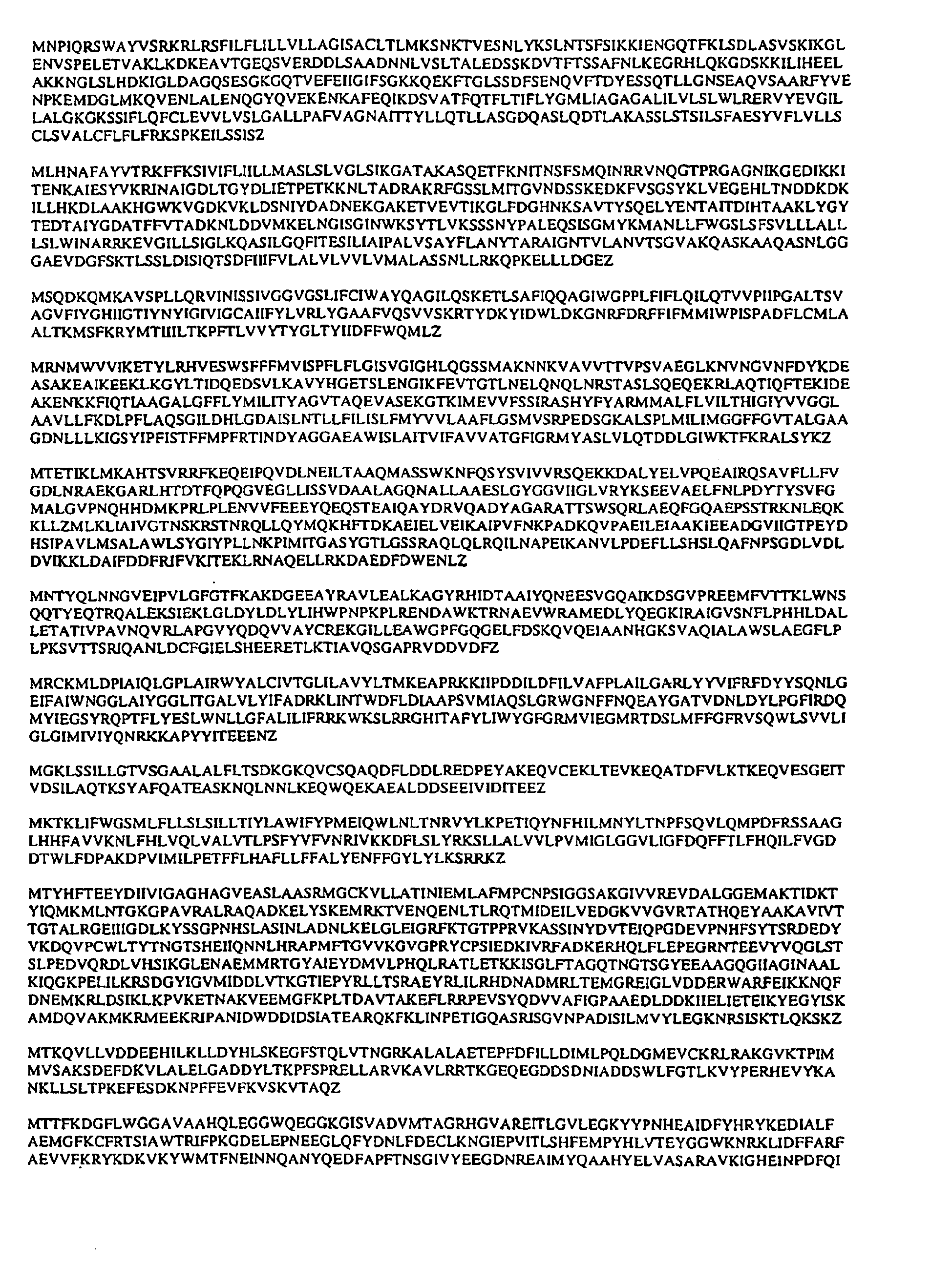

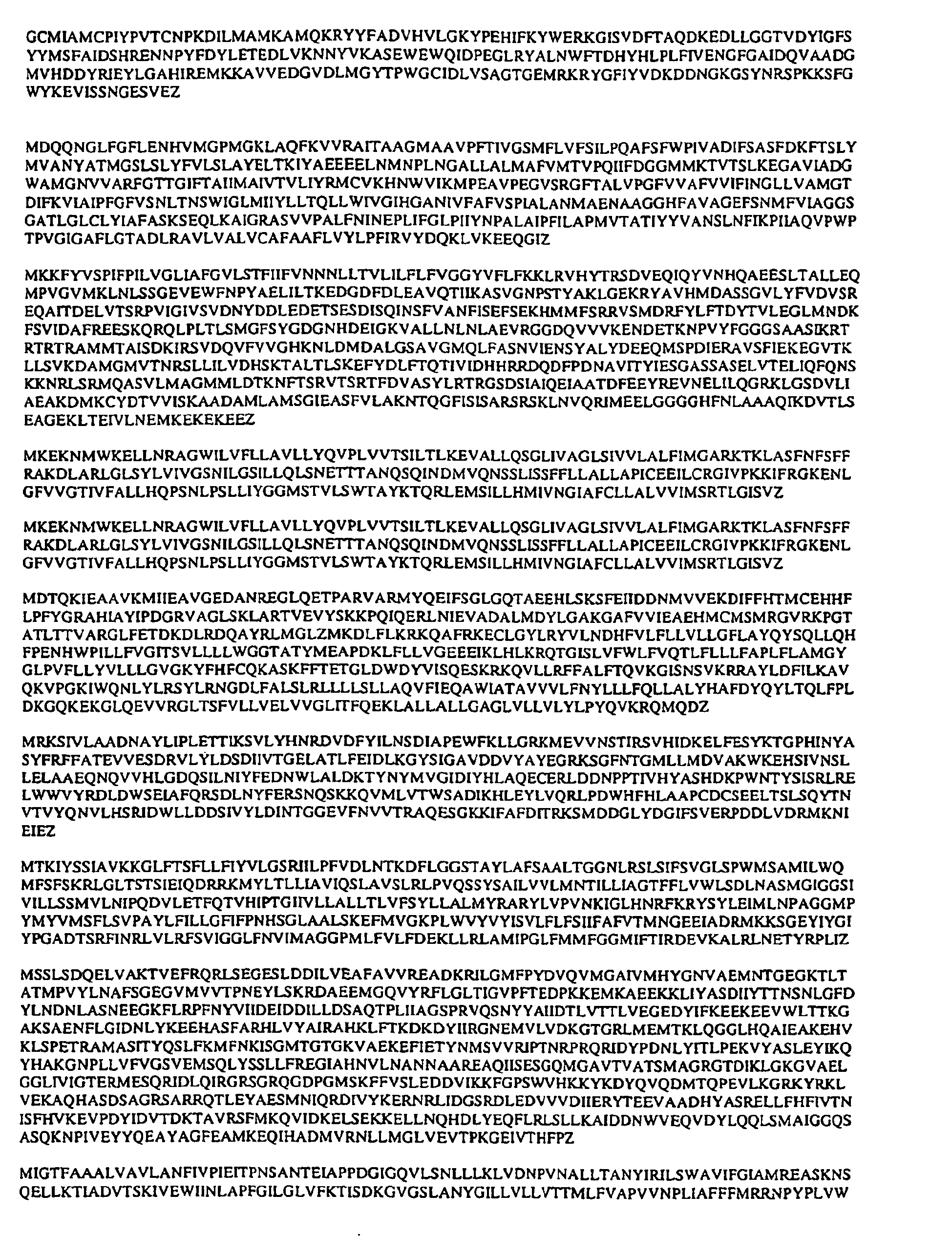

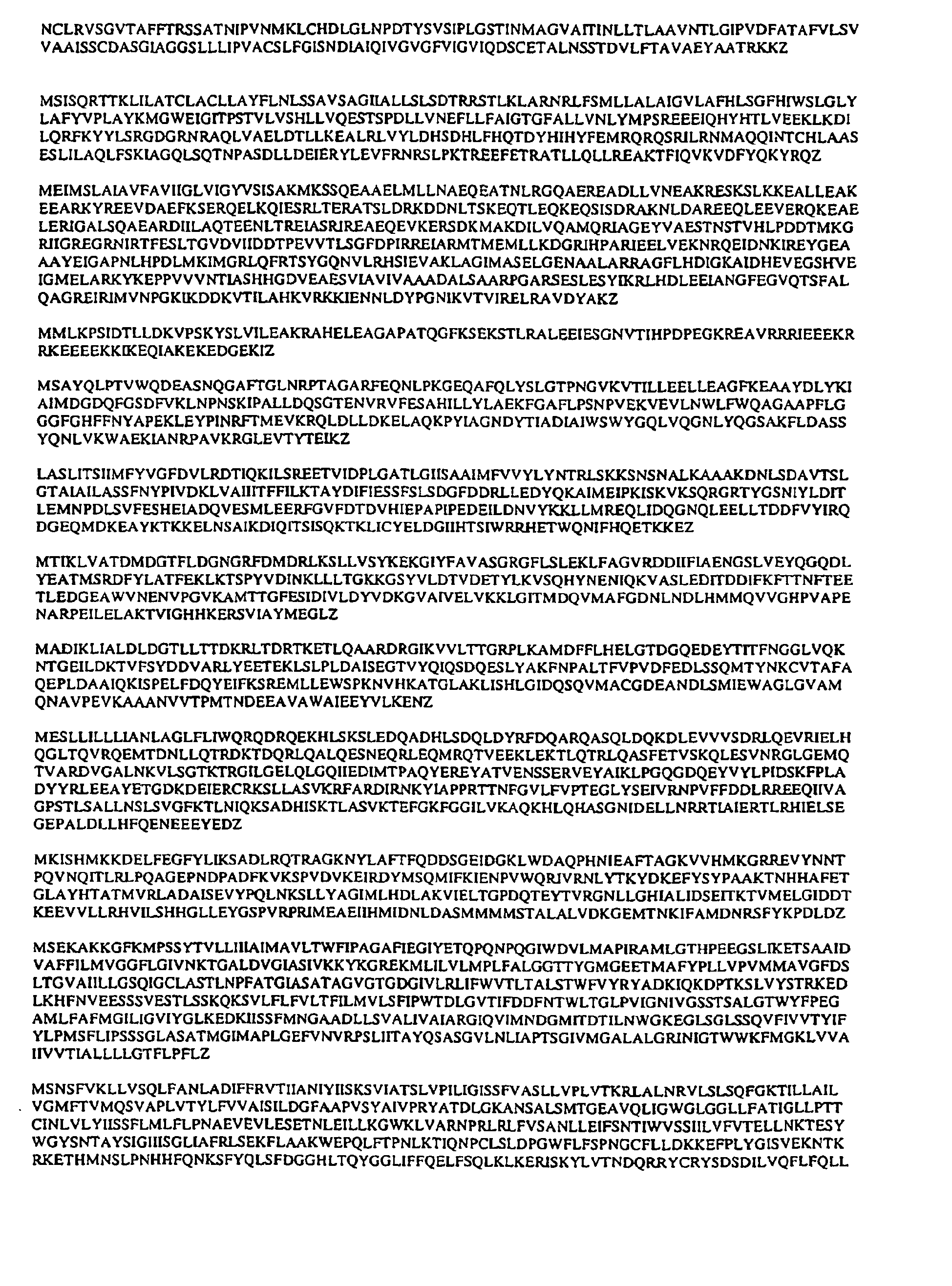

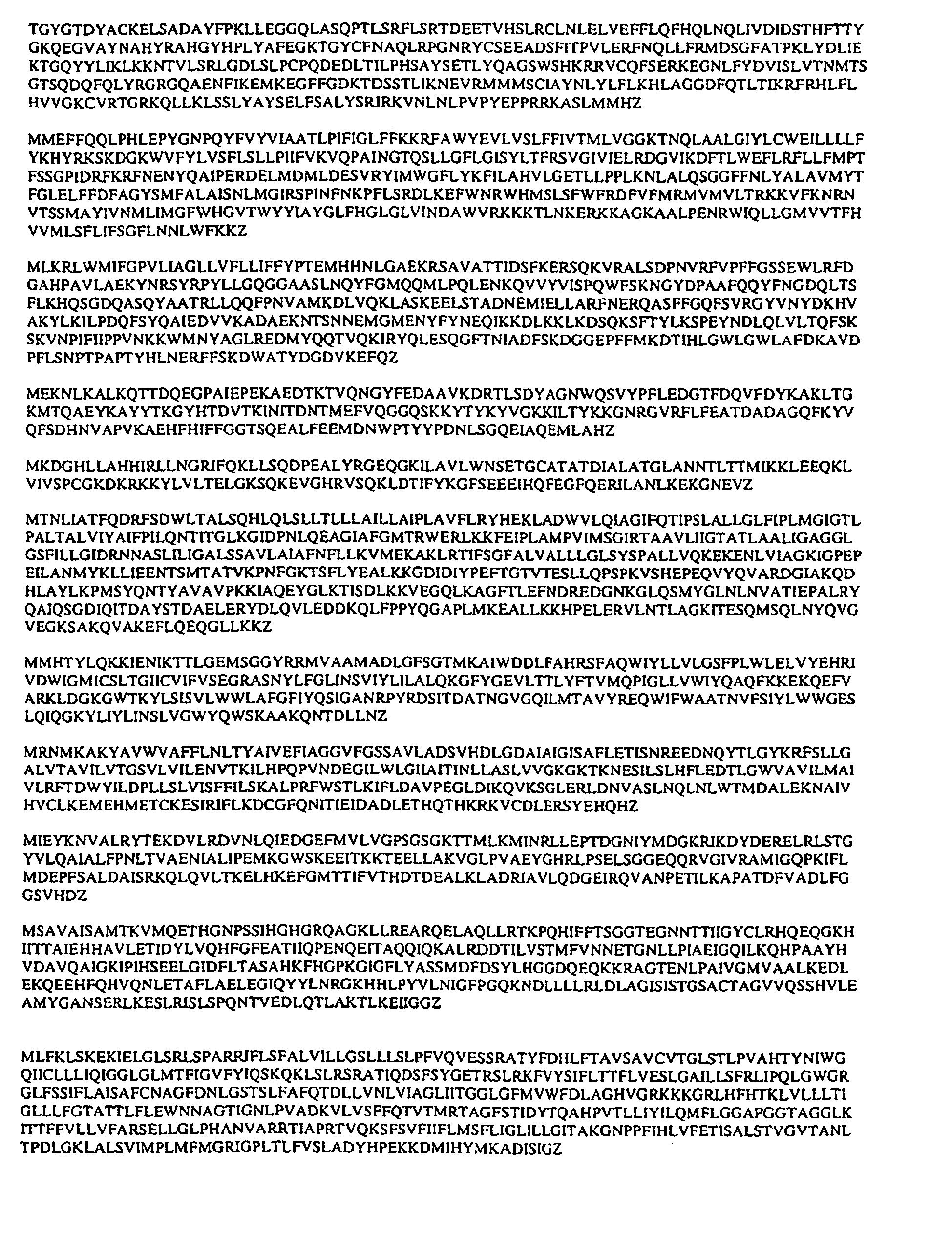

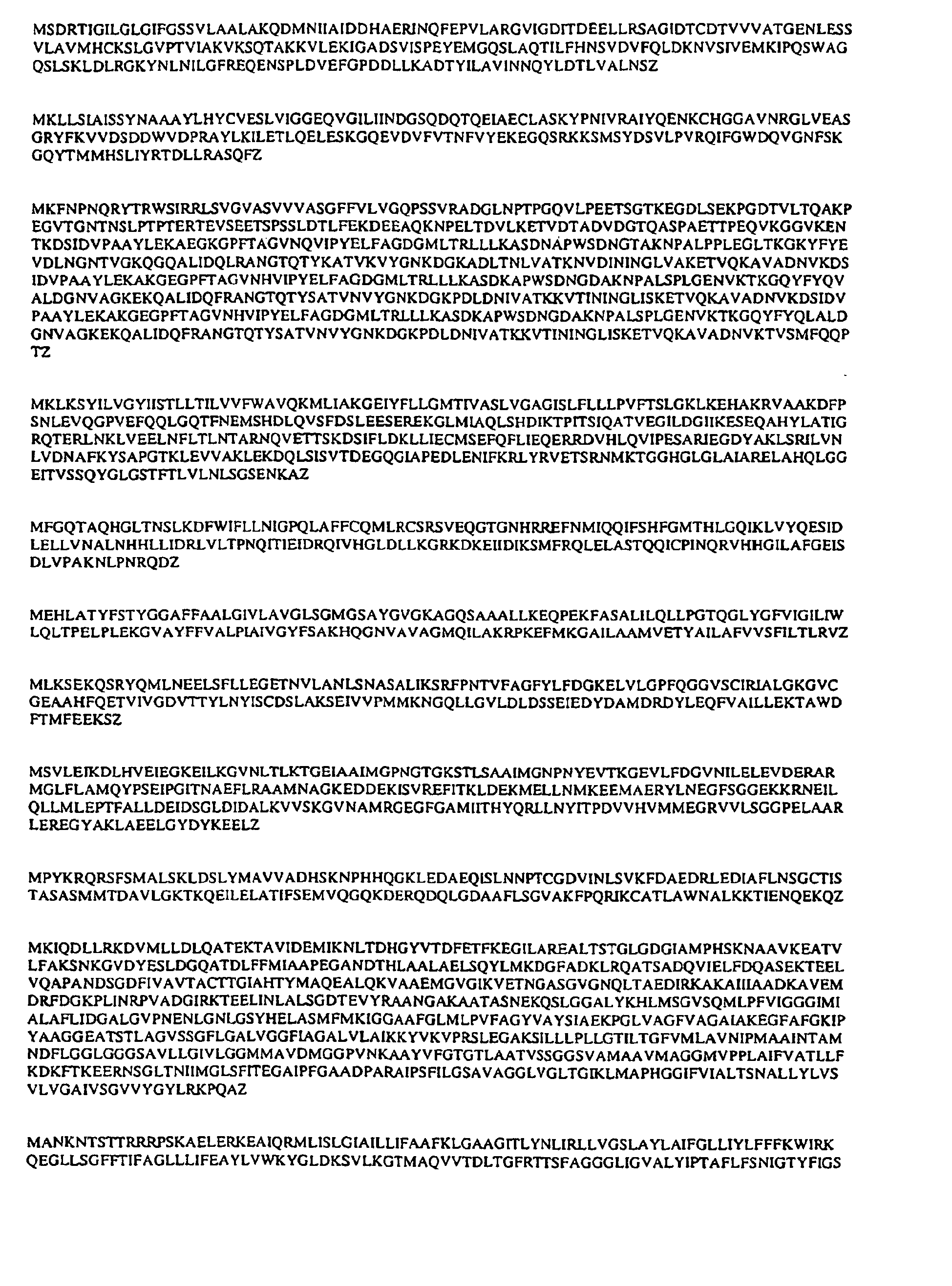

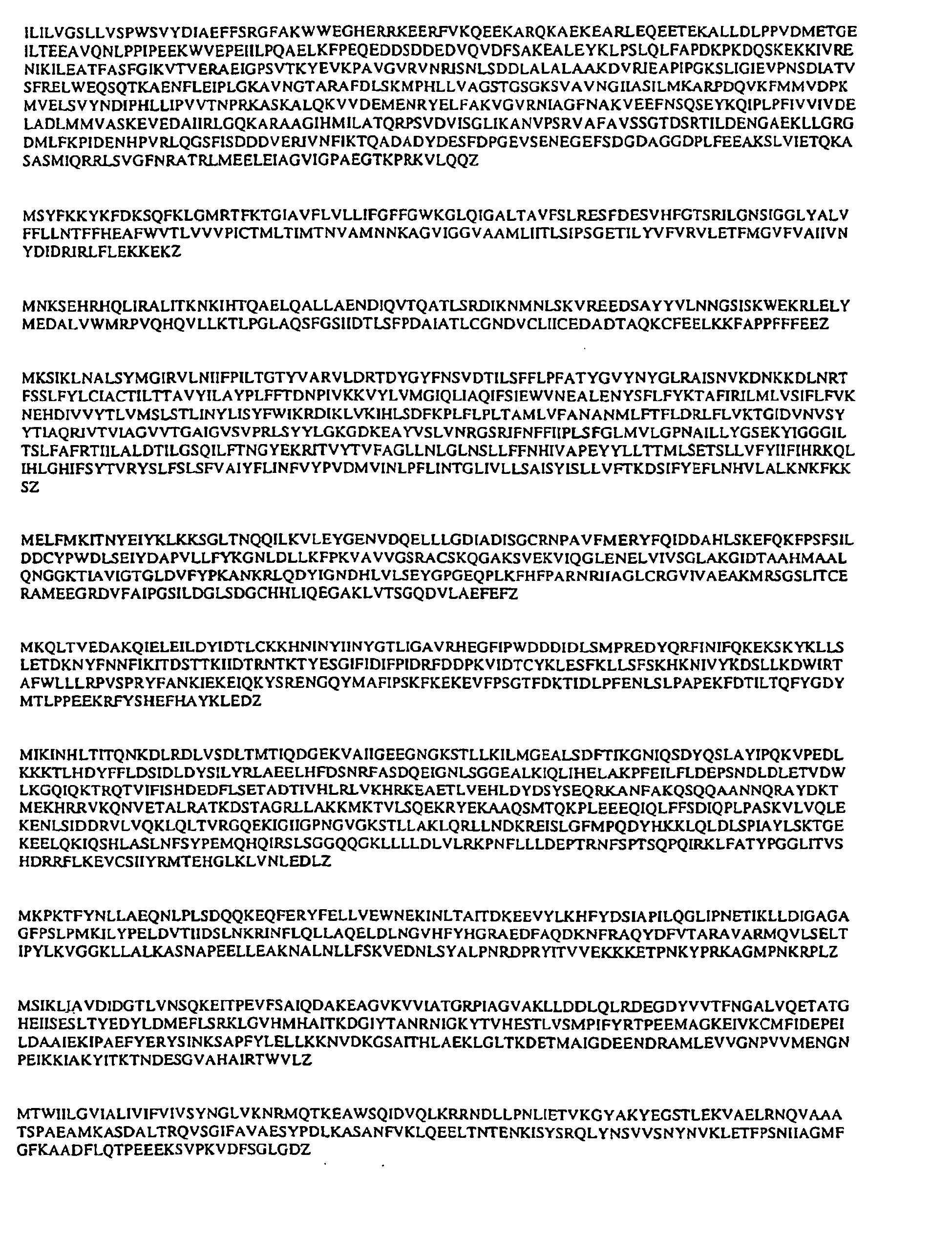

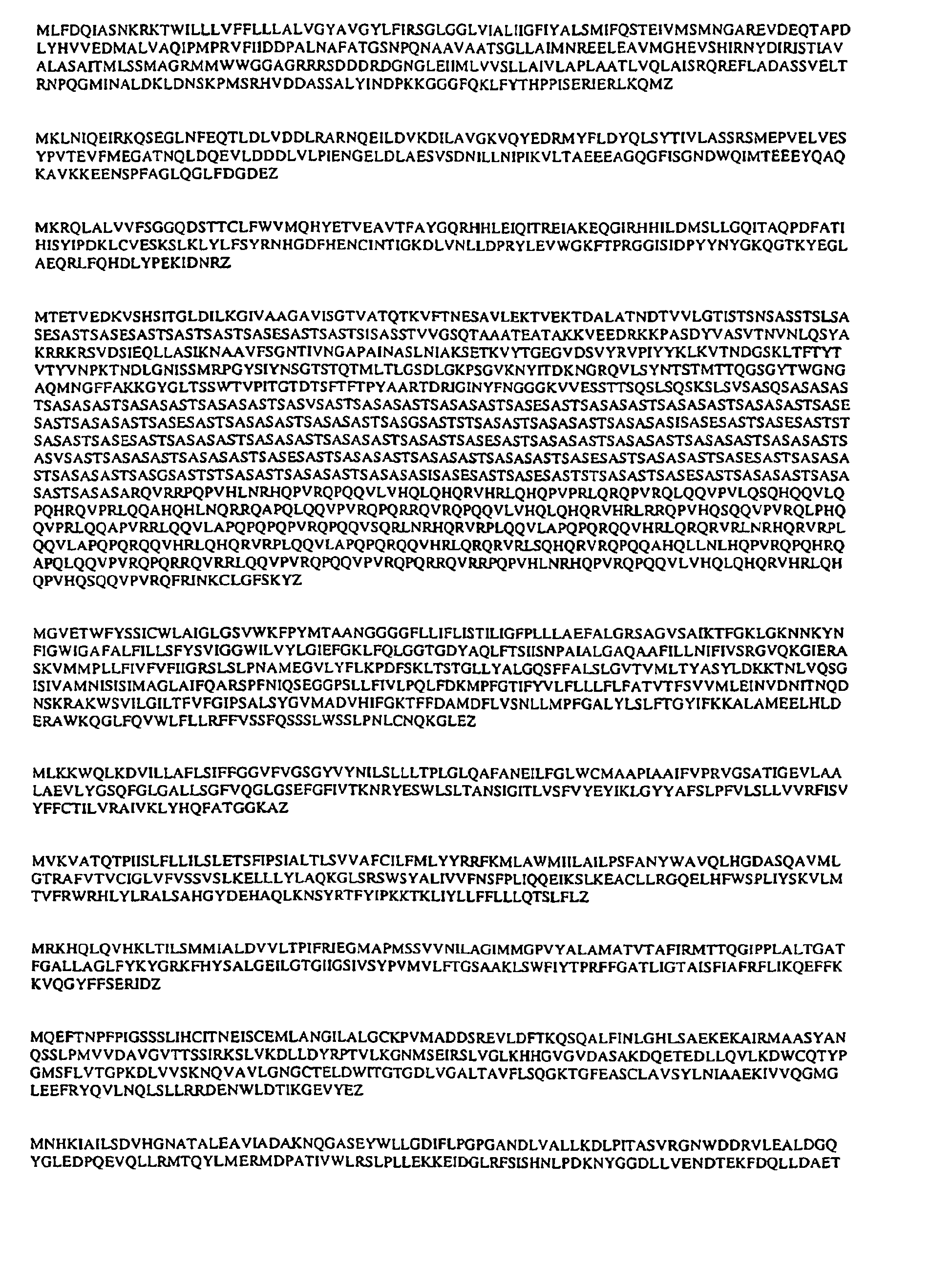

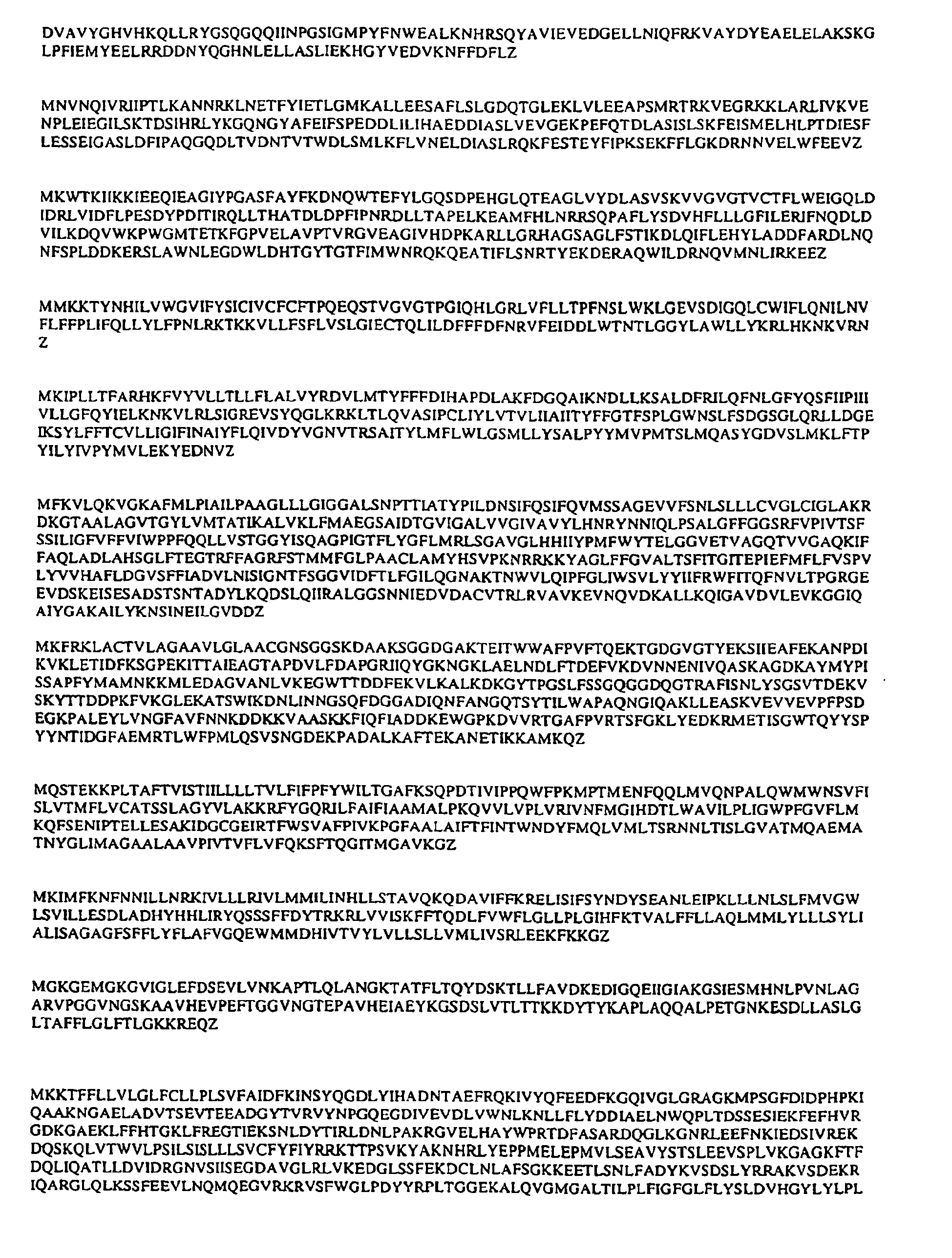

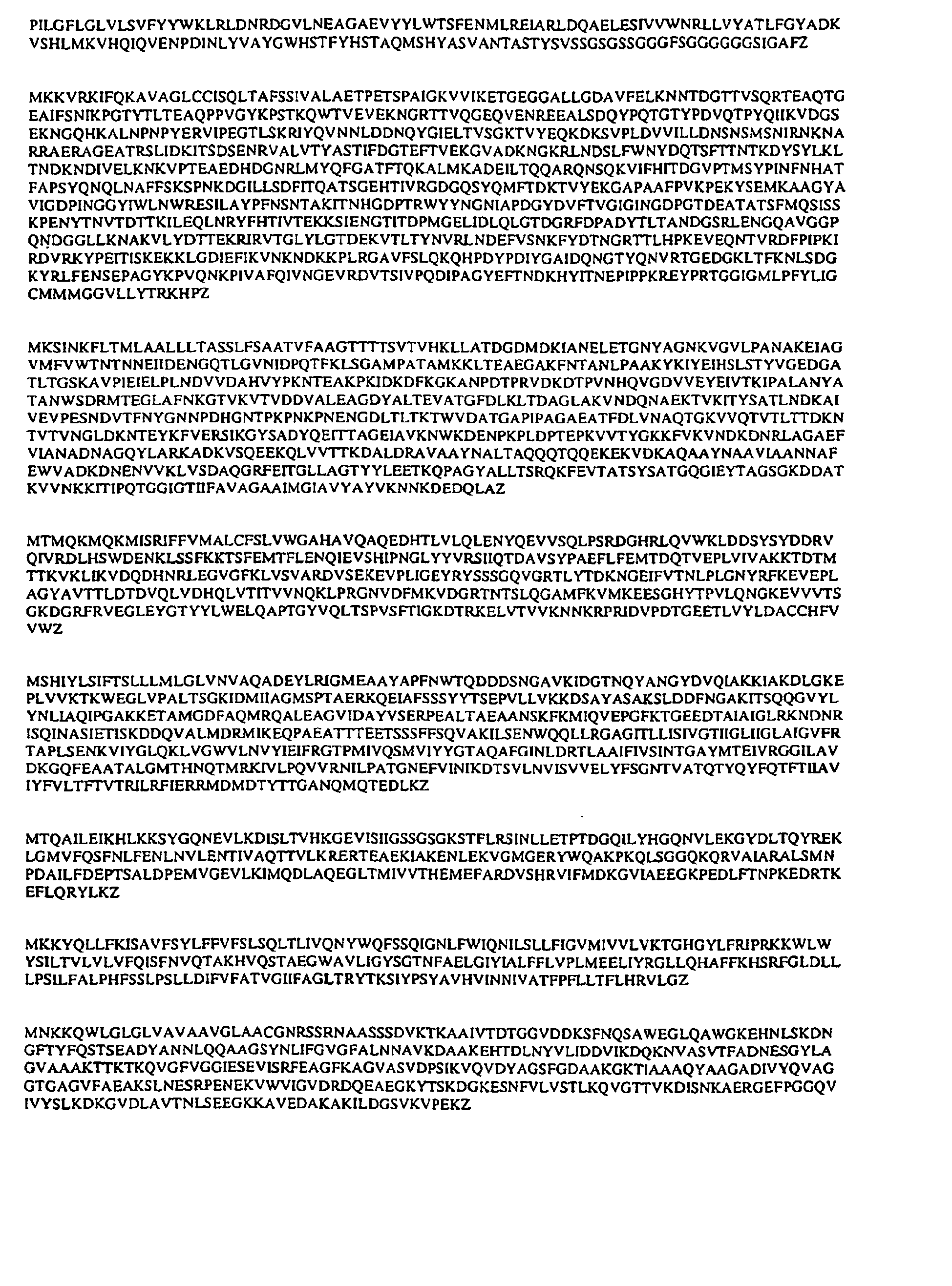

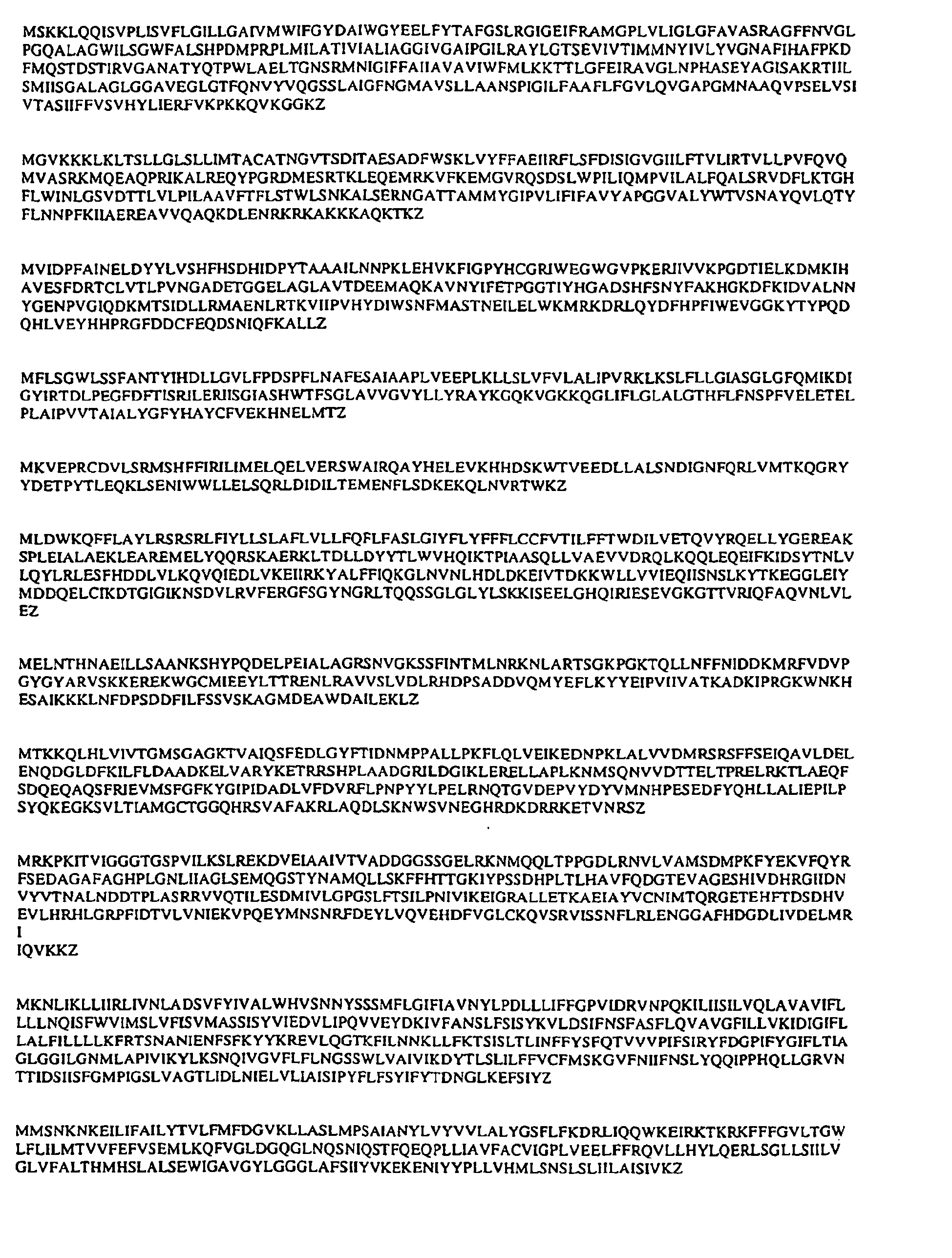

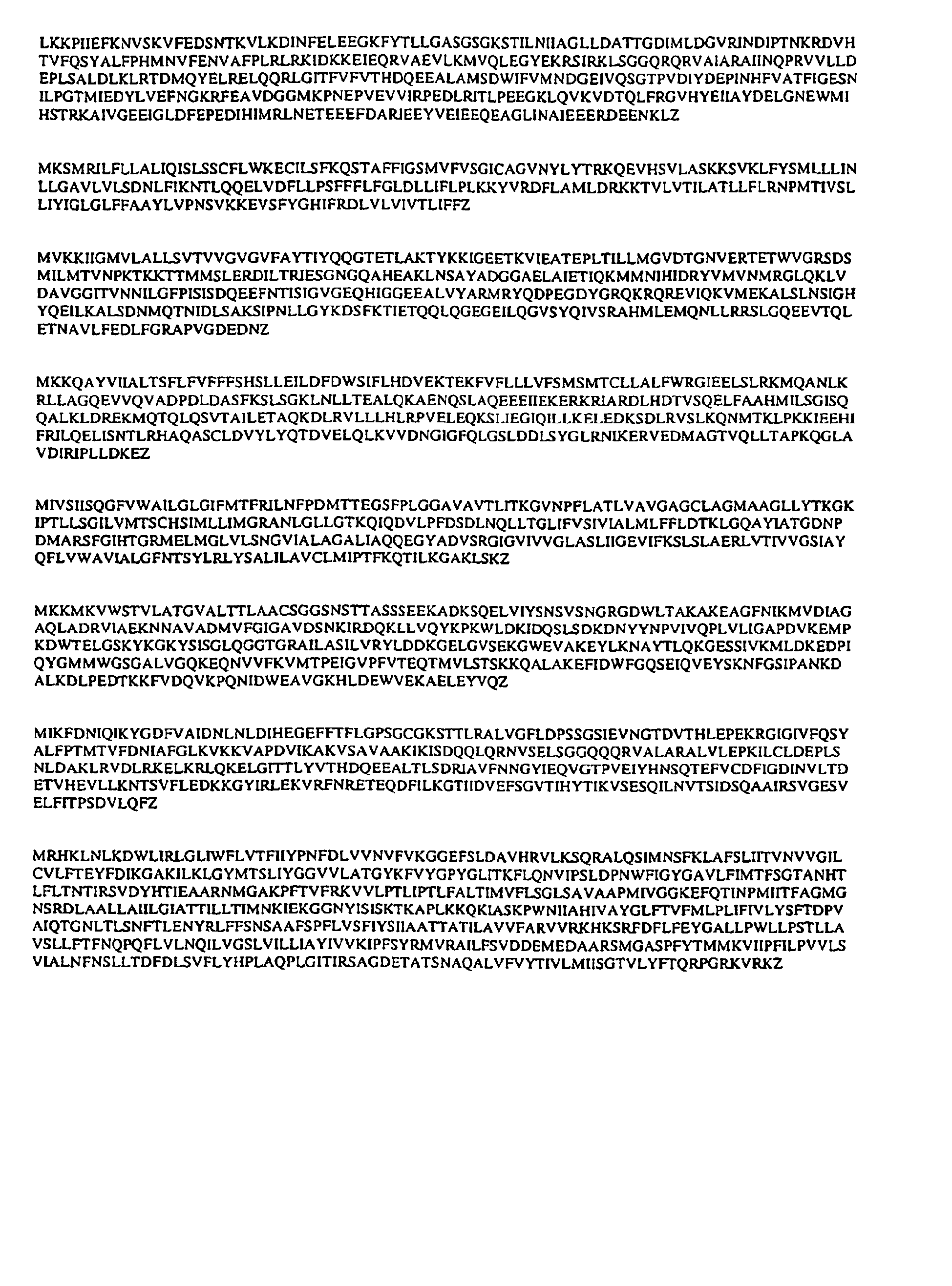

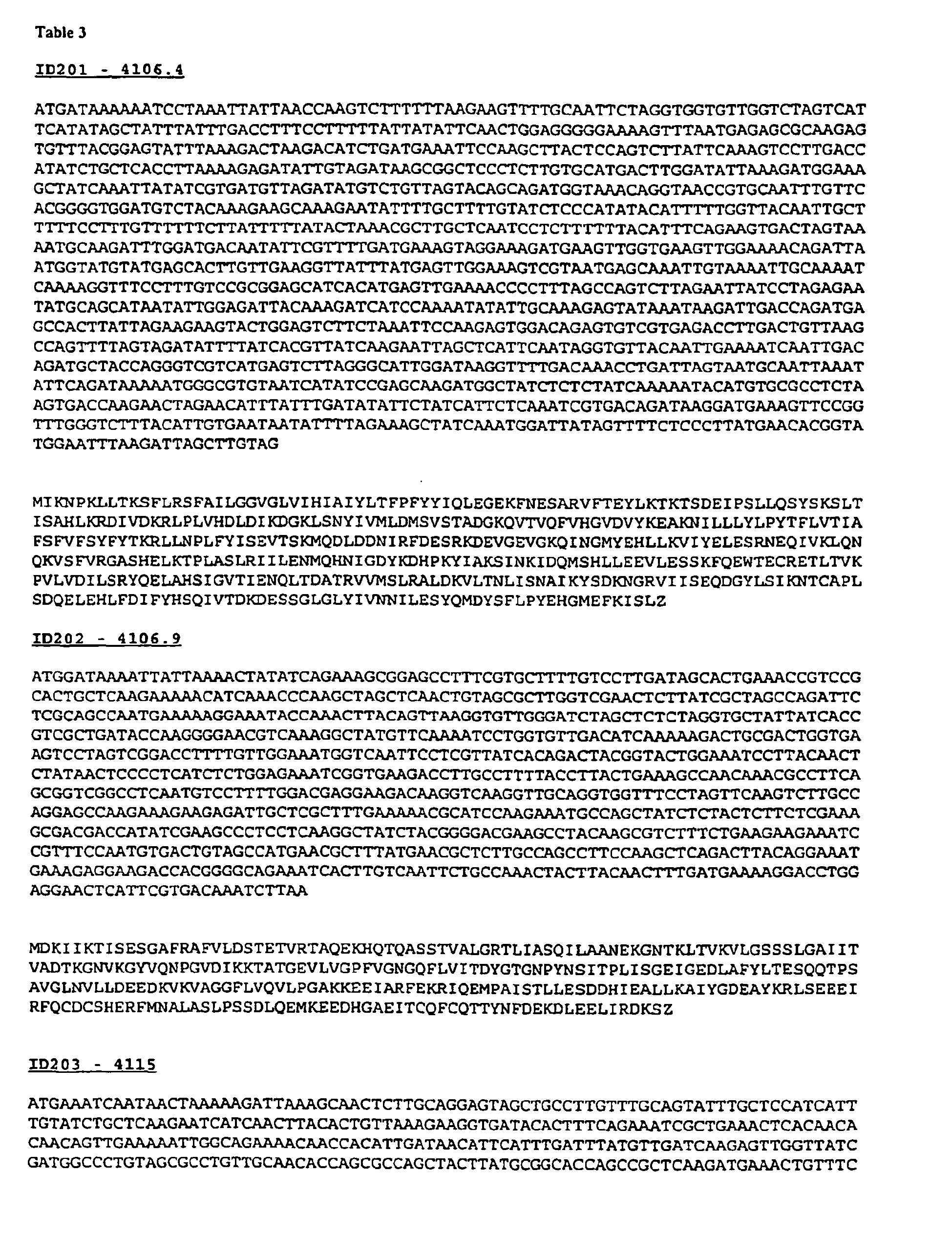

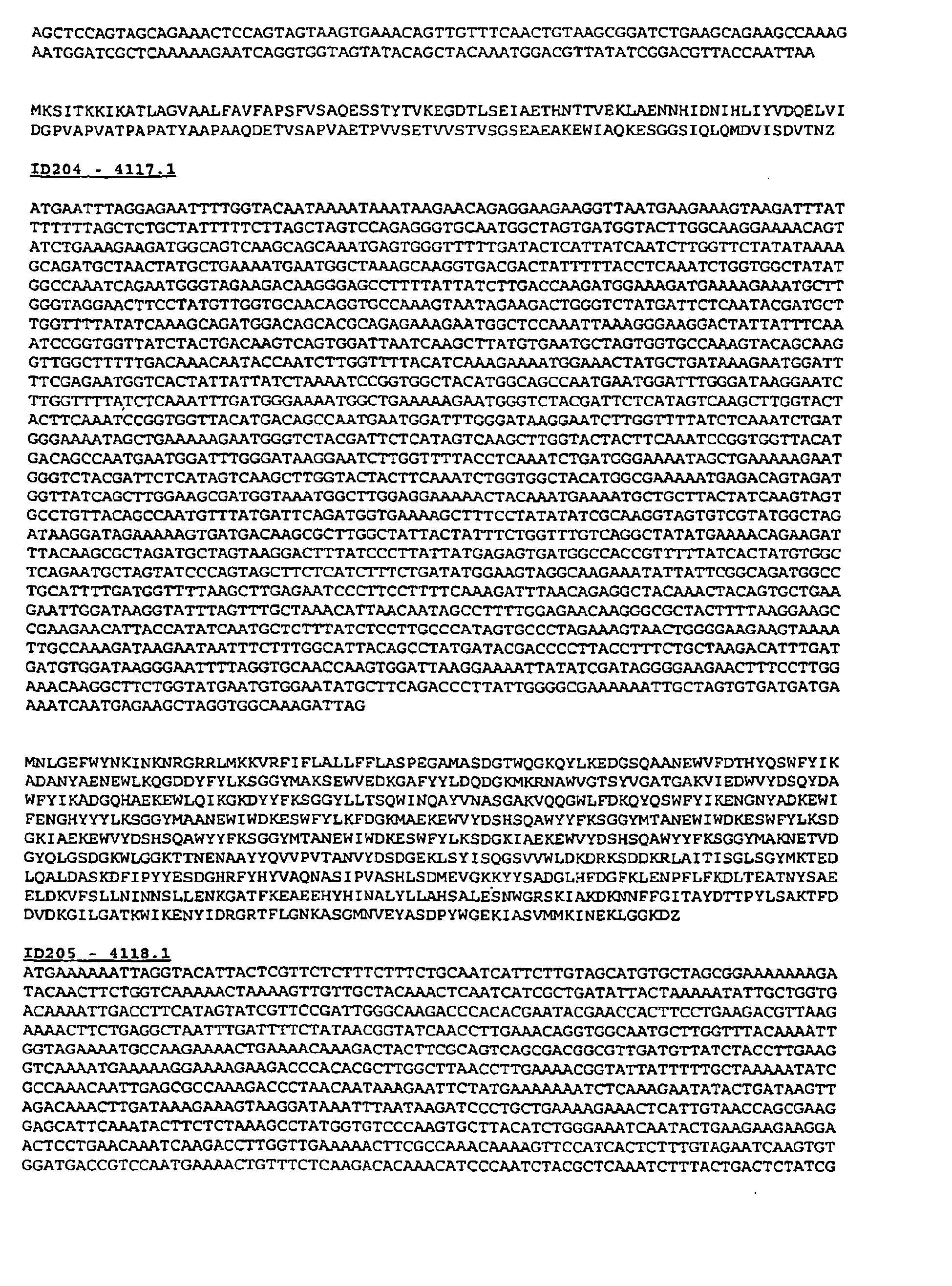

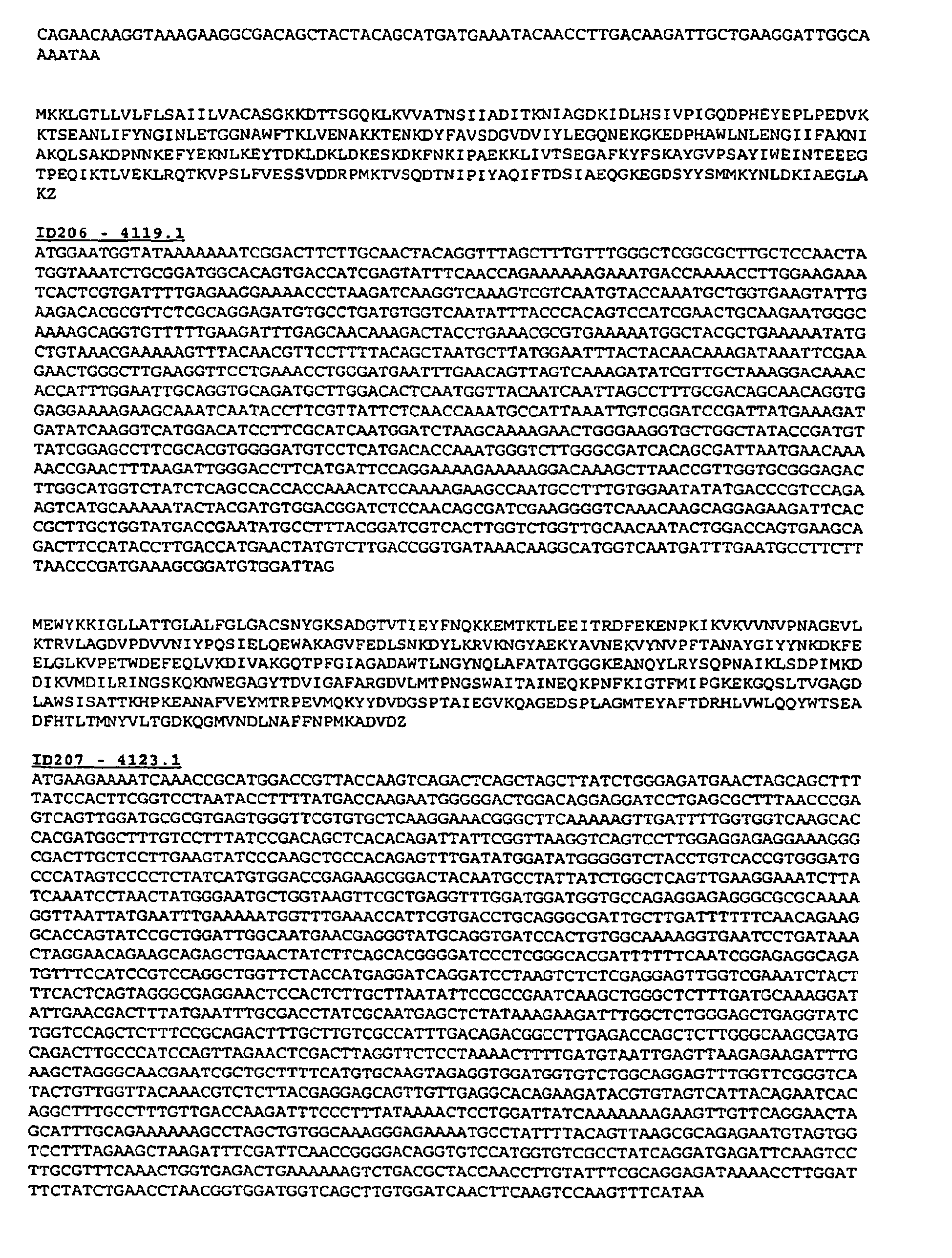

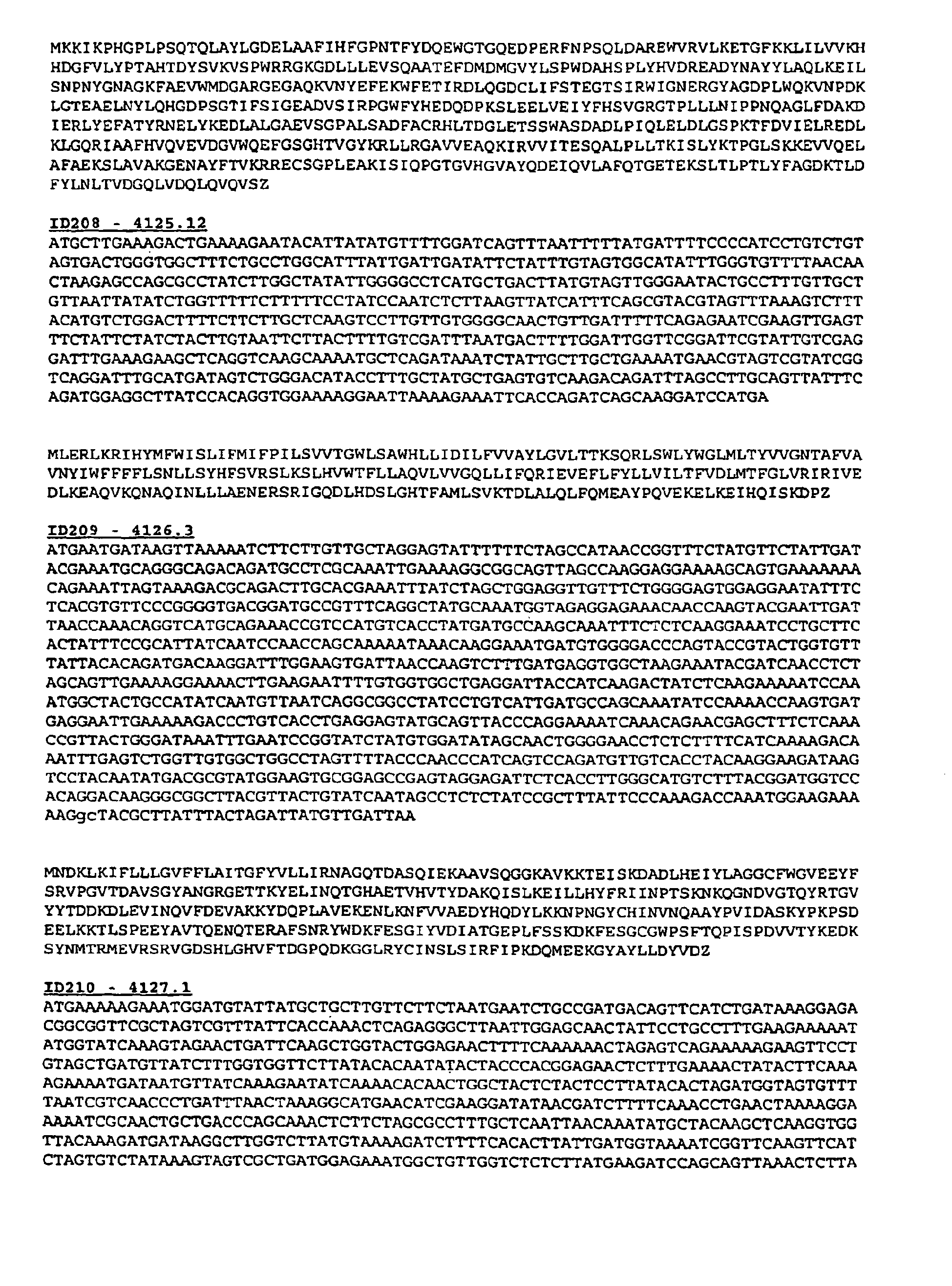

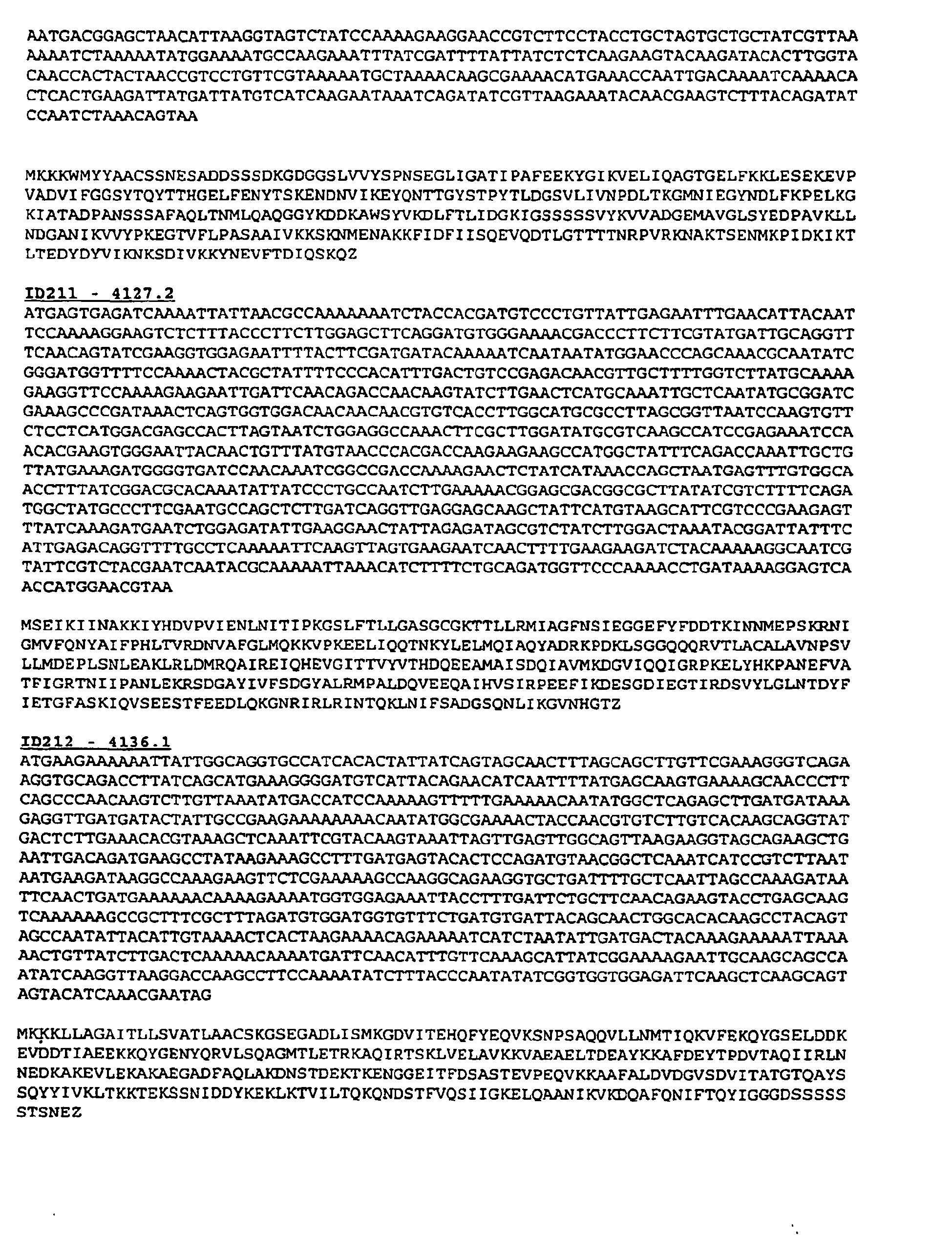

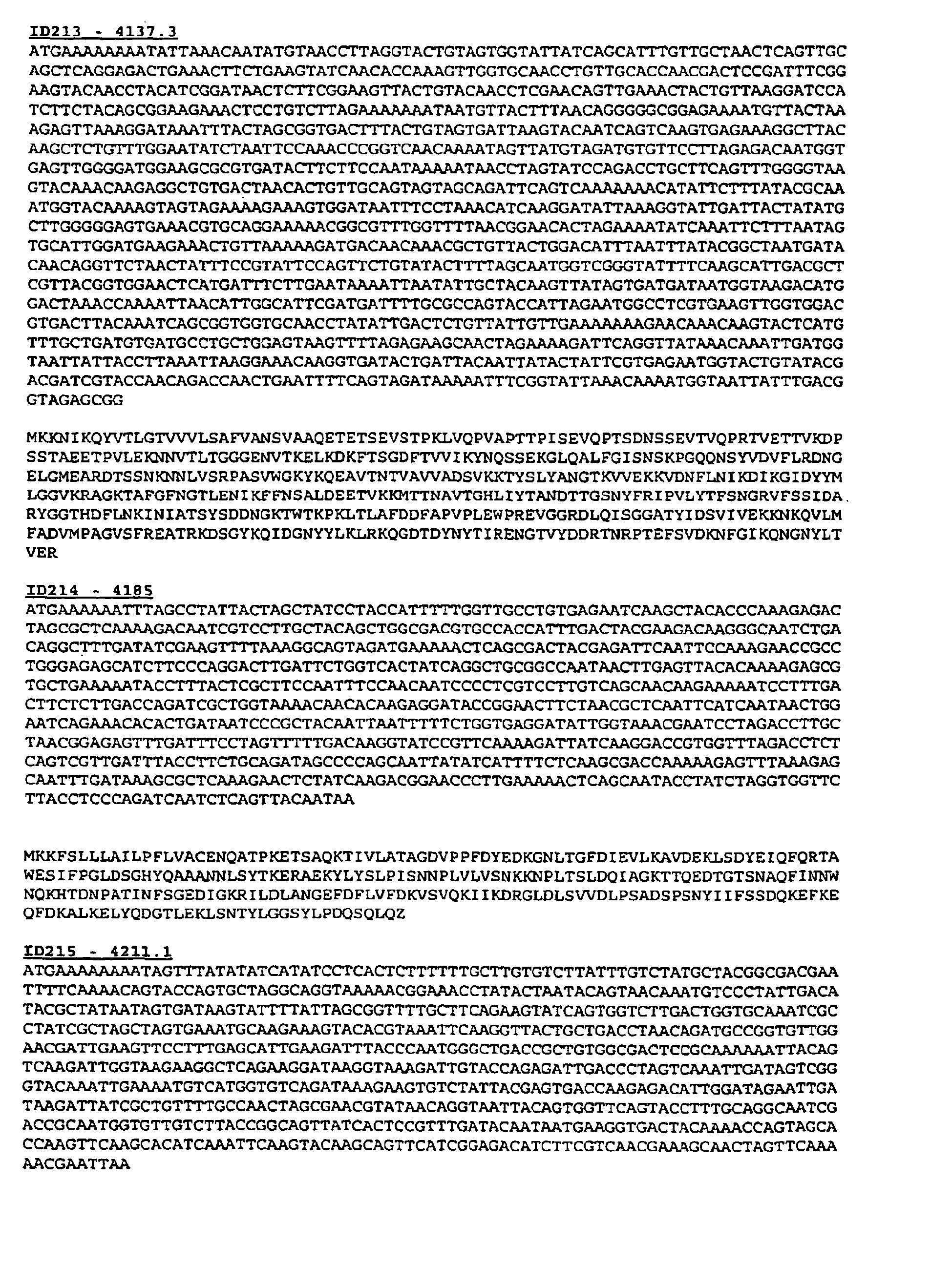

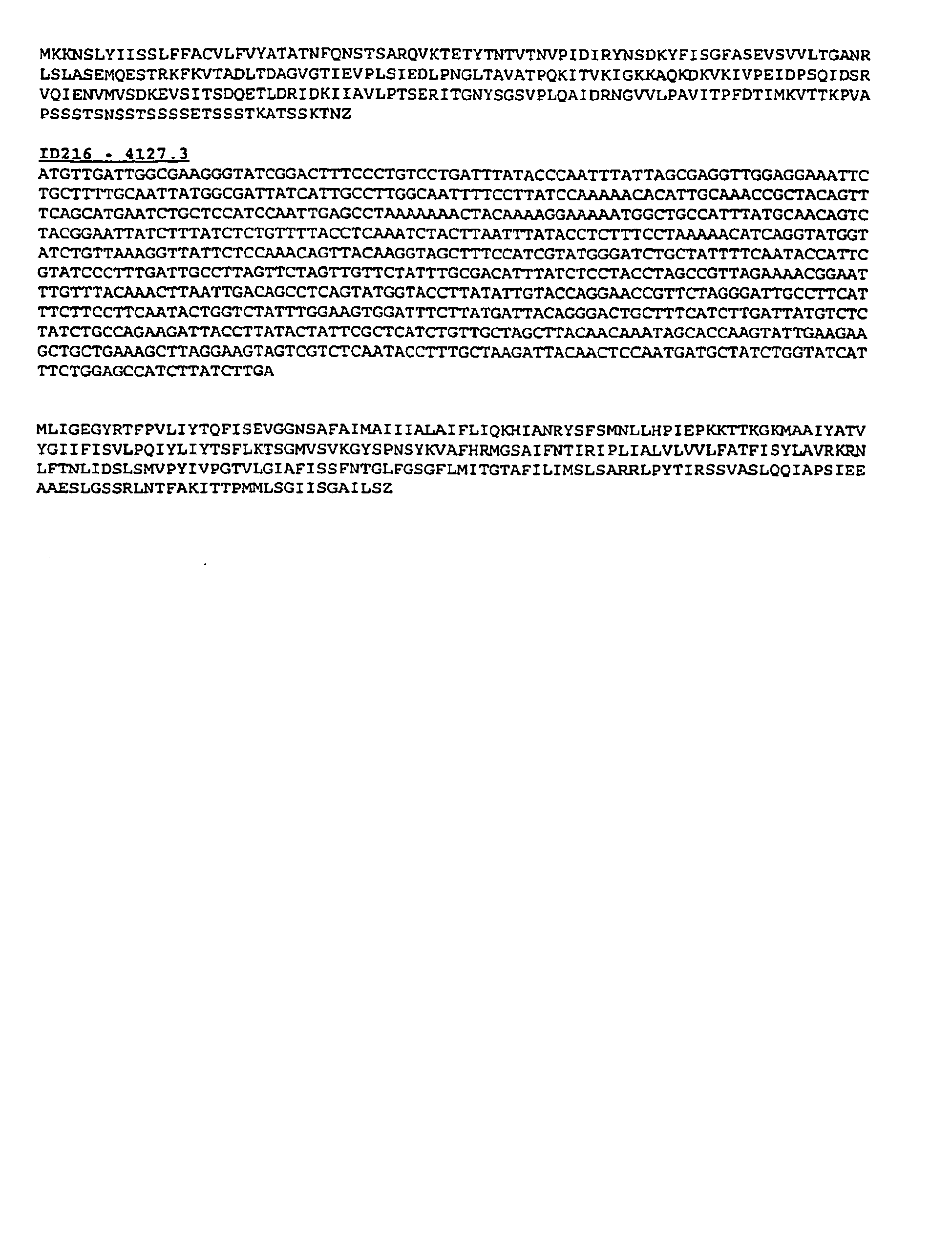

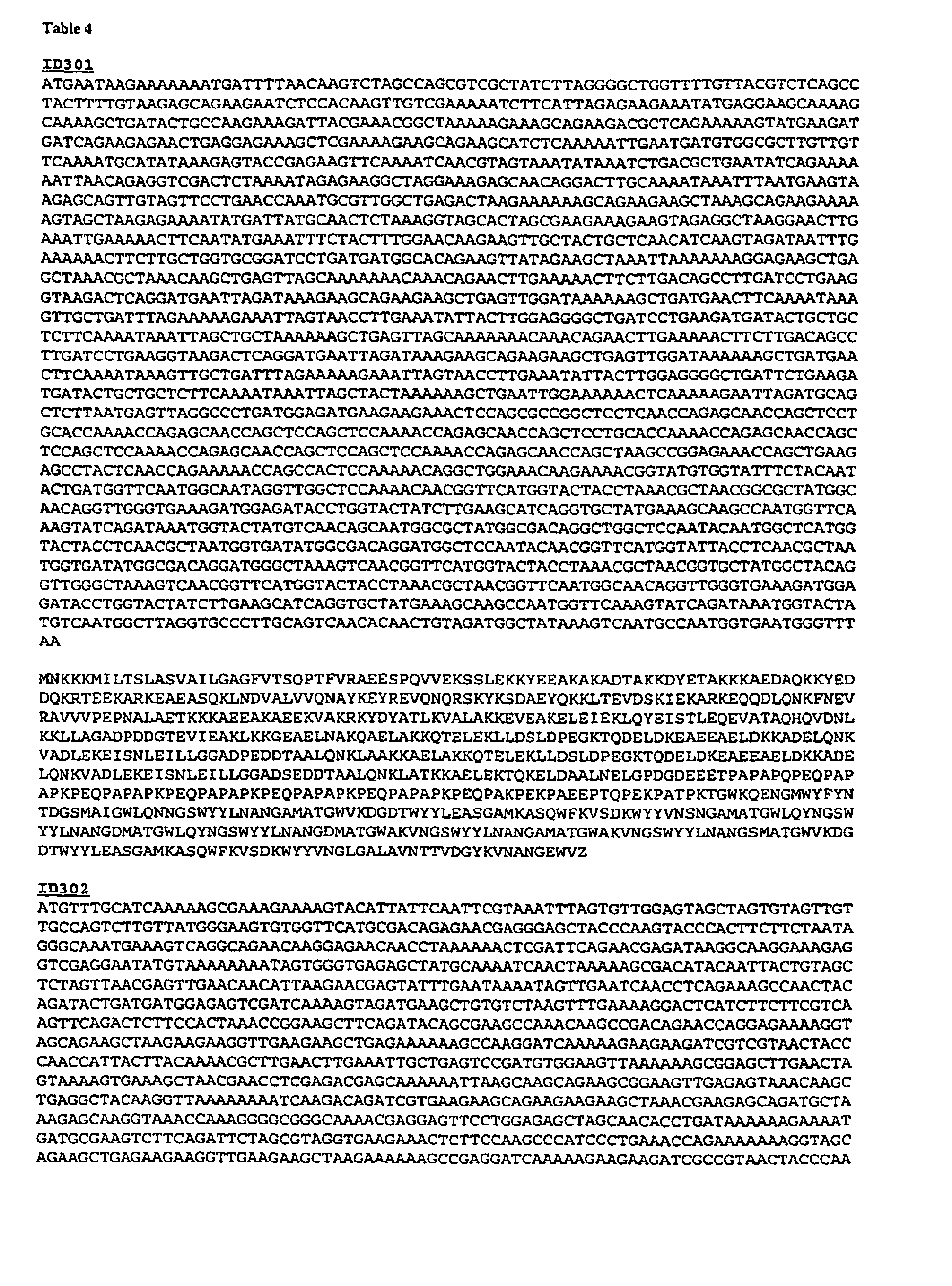

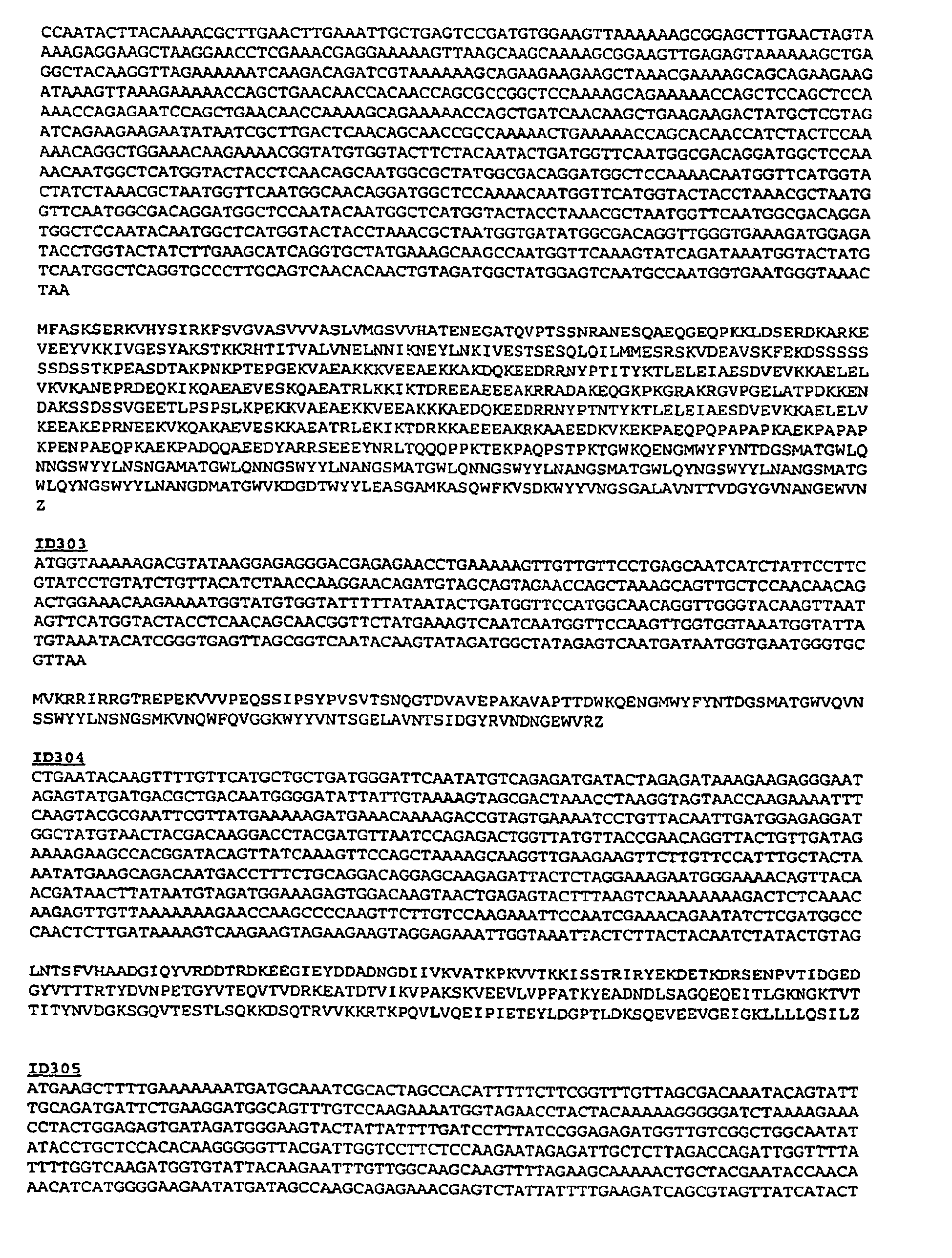

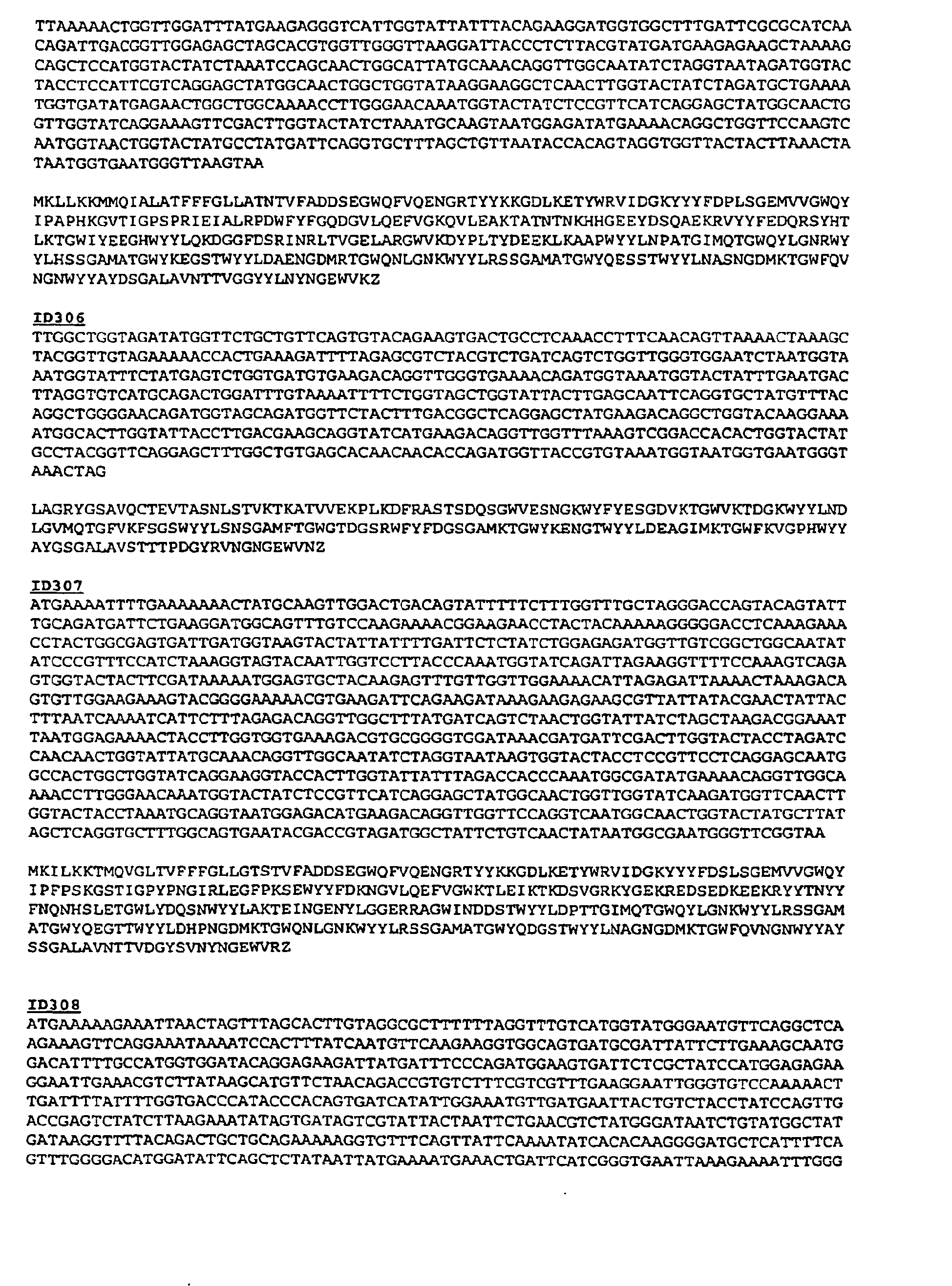

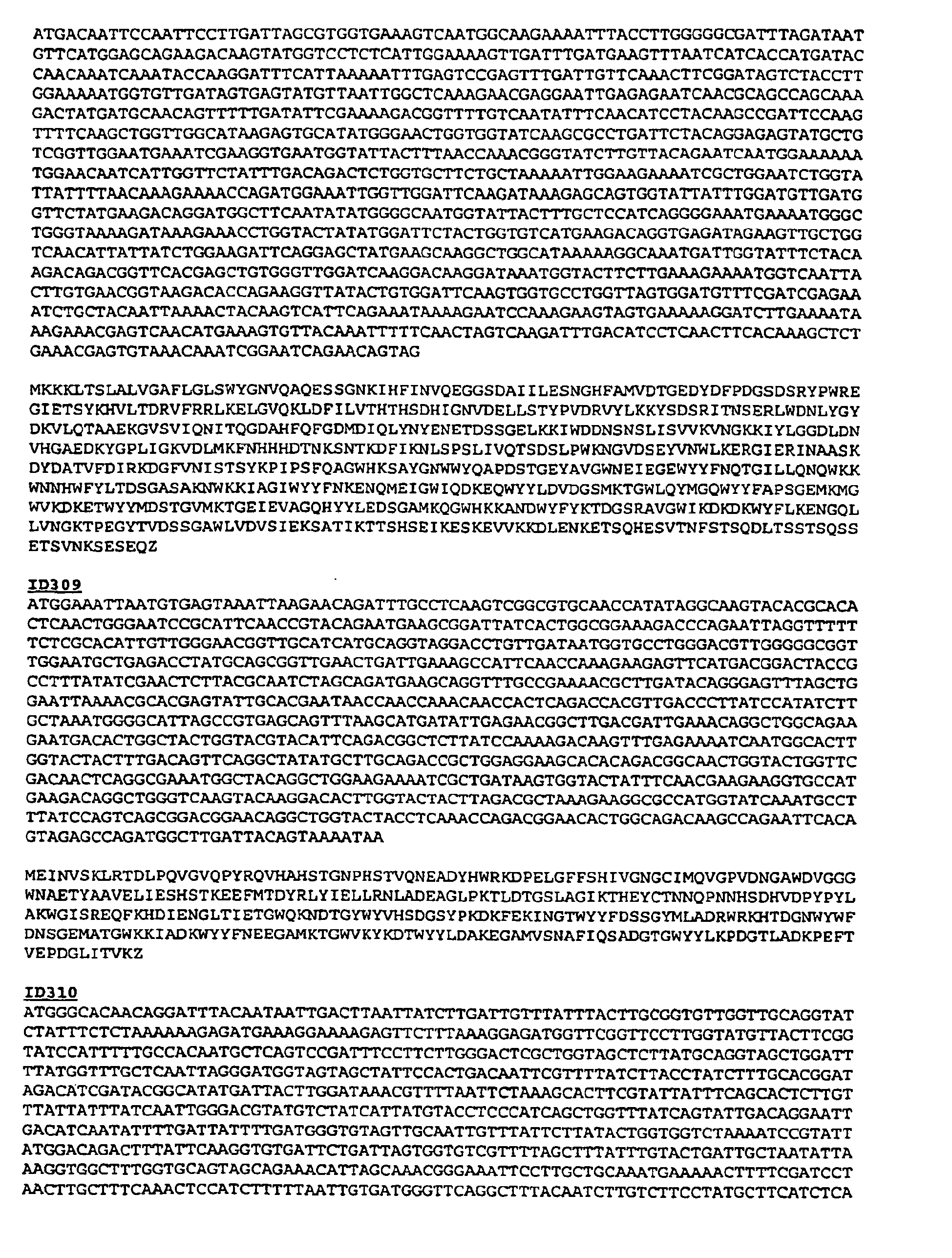

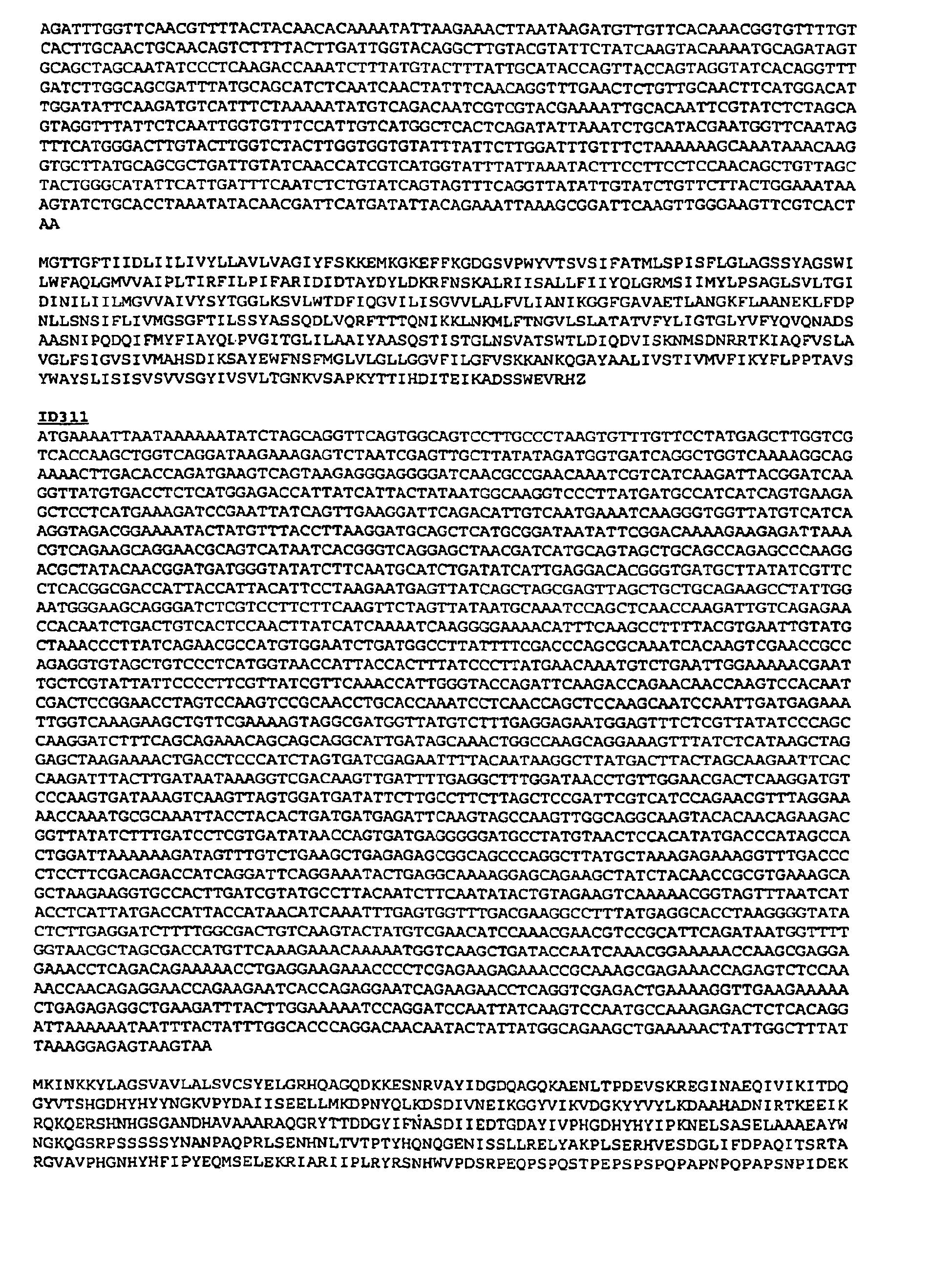

## Claims

1. A vaccine comprising:
(a) A *Streptococcus pneumoniae* protein or polypeptide having the sequence 
MSEIKIINAKKIYHDVPVIENLNITIPKGSLFTLLGASGCGKTTLLRMIAGFNSIE
GGEFYFDDTKINNMEPSKRNIGMVFQNYAIFPHLTVRDNVAFGLMQKKVPKE
ELIQQTNKYLELMQIAQYADRKPDKLSGGQQQRVTLACALAVNPSVLLMDE
PLSNLEAKLRLDMRQAIREIQHEVGITTVYVTHDQEEAMAISDQIAVMKDGVI
QQIGRPKELYHKPANEFVATFIGRTNIIPANLEKRSDGAYIVFSDGYALRMPAL
DQVEEQAIHVSIRPEEFIKDESGDIEGTIRDSVYLGLNTDYFIETGFASKIQVSEE
STFEEDLQKGNRIRLRINTQKLNIFSADGSQNLIKGVNHGT; and/or
(b) An immunogenic fragment of a protein or polypeptide of (a) wherein said fragment retains the immunogenic properties of said protein or polypeptide; and
one or more additional components selected from excipients, diluents, adjuvants or the like.

2. A vaccine composition comprising one or more nucleic acid sequences selected from:
i. 
ATGAGTGAGATCAAAATTATTAACGCCAAAAAAATCTACCACGATGT
CCCTGTTATTGAGAATTTGAACATTACAATTCCAAAAGGAAGTCTCTT
TACCCTTCTTGGAGCTTCAGGATGTGGAAAACGACCCTTCTTCGTAT
GATTGCAGGTTTCAACAGTATCGAAGGTGGAGAATTTTACTTCGATG
ATACAAAAATCAATAATATGGAACCCAGCAAACGCAATATCGGGAT
GGTTTTCCAAAACTACGCTATTTTCCCACATTTGACTGTCCGAGACAA
CGTTGCTTTTGGTCTTATGCAAAAGAAGGTTCCAAAAGAAGAATTGA
TTCAACAGACCAACAAGTATCTTGAACTCATGCAAATTGCTCAATAT
GCGGATCGAAAGCCCGATAAACTCAGTGGTGGACAACAACAACGTG
 
TCACCTTGGCATGCGCCTTAGCGGTTAATCCAAGTGTTCTCCTCATGG
ACGAGCCACTTAGTAATCTGGAGGCCAAACTTCGCTTGGATATGCGT
CAAGCCATCCGAGAAATCCAACACGAAGTGGGAATTACAACTGTTTA
TGTAACCCACGACCAAGAAGAAGCCATGGCTATTTCAGACCAAATTG
CTGTTATGAAAGATGGGGTGATCCAACAAATCGGCCGACCAAAAGA
ACTCTATCATAAACCAGCTAATGAGTTTGTGGCAACCTTTATCGGACG
CACAAATATTATCCCTGCCAATCTTGAAAAACGGAGCGACGGCGCTT
ATATCGTCTTTTCAGATGGCTATGCCCTTCGAATGCCAGCTCTTGATC
AGGTTGAGGAGCAAGCTATTCATGTAAGCATTCGTCCCGAAGAGTTT
ATCAAAGATGAATCTGGAGATATTGAAGGAACTATTAGAGATAGCGT
CTATCTTGGACTAAATACGGATTATTTCATTGAGACAGGTTTTGCCTC
AAAAATTCAAGTTAGTGAAGAATCAACTTTTGAAGAAGATCTACAAA
AAGGCAATCGTATTCGTCTACGAATCAATACGCAAAAATTAAACATC
TTTTCTGCAGATGGTTCCCAAAACCTGATAAAAGGAGTCAACCATGG
AACGTAA or its RNA equivalent;
ii. a sequence which is complementary to the sequences of (i);
iii. a sequence which codes for the same protein or polypeptide, as those sequences of (i);
iv. a sequence which has at least 80% sequence identity with any of those of (i), (ii) and (iii) wherein the protein or polypeptide encoded by the coding strand retains the immunogenic properties of the protein or polypeptide encoded by the sequence of (i);
v. a sequence which codes for an immunogenic fragment of a protein or polypeptide encoded by the sequence of (i) wherein said fragment retains the immunogenic properties of said protein or polypeptide.

## Patentansprüche

1. Impfstoff, der aufweist:
(a) Ein Streptococcus pneumoniae Protein oder Polypeptid mit der Sequenz 
MSEIKIINAKKIYHDVPVIENLNITIPKGSLFTLLGASGCGKTTLLRMIAGFNSIE
GGEFYFDDTKINNMEPSKRNIGMVFQNYAIFPHLTVRDNVAFGLMQKKVPKE
ELIQQTNKYLELMQIAQYADRKPDKLSGGQQQRVTLACALAVNPSVLLMDE
PLSNLEAKLRLDMRQAIREIQHEVGITTVYVTHDQEEAMAISDQIAVMKDGVI
QQIGRPKELYHKPANEFVATFIGRTNIIPANLEKRSDGAYIVFSDGYALRMPAL
DQVEEQAIHVSIRPEEFIKDESGDIEGTIRDSVYLGLNTDYFIETGFASKIQVSEE
STFEEDLQKGNRIRLRINTQKLNIFSADGSQNLIKGVNHGT; und/oder
(b) Ein immunogenes Fragment eines Proteins oder Polypeptids von (a), wobei besagtes Fragment die immunogenen Eigenschaften des besagten Proteins oder Polypeptids beibehält; und
eine oder mehrere zusätzliche Komponenten, die ausgewählt sind aus Exzipienten, Verdünnungsmittel, Adjuvanzien oder dergleichen.

2. Impfstoff-Zusammensetzung, die eine oder mehrere Nukleinsäuresequenzen aufweist, die ausgewählt sind aus:
i. 
ATGAGTGAGATCAAAATTATTAACGCCAAAAAAATCTACCACGATGT
CCCTGTTATTGAGAATTTGAACATTACAATTCCAAAAGGAAGTCTCTT
TACCCTTCTTGGAGCTTCAGGATGTGGAAAACGACCCTTCTTCGTAT
GATTGCAGGTTTCAACAGTATCGAAGGTGGAGAATTTTACTTCGATG
ATACAAAAATCAATAATATGGAACCCAGCAAACGCAATATCGGGAT
GGTTTTCCAAAACTACGCTATTTTCCCACATTTGACTGTCCGAGACAA
CGTTGCTTTTGGTCTTATGCAAAAGAAGGTTCCAAAAGAAGAATTGA
TTCAACAGACCAACAAGTATCTTGAACTCATGCAAATTGCTCAATAT
GCGGATCGAAAGCCCGATAAACTCAGTGGTGGACAACAACAACGTG

TCACCTTGGCATGCGCCTTAGCGGTTAATCCAAGTGTTCTCCTCATGG
ACGAGCCACTTAGTAATCTGGAGGCCAAACTTCGCTTGGATATGCGT
CAAGCCATCCGAGAAATCCAACACGAAGTGGGAATTACAACTGTTTA
TGTAACCCACGACCAAGAAGAAGCCATGGCTATTTCAGACCAAATTG
CTGTTATGAAAGATGGGGTGATCCAACAAATCGGCCGACCAAAAGA
ACTCTATCATAAACCAGCTAATGAGTTTGTGGCAACCTTTATCGGACG
CACAAATATTATCCCTGCCAATCTTGAAAAACGGAGCGACGGCGCTT
ATATCGTCTTTTCAGATGGCTATGCCCTTCGAATGCCAGCTCTTGATC
AGGTTGAGGAGCAAGCTATTCATGTAAGCATTCGTCCCGAAGAGTTT
ATCAAAGATGAATCTGGAGATATTGAAGGAACTATTAGAGATAGCGT
CTATCTTGGACTAAATACGGATTATTTCATTGAGACAGGTTTTGCCTC
AAAAATTCAAGTTAGTGAAGAATCAACTTTTGAAGAAGATCTACAAA
AAGGCAATCGTATTCGTCTACGAATCAATACGCAAAAATTAAACATC
TTTTCTGCAGATGGTTCCCAAAACCTGATAAAAGGAGTCAACCATGG
AACGTAA oder sein RNA Äquivalent;
ii. einer Sequenz, die zu den Sequenzen von (i) komplementär ist;
iii. einer Sequenz, die dasselbe Protein oder Polypeptid kodiert, wie jene Sequenzen von (i);
iv. einer Sequenz, die zumindest eine 80% Sequenzidentität mit einer beliebigen von jenen von (i), (ii) und (iii) aufweist, wobei das Protein oder Polypeptid, das durch den kodierenden Strang kodiert ist, die immunogenen Eigenschaften des Proteins oder Polypeptids, das durch die Sequenz von (i) kodiert ist, beibehält;
v. einer Sequenz, die ein immunogenes Fragment eines Proteins oder Polypeptids, das durch die Sequenz von (i) kodiert ist, kodiert, wobei besagtes Fragment die immunogenen Eigenschaften des besagten Proteins oder Polypeptids beibehält.

## Revendications

1. Vaccin comprenant :
(a) une protéine ou un polypeptide de *Streptococcus pneumoniae* ayant la séquence 
MSEIKIINAKKIYHDVPVIENLNITIPKGSLFTLLGASGCGKTTLLRMIAGFNSIE
GGEFYFDDTKINNMEPSKRNIGMVFQNYAIFPHLTVRDNVAFGLMQKKVPKE
ELIQQTNKYLELMQIAQYADRKPDKLSGGQQQRVTLACALAVNPSVLLMDE
PLSNLEAKLRLDMRQAIREIQHEVGITTVYVTHDQEEAMAISDQIAVMKDGVI
QQIGRPKELYHKPANEFVATFIGRTNIIPANLEKRSDGAYTVFSDGYALRMPAL
DQVEEQAIHVSIRPEHFIKDESGDIEGTIRDSVYLGLNTDYFIETGFASKIQVSEE
STFEEDLQKGNRIRLRINTQKLNIFSADGSQNLIKGVNHGT;
 et/ou
(b) un fragment immunogène d'une protéine ou d'un polypeptide de (a) dans lequel ledit fragment conserve les propriétés immunogènes de ladite protéine ou dudit polypeptide ; et
un ou plusieurs composants additionnels sélectionnés parmi des excipients, diluants, adjuvants, ou similaires.

2. Composition vaccinale comprenant une ou plusieurs séquences d'acide nucléique sélectionnées parmi :
i. 
ATGAGTGAGATCAAAATTATTAACGCCAAAAAAATCTACCACGATGT
CCCTGTTATTGAGAATTTGAACATTACAATTCCAAAAGGAAGTCTCTT
TACCCTTCTTGGAGCTTCAGGATGTGGGAAAACGACCCTTCTTCGTAT
GATTGCAGGTTTCAACAGTATCGAAGGTGGAGAATTTTACTTCGATG
ATACAAAAATCAATAATATGGAACCCAGCAAACGCAATATCGGGAT
GGTTTTCCAAAACTACGCTATTTTCCCACATTTGACTGTCCGAGACAA
CGTTGCTTTTGGTCTTATGCAAAAGAAGGTTCCAAAAGAAGAATTGA
TTCAACAGACCAACAAGTATCTTGAACTCATGCAAATTGCTCAATAT
GCGGATCGAAAGCCCGATAAACTCAGTGGTGGACAACAACAACGTG
 
TCACCTTGGCATGCGCCTTAGCGGTTAATCCAAGTGTTCTCCTCATGG
ACGAGCCACTTAGTAATCTGGAGGCCAAACTTCGCTTGGATATGCGT
CAAGCCATCCGAGAAATCCAACACGAAGTGGGAATTACAACTGTTTA
TGTAACCCACGACCAAGAAGAAGCCATGGCTATTTCAGACCAAATTG
CTGTTATGAAAGATGGGGTGATCCAACAAATCGGCCGACCAAAAGA
ACTCTATCATAAACCAGCTAATGAGTTTGTGGCAACCTTTATCGGACG
CACAAATATTATCCCTGCCAATCTTGAAAAACGGAGCGACGGCGCTT
ATATCGTCTTTTCAGATGGCTATGCCCTTCGAATGCCAGCTCTTGATC
AGGTTGAGGAGCAAGCTATTCATGTAAGCATTCGTCCCGAAGAGTTT
ATCAAAGATGAATCTGGAGATATTGAAGGAACTATTAGAGATAGCGT
CTATCTTGGACTAAATACGGATTATTTCATTGAGACAGGTTTTGCCTC
AAAAATTCAAGTTAGTGAAGAATCAACTTTTGAAGAAGATCTACAAA
AAGGCAATCGTATTCGTCTACGAATCAATACGCAAAAATTAAACATC
TTTTCTGCAGATGGTTCCCAAAACCTGATAAAAGGAGTCAACCATGG
AACGTAA ou son équivalent ARN ;
ii. une séquence qui est complémentaire des séquences de (i) ;
iii. une séquence qui code pour la même protéine ou le même polypeptide que les séquences de (i) ;
iv. une séquence qui a au moins 80% d'identité de séquence avec l'une quelconque de celles de (i), (ii) et (iii), dans laquelle la protéine ou le polypeptide codé par le brin codant conserve les propriétés immunogènes de la protéine ou du polypeptide codé par la séquence de (i) ;
v. une séquence qui code pour un fragment immunogène d'une protéine ou d'un polypeptide codé par la séquence de (i), dans laquelle ledit fragment conserve les propriétés immunogènes de ladite protéine ou dudit polypeptide.
